(19) **Europäisches Patentamt / European Patent Office / Office européen des brevets**

(11) **EP 4 130 736 A1**

(12) # EUROPEAN PATENT APPLICATION
published in accordance with Art. 153(4) EPC

(43) Date of publication:
**08.02.2023 Bulletin 2023/06**

(21) Application number: **21781184.3**

(22) Date of filing: **29.03.2021**

(51) International Patent Classification (IPC):
**G01N 33/00** (1968.09) **G06N 20/00** (2019.01)
**G16C 20/40** (2019.01) **C07K 14/705** (1995.01)
**C12Q 1/02** (1980.01) **C12Q 1/66** (1980.01)
**C12Q 1/6897** (2018.01)

(52) Cooperative Patent Classification (CPC):
**G01N 33/00; G06N 20/00; G16C 20/40;**
C07K 14/705; C12Q 1/02; C12Q 1/66; C12Q 1/6897

(86) International application number:
**PCT/JP2021/013181**

(87) International publication number:
**WO 2021/200780 (07.10.2021 Gazette 2021/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.03.2020 JP 2020060531**
**30.03.2020 JP 2020060627**
**30.03.2020 JP 2020060510**

(71) Applicant: **AJINOMOTO CO., INC.**
**Chuo-ku**
**Tokyo 104-8315 (JP)**

(72) Inventors:
• **IHARA, Yusuke**
**Kawasaki-shi, Kanagawa 210-8681 (JP)**
• **IJICHI, Chiori**
**Kawasaki-shi, Kanagawa 210-8681 (JP)**
• **KAWATO, Yayoi**
**Kawasaki-shi, Kanagawa 210-8681 (JP)**
• **SOMEKAWA, Yasuko**
**Kawasaki-shi, Kanagawa 210-8681 (JP)**
• **HIROKAWA, Takatsugu**
**Tokyo 135-0064 (JP)**

(74) Representative: **Hoffmann Eitle**
**Patent- und Rechtsanwälte PartmbB**
**Arabellastraße 30**
**81925 München (DE)**

(54) **METHOD FOR PREDICTING PRESENCE OR ABSENCE OF AROMA PROPERTIES OR OLFACTORY RECEPTOR ACTIVATION PROPERTIES IN SUBSTANCE**

(57) A technique for predicting the presence or absence of an aroma property or an olfactory receptor activation property in a substance is provided. The presence or absence of the objective property is predicted for a test substance on the basis of the maximum similarity of stereochemical structure between the test substance and a reference substance.

**EP 4 130 736 A1**

[Fig. 10]

**Description**

Technical Field

In an embodiment, the present invention relates to a technique for predicting the presence or absence of an aroma property or an olfactory receptor activation property in a substance. In another embodiment, the present invention relates to a technique for predicting the presence or absence of a constituent, such as an aroma property or a molecular structure, in a substance. In another embodiment, the present invention relates to a technique for predicting the applicability to an aroma property in a substance.

Background Art

Aroma is an important factor that determines the palatability of foods, cosmetics, or the like. Hence, techniques for screening aroma ingredients necessary to reproduce a desired aroma and techniques for reproducing a desired aroma by combining aroma ingredients are industrially important for developing foods, cosmetics, or the like.
Traditionally, screening of aroma ingredients has been carried out by evaluating the aroma of test substances by human sensory test. However, sensory tests have problems such as the need to train experts who can evaluate aroma and low throughput.
In mammals such as human, aroma is recognized by binding of molecules of aroma ingredients to olfactory receptors on olfactory nerve cells in the olfactory epithelium of the upper nasal cavity, and transmission of a response of the receptors to the molecules to the central nervous system. In recent years, there have been reported methods for screening substances that exhibit a desired aroma using a response of olfactory receptors as an indicator (Patent Document 1 etc.).
In recent years, research for predicting aroma properties directly from the structure of compounds has been carried out along with the development of machine learning technology (Non-Patent Documents 1-3). There are three major technical points in improving the accuracy of prediction models: scoring of molecular structures, prediction algorithms, and quantity and quality of data for training. Among them, as existing methods for scoring of molecular structures, there have been known methods of calculating physicochemical features from molecular structures (Dragon, EPI Suite, etc.), methods of generating molecular fingerprints with 1/0 bits for the presence or absence of partial molecular structures (MACCS Keys, Morgan fingerprints, etc.) and calculating a structural similarity between molecules, and methods of forcibly scoring molecular structures as graphs (networks) or images using neural network technology. In all of these methods, it is assumed that one compound has one structure, and information on multiple conformations is ignored.

Prior art references

Patent documents

Patent document 1: JP 2019-037197 A

Non-Patent documents

Non-Patent document 1: Kobi Snitz et. al., Predicting Odor Perceptual Similarity from Odor Structure. PLoS Comput Biol 9(9): e1003184, September 2013.
Non-Patent document 2: Andreas Keller et. al., Predicting human olfactory perception from chemical features of odor molecules. Science, 355(6327):820-826, February 2017.
Non-Patent document 3: Benjamin Sanchez-Lengeling et. al., Machine Learning for Scent: Learning Generalizable Perceptual Representations of Small Molecules. arXiv:1910.10685v1, October 2019.

Summary of Invention

Object to be Achieved by the Invention

In an embodiment, an object of the present invention is to provide a technique for predicting the presence or absence of an aroma property or an olfactory receptor activation property in a substance. In another embodiment, an object of the present invention is to provide a technique for predicting the presence or absence of a constituent, such as an aroma property or a molecular structure, in a substance. In another embodiment, an object of the present invention is to provide a technique for predicting the applicability to an aroma property in a substance.

Means for Achieving the Object

The inventors of the present invention found that the presence or absence of the objective property can be predicted for a test substance on the basis of the maximum similarity of stereochemical structure between the test substance and a reference substance and accomplished an embodiment of the present invention.
The present invention can be thus embodied as follows in an embodiment.

[1] A method for predicting the presence or absence of an objective property for a test substance, the method comprising:

a step of predicting the presence or absence of the objective property for the test substance on the basis of the maximum similarity of stereochemical structure between the test substance and a reference substance, wherein the property is an aroma property or an olfactory receptor activation property.

[2] The method mentioned above, wherein the reference substance includes a positive control for the objective property.

[3] The method mentioned above, wherein the reference substance is one kind of substance.

[4] The method mentioned above, wherein the reference substance is a combination of two or more kinds of substances.

[5] The method mentioned above,

wherein the reference substance includes a positive control for the objective property, and wherein the test substance is predicted to have the objective property when the maximum similarity of stereochemical structure between the test substance and the positive control is high.

[6] The method mentioned above, wherein said predicting comprises a step of clustering the test substance and the reference substance on the basis of the maximum similarity of stereochemical structure between the test substance and the reference substance.

[7] The method mentioned above,

wherein the reference substance includes a positive control for the objective property, and wherein the test substance is predicted to have the objective property when the test substance is clustered into a cluster containing the positive control.

[8] The method mentioned above, wherein the method further comprises a step of calculating the maximum similarity, prior to said predicting.

[9] A method for screening a substance having an objective property, the method comprising:

a step of predicting the presence or absence of the objective property for a test substance by the method mentioned above, and
a step of selecting the test substance predicted to have the objective property as the substance having the objective property,
wherein the property is an aroma property or an olfactory receptor activation property.

[10] The method mentioned above, wherein the method further comprises a step of confirming the presence or absence of the objective property for the test substance predicted to have the objective property.

[11] The method mentioned above, wherein the maximum similarity is used for said predicting in combination with a structural similarity between the test substance and the reference substance other than the maximum similarity.

[12] A method for designing a substance having an objective property, the method comprising:

a step of designing the substance to be designed on the basis of the maximum similarity of stereochemical structure between the substance to be designed and a reference substance,
wherein the property is an aroma property or an olfactory receptor activation property.

[13] The method mentioned above,

wherein the reference substance includes a positive control for the objective property,
wherein said designing is carried out so that the substance to be designed is clustered into a cluster containing the positive control, and
wherein said clustering comprises a step of clustering the substance to be designed and the reference substance on the basis of the maximum similarity of stereochemical structure between the substance to be designed and the reference substance.

The inventors of the present invention also found that a model for predicting the presence or absence of an aroma property or a molecular structure in a substance can be generated by machine learning and accomplished another embodiment of the present invention.
The present invention can be thus embodied as follows in another embodiment.

[1] A method for producing a model for predicting the presence or absence of an objective constituent for a test substance, the model containing a decision tree that outputs a classification result for the presence or absence of the objective constituent in the test substance on the basis of test olfactory receptor activation data of the test substance, the method comprising:

a step of generating the decision tree by machine learning,
wherein the constituent is an aroma property or a molecular structure, and
wherein the test olfactory receptor activation data is data related to activation of a test olfactory receptor by the test substance.

[2] The method mentioned above,

wherein the machine learning is carried out by using a data set containing constituent data and reference olfactory receptor activation data of reference substances,
wherein the constituent data is data related to the objective constituent in the reference substances,
wherein the reference olfactory receptor activation data is data related to activation of reference olfactory receptors by the reference substances,
wherein the reference substances consist of a combination of two or more kinds of substances including a positive control and a negative control, and
wherein the reference olfactory receptors consist of a combination of two or more kinds of olfactory receptors including the test olfactory receptor.

[3] The method mentioned above,

wherein the constituent data is data indicating the presence or absence of the objective constituent in the reference substances, and
wherein the reference olfactory receptor activation data is data indicating the degree of activation of the reference olfactory receptors by the reference substances.

[4] The method mentioned above, wherein the machine learning is carried out by using the constituent data as the objective variable and using the reference olfactory receptor activation data as the explanatory variable.

[5] The method mentioned above, wherein the machine learning is carried out by CART.

[6] The method mentioned above, wherein the machine learning is carried out by ensemble learning.

[7] The method mentioned above, wherein the reference substances consists of a combination of 500 or more kinds of substances.

[8] The method mentioned above, wherein 50% or more of the total number of the reference substances is selected from substances listed in The Good Scents Company.

[9] The method mentioned above, wherein the test olfactory receptor is one kind of olfactory receptor or a combination of two or more kinds of olfactory receptors.

[10] The method mentioned above, wherein the reference olfactory receptors consist of a combination of 300 or more kinds of olfactory receptors.

[11] The method mentioned above, wherein 50% or more of the total number of the reference olfactory receptors is selected from OR1A1, OR1A2, OR1B1, OR1C1, OR1D2, OR1D5, OR1E1, OR1F1, OR1F12, OR1G1, OR1I1, OR1J1, OR1J2, OR1J4, OR1K1, OR1L1, OR1L3, OR1L4, OR1L8, OR1M1, OR1N1, OR1N2, OR1Q1, OR1R1P, OR1S1, OR2A1, OR2A2, OR2A4, OR2A5, OR2A12, OR2A14, OR2A25, OR2AE1, OR2AG1, OR2AG2, OR2AJ1P, OR2AK2, OR2AP1, OR2AT4, OR2B2, OR2B3, OR2B6, OR2B11, OR2C1, OR2C3, OR2D2, OR2D3, OR2F1, OR2G2, OR2G3, OR2G6, OR2H1, OR2H2, OR2J2, OR2J3, OR2K2, OR2L2, OR2L8, OR2L13, OR2M2, OR2M4, OR2M7, OR2S2, OR2T1, OR2T2, OR2T5, OR2T6, OR2T8, OR2T10, OR2T11, OR2T27, OR2T34, OR2V2, OR2W1, OR2W3, OR2Y1, OR2Z1, OR3A1, OR3A2, OR3A3, OR3A4, OR4A5, OR4A15, OR4A16, OR4A47, OR4B1, OR4C3, OR4C5, OR4C6, OR4C11, OR4C12, OR4C13, OR4C15, OR4C16, OR4C46, OR4D1, OR4D2, OR4D5, OR4D6, OR4D9, OR4D10, OR4D11, OR4E2, OR4F3, OR4F5, OR4F6, OR4F14P, OR4F15, OR4G11P, OR4H12P, OR4K1, OR4K2, OR4K5, OR4K 13, OR4K 14, OR4K15, OR4K17, OR4L1, OR4M1, OR4N2, OR4N4, OR4N5, OR4P4, OR4Q3, OR4S1, OR4S2, OR4X1, OR4X2, OR5A1, OR5A2, OR5AC2, OR5AK2, OR5AK3P, OR5AN1, OR5AP2, OR5AR1, OR5AS1, OR5AU1, OR5B2, OR5B3, OR5B12, OR5B17, OR5B21, OR5C1, OR5D13, OR5D14, OR5D16, OR5D18, OR5F1, OR5H1, OR5H2, OR5H6, OR5H14, OR5I1, OR5J2, OR5K1, OR5K3, OR5K4, OR5L2, OR5M3, OR5M8, OR5M9, OR5M10, OR5M11, OR5P3, OR5R1, OR5T1, OR5T2, OR5T3, OR5V1, OR5W2, OR6A2, OR6B1, OR6B2, OR6C1, OR6C2, OR6C3, OR6C4, OR6C6, OR6C65, OR6C66P, OR6C68, OR6C70, OR6C74, OR6C75, OR6C76, OR6F1, OR6J1, OR6K2, OR6K3, OR6K6, OR6M1, OR6N1, OR6N2, OR6P1, OR6Q1, OR6S1, OR6T1, OR6V1, OR6X1, OR6Y1, OR7A3P, OR7A5, OR7A10, OR7A17, OR7C1, OR7C2, OR7D2, OR7D4, OR7E24, OR7G1, OR7G2, OR7G3, OR8A1, OR8B3, OR8B4, OR8B8, OR8B12, OR8D1, OR8D2, OR8D4, OR8G2, OR8G5, OR8H3, OR8I2, OR8J1, OR8J3, OR8K1, OR8K3, OR8K5, OR8S1, OR8U1, OR9A4, OR9G1, OR9G4, OR9I1, OR9K2, OR9Q1, OR9Q2, OR10A3, OR10A4, OR10A5, OR10A6, OR10A7, OR10AD1, OR10AG1, OR10C1, OR10D3, OR10D4P, OR10G2, OR10G3, OR10G4, OR10G6, OR10G7, OR10G9, OR10H2, OR10H4, OR10J1, OR10J3, OR10J5, OR10K1, OR10K2, OR10P1, OR10Q1, OR10R2, OR10S1, OR10T2, OR10V1, OR10W1, OR10X1, OR10Z1, OR11A1, OR11G2, OR11H4, OR11H6, OR11H12, OR11L1, OR12D2, OR12D3, OR13A1, OR13C2, OR13C3, OR13C4, OR13C8, OR13D1, OR13F1, OR13G1, OR13H1, OR13J1, OR14A2, OR14A16, OR14C36, OR14I1, OR14J1, OR14K1, OR14L1P, OR51A1P, OR51A4, OR51A7, OR51B2, OR51B4, OR51B5, OR51B6, OR51D1, OR51E1, OR51E2, OR51F1, OR51F2, OR51F5P, OR51G1, OR51G2, OR51H1, OR51I1, OR51I2, OR51L1, OR51M1, OR51Q1, OR51S1, OR51T1, OR51V1, OR52A1, OR52A4, OR52A5, OR52B2, OR52B4, OR52B6, OR52D1, OR52E2, OR52E4, OR52E5, OR52E8, OR52H1, OR52I2, OR52J3, OR52K2, OR52L2P, OR52M1, OR52N1, OR52N2, OR52N4, OR52N5, OR52P2P, OR52R1, OR52W1, OR52Z1P, OR56A1, OR56A3, OR56A4, OR56A5, OR56B1, OR56B2P, and OR56B4.

[12] The method mentioned above, wherein the reference olfactory receptors are human olfactory receptors.

[13] A model produced by the method mentioned above.

[14] A method for predicting the presence or absence of an objective constituent for a test substance, the method comprising:

a step of predicting the presence or absence of the objective constituent for the test substance on the basis of test olfactory receptor activation data of the test substance and the model, wherein the constituent is an aroma property or a molecular structure.

[15] A method for screening a substance having an objective constituent, the method comprising:

a step of predicting the presence or absence of the objective constituent for a test substance on the basis of test olfactory receptor activation data of the test substance and the model; and a step of selecting the test substance predicted to have the objective constituent as the substance having the objective constituent, wherein the constituent is an aroma property or a molecular structure.

[16] The method mentioned above, wherein the test substance is predicted to have the objective constituent when the test substance is classified into a leaf node in which the ratio of a positive control is 50% or more.

[17] The method mentioned above, wherein the method further comprises a step of confirming the presence or absence of the objective constituent for the test substance predicted to have the objective constituent.

The inventors of the present invention also found that a model for predicting the applicability to an aroma property in a substance can be generated by machine learning and accomplished another embodiment of the present invention.

The present invention can be thus embodied as follows in another embodiment.

[1] A method for producing a model for predicting the applicability to an objective aroma property for a test substance, the model containing a regression equation that outputs a prediction value of the applicability of the test substance on the basis of test olfactory receptor activation data of the test substance, the method comprising:

a step of generating the regression equation by machine learning, and
wherein the test olfactory receptor activation data is data related to activation of a test olfactory receptor by the test substance.

[2] The method mentioned above, wherein the regression equation is a linear regression equation.

[3] The method mentioned above,

wherein the machine learning is carried out by using a data set containing aroma property data and reference olfactory receptor activation data of reference substances,
wherein the aroma property data is data indicating the applicability to the objective aroma property in the reference substances,
wherein the reference olfactory receptor activation data is data related to activation of reference olfactory receptors by the reference substances,
wherein the reference substances consist of a combination of two or more kinds of substances, and
wherein the reference olfactory receptors consist of a combination of two or more kinds of olfactory receptors including the test olfactory receptor.

[4] The method mentioned above, wherein the reference olfactory receptor activation data is data indicating the degree of activation of the reference olfactory receptors by the reference substances.

[5] The method mentioned above, wherein the machine learning is carried out by using the aroma property data as the objective variable and using the reference olfactory receptor activation data as the explanatory variable.

[6] The method mentioned above, wherein the reference substances consists of a combination of 100 or more kinds of substances.

[7] The method mentioned above, wherein 50% or more of the total number of the reference substances is selected from substances listed in Atlas of odor character profiles.

[8] The method mentioned above, wherein the aroma property data is a
"percentage of applicability" value calculated according to the criteria described in Atlas of odor character profiles.

[9] The method mentioned above, wherein the test olfactory receptor is a combination of 10 or more kinds of olfactory receptors.

[10] The method mentioned above, wherein the reference olfactory receptors consist of a combination of 300 or more kinds of olfactory receptors.

[11] The method mentioned above, wherein 50% or more of the total number of the reference olfactory receptors is selected from OR1A1, OR1A2, OR1B1, OR1C1, OR1D2, OR1D5, OR1E1, OR1F1, OR1F12, OR1G1, OR1I1, OR1J1, OR1J2, OR1J4, OR1K1, OR1L1, OR1L3, OR1L4, OR1L8, OR1M1, OR1N1, OR1N2, OR1Q1, OR1R1P, OR1S1, OR2A1, OR2A2, OR2A4, OR2A5, OR2A12, OR2A14, OR2A25, OR2AE1, OR2AG1, OR2AG2, OR2AJ1P, OR2AK2, OR2AP1, OR2AT4, OR2B2, OR2B3, OR2B6, OR2B11, OR2C1, OR2C3, OR2D2, OR2D3, OR2F1, OR2G2, OR2G3, OR2G6, OR2H1, OR2H2, OR2J2, OR2J3, OR2K2, OR2L2, OR2L8, OR2L13, OR2M2, OR2M4, OR2M7, OR2S2, OR2T1, OR2T2, OR2T5, OR2T6, OR2T8, OR2T10, OR2T11, OR2T27, OR2T34, OR2V2, OR2W1, OR2W3, OR2Y1, OR2Z1, OR3A1, OR3A2, OR3A3, OR3A4, OR4A5, OR4A15, OR4A16, OR4A47, OR4B1, OR4C3, OR4C5, OR4C6, OR4C11, OR4C12, OR4C13, OR4C15, OR4C16, OR4C46, OR4D1, OR4D2, OR4D5, OR4D6, OR4D9, OR4D10, OR4D11, OR4E2, OR4F3, OR4F5, OR4F6, OR4F14P, OR4F15, OR4G11P, OR4H12P, OR4K1, OR4K2, OR4K5, OR4K 13, OR4K14, OR4K15, OR4K17, OR4L1, OR4M1, OR4N2, OR4N4, OR4N5, OR4P4, OR4Q3, OR4S1, OR4S2, OR4X1, OR4X2, OR5A1, OR5A2, OR5AC2, OR5AK2, OR5AK3P, OR5AN1, OR5AP2, OR5AR1, OR5AS1, OR5AU1, OR5B2, OR5B3, OR5B12, OR5B17, OR5B21, OR5C1, OR5D13, OR5D14, OR5D16, OR5D18, OR5F1, OR5H1, OR5H2, OR5H6, OR5H14, OR5I1, OR5J2, OR5K1, OR5K3, OR5K4, OR5L2, OR5M3, OR5M8, OR5M9, OR5M10, OR5M11, OR5P3, OR5R1, OR5T1, OR5T2, OR5T3, OR5V1, OR5W2, OR6A2, OR6B1, OR6B2, OR6C1, OR6C2, OR6C3, OR6C4, OR6C6, OR6C65, OR6C66P, OR6C68, OR6C70, OR6C74, OR6C75, OR6C76, OR6F1, OR6J1, OR6K2, OR6K3, OR6K6, OR6M1, OR6N1, OR6N2, OR6P1, OR6Q1, OR6S1, OR6T1, OR6V1, OR6X1, OR6Y1, OR7A3P, OR7A5, OR7A10, OR7A17, OR7C1, OR7C2, OR7D2, OR7D4, OR7E24, OR7G1, OR7G2, OR7G3, OR8A1, OR8B3, OR8B4, OR8B8, OR8B12, OR8D1, OR8D2, OR8D4, OR8G2, OR8G5, OR8H3, OR8I2, OR8J1, OR8J3, OR8K1, OR8K3, OR8K5, OR8S1, OR8U1, OR9A4, OR9G1, OR9G4, OR9I1, OR9K2, OR9Q1, OR9Q2, OR10A3, OR10A4, OR10A5, OR10A6, OR10A7, OR10AD1, OR10AG1, OR10C1, OR10D3, OR10D4P, OR10G2, OR10G3, OR10G4, OR10G6, OR10G7, OR10G9, OR10H2, OR10H4, OR10J1, OR10J3, OR10J5, OR10K1, OR10K2, OR10P1, OR10Q1, OR10R2, OR10S1, OR10T2, OR10V1, OR10W1, OR10X1, OR10Z1, OR11A1, OR11G2, OR11H4, OR11H6, OR11H12, OR11L1, OR12D2, OR12D3, OR13A1, OR13C2, OR13C3, OR13C4, OR13C8, OR13D1, OR13F1, OR13G1, OR13H1, OR13J1, OR14A2, OR14A16, OR14C36, OR14I1, OR14J1, OR14K1, OR14L1P, OR51A1P, OR51A4, OR51A7, OR51B2, OR51B4, OR51B5, OR51B6, OR51D1, OR51E1, OR51E2, OR51F1, OR51F2, OR51F5P, OR51G1, OR51G2, OR51H1, OR51I1, OR51I2, OR51L1, OR51M1, OR51Q1, OR51S1, OR51T1, OR51V1, OR52A1, OR52A4, OR52A5, OR52B2, OR52B4, OR52B6, OR52D1, OR52E2, OR52E4, OR52E5, OR52E8, OR52H1, OR52I2, OR52J3, OR52K2, OR52L2P, OR52M1, OR52N1, OR52N2, OR52N4, OR52N5, OR52P2P, OR52R1, OR52W1, OR52Z1P, OR56A1, OR56A3, OR56A4, OR56A5, OR56B1, OR56B2P, and OR56B4.

[12] The method mentioned above, wherein the reference olfactory receptors are human olfactory receptors.

[13] The method mentioned above, wherein the reference olfactory receptor activation data for an olfactory receptor providing an absolute value of the correlation coefficient between the aroma property data and the reference olfactory receptor activation data of more than 0.2 among the reference olfactory receptors is used as the explanatory variable for the machine learning.

[14] The method mentioned above, wherein the step comprises a step of calculating the correlation coefficient between the aroma property data and the reference olfactory receptor activation data, prior to the machine learning.

[15] A model produced by the method mentioned above.

[16] A method for predicting the applicability to an objective aroma property for a test substance, the method comprising:
a step of predicting the applicability to an objective aroma property for the test substance on the basis of test olfactory receptor activation data of the test substance and the model.

[17] A method for screening a substance having a high applicability to an objective aroma property, the method comprising:

a step of predicting the applicability to an objective aroma property for a test substance on the basis of test olfactory receptor activation data of the test substance and the model; and
a step of selecting the test substance predicted to have a high applicability to an objective aroma property as the substance having a high applicability to an objective aroma property.

[18] The method mentioned above, wherein the method further comprises a step of confirming the applicability to an objective aroma property for the test substance predicted to have a high applicability to an objective aroma property.

Brief Description of Drawings

[FIG. 1] A diagram (halftone image) showing a heat map of a matrix of a stereochemical structural similarity.
[FIG. 2] A diagram (halftone image) showing a result of visualization of a stereochemical structural similarity space by t-SNE.
[FIG. 3] A diagram (halftone image) showing distribution of "OR4S2 activity" in a stereochemical structural similarity space.
[FIG. 4] A diagram (halftone image) showing distribution of "OR5K1 activity" in a stereochemical structural similarity space.
[FIG. 5] A diagram (halftone image) showing distribution of "OR10G4 activity" in a stereochemical structural similarity space.
[FIG. 6] A diagram (halftone image) showing distribution of aroma property "onion" in a stereochemical structural similarity space.
[FIG. 7] A diagram (halftone image) showing distribution of aroma property "nutty" in a stereochemical structural similarity space.
[FIG. 8] A diagram (halftone image) showing distribution of aroma property "phenolic" in a stereochemical structural similarity space.
[FIG. 9] A diagram (halftone image) showing a result of mixing a stereochemical structural similarity and a molecular fingerprint similarity at various mixing ratios, and evaluating the correlation between the resulting mixtures and an odor similarity.
[FIG. 10] A diagram (halftone image) showing a result of mixing a stereochemical structural similarity and a molecular fingerprint similarity at various mixing ratios, and evaluating the correlation between the resulting mixtures and an odor similarity.
[FIG. 11] A diagram showing a tree model of the aroma property "burnt".
[FIG. 12] A diagram showing a tree model of the aroma property "sweet".
[FIG. 13] A diagram showing a tree model of the aroma property "nutty".
[FIG. 14] A diagram showing a tree model of pyrazine skeleton.
[FIG. 15] A diagram showing a tree model of aldehyde group.
[FIG. 16] A diagram showing a tree model of ester bond.
[FIG. 17] A diagram showing a relationship between measured P.A. values and predicted P.A. values for the aroma property "STARWBERRY".
[FIG. 18] A diagram showing a relationship between measured P.A. values and predicted P.A. values for the aroma property "ANISE (LICORICE)".
[FIG. 19] A diagram showing a relationship between measured P.A. values and predicted P.A. values for the aroma property "NEW RUBBER".

Modes for Carrying out the Invention

(A) 1st embodiment of the present invention

Hereinafter, the 1st embodiment of the present invention, specifically, the prediction method of the present invention and the design method of the present invention according to the 1st embodiment of the present invention, will be described.

<1> Prediction method of the present invention according to the 1st embodiment of the present invention

The prediction method of the present invention is a method for predicting the presence or absence of an objective property for a test substance. The phrase "predicting the presence or absence of an objective property for a test substance" refers to predicting whether or not the test substance has the objective property. Predicting the presence or absence of the objective property for the test substance is hereinafter also referred to simply as "prediction". The prediction can be carried out on the basis of the maximum similarity of stereochemical structure between the test substance and a reference substance. That is, the prediction method of the present invention may comprise a step of predicting the presence or absence of the objective property for the test substance on the basis of the maximum similarity of stereochemical structure between the test substance and the reference substance. This step is also referred to as "prediction step". By carrying out the prediction on the basis of the maximum similarity of stereochemical structure between the test substance and the reference substance, for example, the accuracy of the prediction can be improved as compared to the case where the prediction is carried out on the basis of a structural similarity between substances in which multiple conformations are not taken into account, such as the molecular fingerprinting similarity.

In addition, by predicting the presence or absence of the objective property for the test substance, a substance having the objective property can be screened. That is, the test substance predicted to have the objective property can be selected as the substance having the objective property, and thereby the substance having the objective property can be screened. That is, the prediction method of the present invention may be a method for screening the substance having the objective property. That is, the prediction method of the present invention may further comprise a step of selecting the test substance predicted to have the objective property as the substance having the objective property. That is, the screening method may be a method for screening the substance having the objective property, the method comprising a step of predicting the presence or absence of the objective property for the test substance on the basis of the maximum similarity of stereochemical structure between the test substance and the reference substance, and a step of selecting the test substance predicted to have the objective property as the substance having the objective property. Also, in other words, the screening method may be a method for screening the substance having the objective property, the method comprising a step of predicting the presence or absence of the objective property for the test substance by the prediction method of the present invention, and a step of selecting the test substance predicted to have the objective property as the substance having the objective property.

The prediction method of the present invention may further comprise a step of calculating the maximum similarity of stereochemical structure between the test substance and the reference substance, prior to the prediction step. This step is also referred to as "calculation step".

<1-1> Objective property

The phrase "objective property" refers to a property to be predicted. Examples of the property include aroma property and olfactory receptor activation property.

The phrase "aroma property" refers to a property of exhibiting an aroma. The type of the aroma is not particularly limited. Examples of the aroma include absinthe, acacia, acai, acerola, acetic, acetone, acidic, acorn, acrylate, agarwood, alcoholic, aldehydic, alfalfa, algae, alliaceous, allspice, almond, almond bitter almond, almond roasted almond, almond toasted almond, amber, ambergris, ambrette, ammoniacal, angelica, animal, anise, anisic, apple, apple cooked apple, apple dried apple, apple green apple, apple red apple, apple skin, apricot, aromatic, arrack, artichoke, asafetida, asparagus, astringent, autumn, avocado, bacon, baked, balsamic, banana, banana peel, banana ripe banana, banana unripe banana, barley roasted barley, basil, bay, bean green bean, beany, beef juice, beefy, beefy roasted beefy, beer, beeswax, benzoin, bergamot, berry, berry ripe berry, bitter, blackberry, bloody, blueberry, bois de rose, boronia, bouillon, boysenberry, brandy, bread baked, bread crust, bread rye bread, bready, broccoli, brothy, brown, bubble gum, buchu, burnt, butter rancid, buttermilk, butterscotch, buttery, cabbage, calamus, camphoreous, cananga, candy, cantaloupe, capers, caramellic, caraway, cardamom, carnation, carrot, carrot seed, carvone, cascarilla, cashew, cassia, castoreum, catty, cauliflower, cedar, cedarwood, celery, cereal, chamomile, charred, cheesy, cheesy bleu cheese, cheesy cheddar cheese, cheesy feta cheese, cheesy gorgonzola cheese, cheesy gouda cheese, cheesy limburger cheese, cheesy parmesan cheese, cheesy roquefort cheese, chemical, cherry, cherry maraschino cherry, chervil, chestnut, chicken, chicken coup, chicken fat, chicken roasted chicken, chicory, chive, chocolate, chocolate dark chocolate, chocolate white chocolate, chrysanthemum, cider, cilantro, ciltrano, cinnamon, cinnamyl, cistus, citronella, citrus, citrus peel, citrus rind, civet, clam, clean, cloth laundered cloth, clove, clover, cocoa, coconut, coffee, coffee roasted coffee, cognac, cologne, cooked, cookie, cooling, copaiba, coriander, corn, corn chip, commeal, cornmint, cortex, costus, cotton candy, coumarinic, cranberry, creamy, cubeb, cucumber, cucumber skin, cumin, currant black currant, currant bud black currant bud, currant red currant, curry, custard, cyclamen, cypress, dairy, date, davana, deertongue, dewy, dill, dirty, dragon fruit, dry, durian, dusty, earthy, egg nog, egg yolk, eggy, elderberry, elderflower, elemi, estery, ethereal, eucalyptus, fatty, fecal, fennel, fenugreek, fermented, fig, filbert, fir needle, fishy, fleshy, floral, foliage, forest, fougere, frankincense, freesia, fresh, fresh outdoors, fried, fruit dried fruit, fruit overripe fruit, fruit ripe fruit, fruit tropical fruit, fruity, fudge, fungal, fusel, galanga, galbanum, gardenia, garlic, gasoline, gassy, genet, geranium, ginger, ginseng, goaty, goji berry, gooseberry, gourmand, graham cracker, grain, grain toasted grain, grape, grape skin, grapefruit, grapefruit peel, grassy, gravy, greasy, green, grilled, guaiacol, guaiacwood, guava, hairy, ham, harsh, hawthorn, hay, hay new mown hay, hazelnut, hazelnut roasted hazelnut, heather, heliotrope, herbal, hibiscus, honey, honeydew, honeysuckle, hops, horehound, horseradish, huckleberry, humus, hyacinth, hyssop, immortelle, incense, jackfruit, jammy, jasmin, jonquil, juicy, juicy fruit, juniper, ketonic, kimchi, kiwi, kokumi, kumquat, labdanum, lachrymatory, lactonic, lamb, lard, lavandin, lavender, lavender spike lavender, leafy, leathery, leek, lemon, lemon peel, lemongrass, lettuce, licorice, licorice black licorice, lilac, lily, lily of the valley, lime, linden flower, lingonberry, liver, lobster, loganberry, lovage, lychee, macadamia, mace, magnolia, mahogany, malty, mandarin, mango, maple, marigold, marine, marjoram, marshmallow, marzipan, mastic, meaty, meaty roasted meaty, medicinal, melon, melon rind, melon unripe melon, mentholic, metallic, milky, mimosa, minty, molasses, moldy, mossy, muguet, mulberry, mushroom, musk, mustard, musty, mutton, myrrh, naphthyl, narcissus, nasturtium, natural, neroli, noni fruit, nut flesh, nut skin, nutmeg, nutty, oakmoss, oatmeal, oats, ocean, oily, onion, onion cooked onion, onion green onion, opoponax, orange, orange bitter orange, orange peel, orange rind, orangeflower, orchid, oriental, origanum, orris, osmanthus, oyster, ozone, painty, palmarosa, papaya, paper, parsley, passion fruit, patchouli, pea green pea, peach, peanut, peanut butter, peanut roasted peanut, pear, pear skin, pecan, peely, pennyroyal, peony, pepper bell pepper, pepper black pepper, peppermint, peppery, peru balsam, petal, petitgrain, petroleum, phenolic, pimenta, pine, pineapple, pistachio, plastic, plum, plum skin, pomegranate, popcorn, pork, potato, potato baked potato, potato chip, potato raw potato, powdery, praline, privet, privetblossom, prune, pulpy, pumpkin, pungent, quince, radish, rain, raisin, rancid, raspberry, raw, reseda, resinous, rhubarb, rindy, ripe, roasted, root beer, rooty, rose, rose dried rose, rose red rose, rose tea rose, rose white rose, rosemary, rubbery, rue, rummy, saffron, sage, sage clary sage, salmon, salty, sandalwood, sandy, sappy, sarsaparilla, sassafrass, sauerkraut, sausage, sausage smoked sausage, savory, sawdust, scallion, seafood, seashore, seaweed, seedy, sesame, sharp, shellfish, shrimp, skunk, smoky, soapy, soft, solvent, soup, sour, spearmint, spicy, spinach, spruce, starchy, starfruit, storax, strawberry, stringent, styrene, sugar, sugar brown sugar, sugar burnt sugar, sulfurous, sweaty, sweet, sweet pea, taco, tagette, tallow, tamarind, tangerine, tansy, tarragon, tart, tea, tea black tea, tea green tea, tea rooibos tea, tea white tea, tequila, terpenic, thujonic, thyme, toasted, tobacco, toffee, tolu balsam, tomato, tomato leaf, tonka, tropical, truffle, tuberose, tuna, turkey, turmeric, turnup, tutti frutti, umami, urine, valerian root, vanilla, vegetable, verbena, vetiver, vinegar, violet, violet leaf, walnut, warm, wasabi, watercress, watermelon, watermelon rind, watery, waxy, weedy, wet, whiskey, winey, wintergreen, woody, woody burnt wood, woody oak wood, woody old wood, wormwood, yeasty, ylang, yogurt, yuzu, zedoary, zesty, bark, birch bark, blood, raw meat, burnt candle, burnt milk, burnt pepper, burnt rubber, cadaverous (dead animal), cardboard, cat urine, chalky, cleaning fluid, cooked vegetables, cork, creosote, crushed grass, crushed weeds, dirty linen, disinfectant, carbolic, fermented (rotten) fruit, fragrant, fresh green vegetable, fresh tobacco smoke, fried chicken, heavy, household gas, kerosene, kippery (smoked fish), laurel leaves, light, mothballs, mouse, nail polish remover, new rubber, peanut butter, perfumery, putrid, four, decayde, rope, seasoning (for meat), seminal, sperm-like, sewer, sickening, sooty, sour milk, stale, stale tobacco smoke, tab, tea leaves, turpentine (pine oil), varnish, wet paper, wet wool, and wet dog. The aroma may be one kind of aroma, or may be a combination of two or more kinds of aromas. That is, the phrase "presence or absence of an aroma property" may refer to the presence or absence of a property of exhibiting any one kind of aroma, or may refer to the presence or absence of respective properties of exhibiting two or more kinds of aromas, i.e., patterns indicating which aroma(s) is/are exhibited and which aroma(s) is/are not exhibited for two or more kinds of aromas.

The phrase "olfactory receptor activation property" refers to a property of activating an olfactory receptor. The type of the olfactory receptor is not particularly limited.

Examples of the olfactory receptor include OR1A1, OR1A2, OR1B1, OR1C1, OR1D2, OR1D4, OR1D5, OR1E1, OR1E2, OR1F1, OR1F12, OR1G1, OR1I1, OR1J1, OR1J2, OR1J4, OR1K1, OR1L1, OR1L3, OR1L4, OR1L6, OR1L8, OR1M1, OR1N1, OR1N2, OR1Q1, OR1R1P, OR1S1, OR1S2, OR2A1, OR2A2, OR2A4, OR2A5, OR2A7, OR2A12, OR2A14, OR2A25, OR2AE1, OR2AG1, OR2AG2, OR2AJ1P, OR2AK2, OR2AP1, OR2AT4, OR2B2, OR2B3, OR2B6, OR2B11, OR2C1, OR2C3, OR2D2, OR2D3, OR2F1, OR2F2, OR2G2, OR2G3, OR2G6, OR2H1, OR2H2, OR2J1P, OR2J2, OR2J3, OR2K2, OR2L2, OR2L3, OR2L5, OR2L8, OR2L13, OR2M2, OR2M3, OR2M4, OR2M5, OR2M7, OR2S2, OR2T1, OR2T2, OR2T3, OR2T4, OR2T5, OR2T6, OR2T7, OR2T8, OR2T10, OR2T11, OR2T12, OR2T27, OR2T29, OR2T33, OR2T34, OR2T35, OR2V1, OR2V2, OR2W1, OR2W3, OR2Y1, OR2Z1, OR3A1, OR3A2, OR3A3, OR3A4, OR4A4P, OR4A5, OR4A15, OR4A16, OR4A47, OR4B1, OR4C3, OR4C5, OR4C6, OR4C11, OR4C12, OR4C13, OR4C15, OR4C16, OR4C45, OR4C46, OR4D1, OR4D2, OR4D5, OR4D6, OR4D9, OR4D10, OR4D11, OR4E2, OR4F3, OR4F4, OR4F5, OR4F6, OR4F14P, OR4F15, OR4F17, OR4F21, OR4G11P, OR4H12P, OR4K1, OR4K2, OR4K5, OR4K13, OR4K14, OR4K15, OR4K17, OR4L1, OR4M1, OR4M2, OR4N2, OR4N4, OR4N5, OR4P4, OR4Q3, OR4S1, OR4S2, OR4X1, OR4X2, OR5A1, OR5A2, OR5AC2, OR5AK2, OR5AK3P, OR5AN1, OR5AP2, OR5AR1, OR5AS1, OR5AU1, OR5B2, OR5B3, OR5B12, OR5B17, OR5B21, OR5C1, OR5D13, OR5D14, OR5D16, OR5D18, OR5F1, OR5H1, OR5H2, OR5H6, OR5H14, OR5H15, OR5I1, OR5J2, OR5K1, OR5K2, OR5K3, OR5K4, OR5L1, OR5L2, OR5M1, OR5M3, OR5M8, OR5M9, OR5M10, OR5M11, OR5P2, OR5P3, OR5R1, OR5T1, OR5T2, OR5T3, OR5V1, OR5W2, OR6A2, OR6B1, OR6B2, OR6B3, OR6C1, OR6C2, OR6C3, OR6C4, OR6C6, OR6C65, OR6C66P, OR6C68, OR6C70, OR6C74, OR6C75, OR6C76, OR6F1, OR6J1, OR6K2, OR6K3, OR6K6, OR6M1, OR6N1, OR6N2, OR6P1, OR6Q1, OR6S1, OR6T1, OR6V1, OR6X1, OR6Y1, OR7A3P, OR7A5, OR7A10, OR7A17, OR7C1, OR7C2, OR7D2, OR7D4, OR7E24, OR7G1, OR7G2, OR7G3, OR8A1, OR8B2, OR8B3, OR8B4, OR8B8, OR8B12, OR8D1, OR8D2, OR8D4, OR8G1, OR8G2, OR8G5, OR8H1, OR8H2, OR8H3, OR8I2, OR8J1, OR8J3, OR8K1, OR8K3, OR8K5, OR8S1, OR8U1, OR8U8, OR9A2, OR9A4, OR9G1, OR9G4, OR9I1, OR9K2, OR9Q1, OR9Q2, OR10A2, OR10A3, OR10A4, OR10A5, OR10A6, OR10A7, OR10AD1, OR10AG1, OR10C1, OR10D3, OR10D4P, OR10G2, OR10G3, OR10G4, OR10G6, OR10G7, OR10G8, OR10G9, OR10H1, OR10H2, OR10H3, OR10H4, OR10H5, OR10J1, OR10J3, OR10J5, OR10K1, OR10K2, OR10P1, OR10Q1, OR10R2, OR10S1, OR10T2, OR10V1, OR10W1, OR10X1, OR10Z1, OR11A1, OR11G2, OR11H1, OR11H2, OR11H4, OR11H6, OR11H12, OR11L1, OR12D2, OR12D3, OR13A1, OR13C2, OR13C3, OR13C4, OR13C5, OR13C8, OR13C9, OR13D1, OR13F1, OR13G1, OR13H1, OR13J1, OR14A2, OR14A16, OR14C36, OR14I1, OR14J1, OR14K1, OR14L1P, OR51A1P, OR51A2, OR51A4, OR51A7, OR51B2, OR51B4, OR51B5, OR51B6, OR51D1, OR51E1, OR51E2, OR51F1, OR51F2, OR51F5P, OR51G1, OR51G2, OR51H1, OR51I1, OR51I2, OR51L1, OR51M1, OR51Q1, OR51S1, OR51T1, OR51V1, OR52A1, OR52A4, OR52A5, OR52B2, OR52B4, OR52B6, OR52D1, OR52E2, OR52E4, OR52E5, OR52E6, OR52E8, OR52H1, OR52I1, OR52I2, OR52J3, OR52K1, OR52K2, OR52L1, OR52L2P, OR52M1, OR52N1, OR52N2, OR52N4, OR52N5, OR52P2P, OR52R1, OR52W1, OR52Z1P, OR56A1, OR56A3, OR56A4, OR56A5, OR56B1, OR56B2P, and OR56B4.

Particular examples of the olfactory receptor include OR1A1, OR1A2, OR1B1, OR1C1, OR1D2, OR1D5, OR1E1, OR1F1, OR1F12, OR1G1, OR1I1, OR1J1, OR1J2, OR1J4, OR1K1, OR1L1, OR1L3, OR1L4, OR1L8, OR1M1, OR1N1, OR1N2, OR1Q1, OR1R1P, OR1S1, OR2A1, OR2A2, OR2A4, OR2A5, OR2A12, OR2A14, OR2A25, OR2AE1, OR2AG1, OR2AG2, OR2AJ1P, OR2AK2, OR2AP1, OR2AT4, OR2B2, OR2B3, OR2B6, OR2B11, OR2C1, OR2C3, OR2D2, OR2D3, OR2F1, OR2G2, OR2G3, OR2G6, OR2H1, OR2H2, OR2J2, OR2J3, OR2K2, OR2L2, OR2L8, OR2L13, OR2M2, OR2M4, OR2M7, OR2S2, OR2T1, OR2T2, OR2T5, OR2T6, OR2T8, OR2T10, OR2T11, OR2T27, OR2T34, OR2V2, OR2W1, OR2W3, OR2Y1, OR2Z1, OR3A1, OR3A2, OR3A3, OR3A4, OR4A5, OR4A15, OR4A16, OR4A47, OR4B1, OR4C3, OR4C5, OR4C6, OR4C11, OR4C12, OR4C13, OR4C15, OR4C16, OR4C46, OR4D1, OR4D2, OR4D5, OR4D6, OR4D9, OR4D10, OR4D11, OR4E2, OR4F3, OR4F5, OR4F6, OR4F14P, OR4F15, OR4G11P, OR4H12P, OR4K1, OR4K2, OR4K5, OR4K13, OR4K14, OR4K15, OR4K17, OR4L1, OR4M1, OR4N2, OR4N4, OR4N5, OR4P4, OR4Q3, OR4S1, OR4S2, OR4X1, OR4X2, OR5A1, OR5A2, OR5AC2, OR5AK2, OR5AK3P, OR5AN1, OR5AP2, ORSAR1, ORSAS1, OR5AU1, OR5B2, OR5B3, OR5B12, OR5B17, OR5B21, OR5C1, OR5D13, OR5D14, OR5D16, OR5D18, OR5F1, OR5H1, OR5H2, OR5H6, OR5H14, OR5I1, OR5J2, OR5K1, OR5K3, OR5K4, OR5L2, OR5M3, OR5M8, OR5M9, OR5M10, OR5M11, OR5P3, OR5R1, OR5T1, OR5T2, OR5T3, OR5V1, OR5W2, OR6A2, OR6B1, OR6B2, OR6C1, OR6C2, OR6C3, OR6C4, OR6C6, OR6C65, OR6C66P, OR6C68, OR6C70, OR6C74, OR6C75, OR6C76, OR6F1, OR6J1, OR6K2, OR6K3, OR6K6, OR6M1, OR6N1, OR6N2, OR6P1, OR6Q1, OR6S1, OR6T1, OR6V1, OR6X1, OR6Y1, OR7A3P, OR7A5, OR7A10, OR7A17, OR7C1, OR7C2, OR7D2, OR7D4, OR7E24, OR7G1, OR7G2, OR7G3, OR8A1, OR8B3, OR8B4, OR8B8, OR8B12, OR8D1, OR8D2, OR8D4, OR8G2, OR8G5, OR8H3, OR8I2, OR8J1, OR8J3, OR8K1, OR8K3, OR8K5, OR8S1, OR8U1, OR9A4, OR9G1, OR9G4, OR9I1, OR9K2, OR9Q1, OR9Q2, OR10A3, OR10A4, OR10A5, OR10A6, OR10A7, OR10AD1, OR10AG1, OR10C1, OR10D3, OR10D4P, OR10G2, OR10G3, OR10G4, OR10G6, OR10G7, OR10G9, OR10H2, OR10H4, OR10J1, OR10J3, OR10J5, OR10K1, OR10K2, OR10P1, OR10Q1, OR10R2, OR10S1, OR10T2, OR10V1, OR10W1, OR10X1, OR10Z1, OR11A1, OR11G2, OR11H4, OR11H6, OR11H12, OR11L1, OR12D2, OR12D3, OR13A1, OR13C2, OR13C3, OR13C4, OR13C8, OR13D1, OR13F1, OR13G1, OR13H1, OR13J1, OR14A2, OR14A16, OR14C36, OR14I1, OR14J1, OR14K1, OR14L1P, OR51A1P, OR51A4, OR51A7, OR51B2, OR51B4, OR51B5, OR51B6, OR51D1, OR51E1, OR51E2, OR51F1, OR51F2, OR51F5P, OR51G1, OR51G2, OR51H1, OR51I1, OR51I2, OR51L1, OR51M1, OR51Q1, OR51S1, OR51T1, OR51V1, OR52A1, OR52A4, OR52A5, OR52B2, OR52B4, OR52B6, OR52D1, OR52E2, OR52E4, OR52E5, OR52E8, OR52H1, OR52I2, OR52J3, OR52K2, OR52L2P, OR52M1, OR52N1, OR52N2, OR52N4, OR52N5, OR52P2P, OR52R1, OR52W1, OR52Z1P, OR56A1, OR56A3, OR56A4, OR56A5, OR56B1, OR56B2P, and OR56B4.

A gene encoding the olfactory receptor is referred to as "olfactory receptor gene". The olfactory receptor may be one kind of olfactory receptor, or may be a combination of two or more kinds of olfactory receptors. That is, the phrase "presence or absence of an olfactory receptor activation property" may refer to the presence or absence of a property of activating any one kind of olfactory receptor, or may refer to the presence or absence of respective properties of activating two or more kinds of olfactory receptors, i.e., patterns indicating which olfactory receptor(s) is/are activated and which olfactory receptor(s) is/are not activated for two or more kinds of olfactory receptors.

Examples of the olfactory receptor gene and olfactory receptor include olfactory receptor genes and olfactory receptors of various organisms. Examples of the organisms include, for example, animals such as mammals. Specific examples of the animals such as mammals include, for example, Homo sapiens (human), Mus musculus (mouse), Rattus norvegicus (rat), Canis lupus familiaris (dog), Felis catus (cat), Bos taurus (cow), Sus scrofa (pig), Pan troglodytes (chimpanzee), Macaca fascicularis (crab-eating macaque), and Equus caballus (horse). Particular examples of the animals such as mammals include Homo sapiens. The nucleotide sequences of the olfactory receptor genes and the amino acid sequences of the olfactory receptors of various organisms can be obtained from, for example, public database such as NCBI and Ensembl.

The olfactory receptor may be, for example, a protein having any of known or natural amino acid sequences of such olfactory receptors as described above. The olfactory receptor may also be, for example, a variant of a protein having any of the known or natural amino acid sequences of such olfactory receptors as described above. That is, the olfactory receptor specified with each of the aforementioned names shall include proteins having any of the known or natural amino acid sequences of the olfactory receptor specified with each of the aforementioned names and variants thereof. The phrase "a protein has an amino acid sequence" means that the protein comprises the amino acid sequence, and also includes cases where the protein consists of the amino acid sequence. Examples of the variant include a protein having any of the known or natural amino acid sequences including substitution, deletion, insertion, and/or addition of one or several amino acid residues at one or several positions. The number meant by the term "one or several" used above may specifically be, for example, 1 to 50, 1 to 40, or 1 to 30, preferably 1 to 20, more preferably 1 to 10, still more preferably 1 to 5, particularly preferably 1 to 3. Examples of the variant also include a protein having an amino acid sequence showing an identity of, for example, 50% or more, 65% or more, or 80% or more, preferably 90% or more, more preferably 95% or more, still more preferably 97% or more, particularly preferably 99% or more, to the total amino acid sequence of any of the known or natural amino acid sequences. An olfactory receptor specified with the type of organism from which the olfactory receptor is derived is not limited to olfactory receptors themselves found in that organism, and shall also include proteins having any of the amino acid sequences of olfactory receptors found in that organism and variants thereof. Such variants may or may not be found in that organism. That is, for example, the term "olfactory receptor of human" is not limited to olfactory receptors themselves found in human, and shall also include proteins having any of the amino acid sequences of olfactory receptors found in human and variants thereof. The olfactory receptor may also be, for example, a chimeric protein of two or more kinds of olfactory receptors having different origins. That is, the olfactory receptor specified with each of the aforementioned names shall include chimeric proteins of two or more kinds of the olfactory receptor specified with each of the aforementioned names having different origins.

The term "identity" between amino acid sequences means an identity between the amino acid sequences calculated by blastp with default scoring parameters (i.e., Matrix, BLOSUM62; Gap Costs, Existence = 11, Extension = 1; Compositional Adjustments, Conditional compositional score matrix adjustment).

<1-2> Test substance

The phrase "test substance" refers to a substance for which the presence or absence of the objective property is to be predicted. In other words, the phrase "test substance" refers to a substance to be used as a candidate for the substance having the objective property in the method of screening the substance having the objective property. The test substance is not particularly limited, so long as the structure thereof has been identified.

It is sufficient that the structure of the test substance has been identified to such an extent that multiple conformations of the test substance can be generated. It is sufficient that the structure of the test substance has been identified, for example, as a chemical structural formula. The structure of the test substance may or may not be known. When the structure of the test substance is not known, it is sufficient to identify the structure of the test substance as required prior to generation of the multiple conformations. Methods for identifying the structure of the test substance are not particularly limited. The structure of the test substance can be identified, for example, by known methods for identifying the structure of substances. Examples of such methods include nuclear magnetic resonance (NMR), electron spin resonance (ESR), ultraviolet-visible-near-infrared spectroscopy (UV-Vis-NIR), infrared spectroscopy (IR), Raman spectroscopy, and mass spectrometry (MS). These methods may be used individually or in combination as appropriate.

The test substance may be a known substance or a novel substance. The test substance may be a natural substance or an artificial substance. The test substance may be, for example, a compound library produced using combinatorial chemistry techniques. Examples of the test substance include, for example, alcohols, ketones, aldehydes, ethers, esters, hydrocarbons, sugars, organic acids, nucleic acids, amino acids, peptides, and other various organic or inorganic ingredients. Furthermore, particular examples of the test substance include existing food additives. The phrase "existing food additive" refers to a substance that have already been approved for use as a food additive. The test substance may also be a virtual substance, i.e., a substance having a virtual structure. Examples of the virtual substance include those listed in compound databases such as GDB-11, GDB-13, GDB-17, ZINC15, FooDB, and VCF (Volatile Compounds in Food). As the test substance, one kind of test substance may be used, or a combination of two or more kinds of test substances may be used. The test substance may be selected so as to include such a substance as exemplified above, such as existing food additives. That is, as the test substance, for example, one kind of existing food additive may be used, a combination of two or more kinds of food additives may be used, or a combination of one or more kinds of food additives and one or more kinds of other substances may be used. The phrase "a combination of two or more kinds of test substances is used" refers to predicting the presence or absence of the objective property for each of the two or more kinds of test substances.

<1-3> Reference substance

The phrase "reference substance" refers to a substance that can be used as an indicator of the presence or absence of the objective property. The reference substance is not particularly limited, so long as the structure thereof has been identified and the presence or absence of the objective property therein has been identified.

It is sufficient that the structure of the reference substance has been identified to such an extent that multiple conformations of the reference substance can be generated. It is sufficient that the structure of the reference substance has been identified, for example, as a chemical structural formula. The structure of the reference substance may or may not be known. When the structure of the reference substance is not known, it is sufficient to identify the structure of the reference substance as required prior to generation of the multiple conformations. Methods for identifying the structure of the reference substance are not particularly limited. The structure of the reference substance can be identified, for example, by known methods for identifying the structure of substances. Examples of such methods include nuclear magnetic resonance (NMR), electron spin resonance (ESR), ultraviolet-visible-near-infrared spectroscopy (UV-Vis-NIR), infrared spectroscopy (IR), Raman spectroscopy, and mass spectrometry (MS). These methods may be used individually or in combination as appropriate.

The presence or absence of the objective property in the reference substance may or may not be known. When the presence or absence of the objective property in the reference substance is not known, it is sufficient to identify the presence or absence of the objective property in the reference substance as required prior to carrying out the prediction step. Methods for identifying the presence or absence of a property in the reference substance are not particularly limited. The presence or absence of a property in the reference substance can be identified, for example, by known methods for identifying the presence or absence of a property in substances. The presence or absence of the aroma property in the reference substance can be identified, for example, by sensory evaluation by expert panels. The presence or absence of the olfactory receptor activation property in the reference substance can be identified, for example, by bringing the olfactory receptor into contact with the reference substance and measuring the presence or absence of activation of the olfactory receptor by the contact with the reference substance. Contact between the olfactory receptor and the reference substance and measurement of the presence or absence of activation of the olfactory receptor by the contact can be carried out, for example, by referring to methods for screening substances exhibiting an objective aroma using a response of olfactory receptors as an indicator (e.g., JP 2019-037197 A). The olfactory receptor may be used, for example, in the form carried on cells such as animal cells. Activation of the olfactory receptor can be measured, for example, on the basis of an increased in the intracellular calcium level or the intracellular cAMP level as an indicator. Examples of methods for measuring the intracellular cAMP level include, for example, ELISA and reporter assay. Examples of the reporter assay include, for example, luciferase assay. According to the reporter assay, the intracellular cAMP level can be measured by using a reporter gene (e.g., luciferase gene) configured to be expressed in a cAMP level-dependent manner. Examples of methods for measuring the intracellular calcium level include, for example, calcium imaging.

The degree of the objective property in the reference substance may also have been identified. The phrase "degree of an objective property" in the case of the aroma property may refer to an intensity with which a substance exhibits an aroma. The phrase "degree of an objective property" in the case of the olfactory receptor activation property may refer to an intensity with which a substance activates an olfactory receptor. The degree of the objective property in the reference substance can be identified, for example, by the same method as identification of the presence or absence of the objective property in the reference substance.

Contact between the olfactory receptor and the reference substance and measurement of the presence or absence or degree of activation of the olfactory receptor by the contact can be carried out, specifically, for example, according to the following procedure.

That is, the presence or absence or degree of activation of the olfactory receptor by the reference substance can be determined by bringing the olfactory receptor into contact with the reference substance and using an activation degree D1, which is the degree of activation of the olfactory receptor upon carrying out the contact (i.e., under conditions of bringing the olfactory receptor into contact with the reference substance), as an indicator. The concentration of the reference substance in contact with the olfactory receptor can be set according to various conditions, such as the type of the olfactory receptor and the type of the reference substance. The concentration of the reference substance in contact with the olfactory receptor may be, for example, 3 to 1000 $\mu$M. The concentration of the reference substance in contact with the olfactory receptor may be, typically, 300 $\mu$M. For a reference substance cytotoxic at 300 $\mu$M, the concentration of the reference substance in contact with the olfactory receptor may be, for example, 3 $\mu$M, 10 $\mu$M, 30 $\mu$M, or 100 $\mu$M.

The presence or absence or degree of activation of the olfactory receptor by the reference substance can be determined, specifically, by comparing the activation degree D1 and an activation degree D2, which is the degree of activation of the olfactory receptor under control conditions. Examples of the control conditions include conditions of not bringing the olfactory receptor into contact with the reference substance.

The activation degree D1 and the activation degree D2 each can be obtained and used as data reflecting a parameter that acts as an indicator of activation of the olfactory receptor. Examples of the parameter that acts as an indicator of activation of the olfactory receptor include intracellular calcium level and intracellular cAMP level. Examples of data reflecting the intracellular cAMP level in the case of the luciferase assay include luminescence intensity. The data reflecting a parameter that acts as an indicator of activation of the olfactory receptor can be used as it is, or can be subject to processing such as correction as required and then used.

When the activation degree D1 is high, it may be judged that the olfactory receptor was activated by the reference substance. For example, when the ratio of the activation degree D1 to the activation degree D2 (i.e., D1/D2) is 1.5 or more, 2 or more, 3 or more, 5 or more, 10 or more, 20 or more, 50 or more, or 100 or more, it may be judged that the olfactory receptor was activated by the reference substance. Examples of the ratio of the activation degree D1 to the activation degree D2 include the "normalized response" value described in Examples.

Furthermore, the degree of activation of the olfactory receptor by the reference substance can be determined on the basis of a comparison result between the activation degree D1 and the activation degree D2 as an indicator. For example, the ratio of the activation degree D1 to the activation degree D2 (i.e., D1/D2) can be regarded as the degree of activation of the olfactory receptor by the reference substance. Examples of the ratio of the activation degree D1 to the activation degree D2 include the "normalized response" value described in Examples.

Examples of the reference substance include positive control and negative control. The term "positive control" refers to a substance having the objective property. The term "negative control" refers to a substance not having the objective property. The reference substance may include at least the positive control.

The reference substance may be a known substance or a novel substance. The reference substance may be a natural substance or an artificial substance. The reference substance may be, for example, a compound library produced using combinatorial chemistry techniques. Examples of the reference substance include, for example, alcohols, ketones, aldehydes, ethers, esters, hydrocarbons, sugars, organic acids, nucleic acids, amino acids, peptides, and other various organic or inorganic ingredients. Specific examples of the reference substance include substances in which the presence or absence and/or degree of the objective property is known. Examples of the substances in which the presence or absence and/or degree of the objective property is known include substances listed in The Good Scents Company (http://www.thegoodscentscompany.com/). That is, the reference substance may include the substance(s) listed in The Good Scents Company. For example, 50% or more, 60% or more, 70% or more, 80% or more, 90% or more, or 95% or more of the total number of the reference substances may be selected from the substances listed in The Good Scents Company. The substances listed in The Good Scents Company each may be regarded, for example, as a substance exhibiting the aroma(s) listed in "Odor Description" therein, i.e., as a positive control for the aroma(s) listed in "Odor Description" therein. The substances listed in The Good Scents Company each may be regarded, for example, as a substance not exhibiting the aroma(s) not listed in "Odor Description" therein, i.e., as a negative control for the aroma(s) listed in "Odor Description" therein. Examples of the substances in which the presence or absence and/or degree of the objective property is known also include substances listed in Atlas of odor character profiles (Dravnieks, A., ASTM data series publication, DS 61, PCN 05-061000-36, 1985). That is, the reference substance may include the substance(s) listed in Atlas of odor character profiles. The substances listed in Atlas of odor character profiles each may be regarded, for example, as a positive control or a negative control for each aroma depending on the "percentage of applicability" value of the aroma. That is, the substances listed in Atlas of odor character profiles each may be regarded, for example, as a positive control for a certain aroma when the "percentage of applicability" value of the certain aroma is high. Furthermore, the substances listed in Atlas of odor character profiles each may be regarded, for example, as a negative control for a certain aroma when the "percentage of applicability" value of the certain aroma is low. The phrase "the percentage of applicability value is high" may mean, for example, that the "percentage of applicability" value is 4 or more, 7 or more, 10 or more, 15 or more, or 20 or more. The phrase "the percentage of applicability value is low" may mean, for example, that the "percentage of applicability" value is less than 4, 3 or less, 2 or less, 1 or less, or 0.5 or less. As the reference substance, one kind of reference substance may be used, or a combination of two or more kinds of reference substances may be used.

Each of the number of the reference substances, the number of the positive controls, and the number of the negative controls, for example, may be 1 or more, 2 or more, 3 or more, 5 or more, 7 or more, 10 or more, 15 or more, 20 or more, 25 or more, 30 or more, 40 or more, 50 or more, 70 or more, 100 or more, 150 or more, 200 or more, 300 or more, 400 or more, 500 or more, 600 or more, 700 or more, 800 or more, 900 or more, or 1000 or more, may be 10000 or less, 5000 or less, 2000 or less, 1000 or less, 500 or less, 200 or less, 150 or less, 100 or less, 70 or less, 50 or less, 40 or less, 30 or less, 25 or less, 20 or less, 15 or less, or 10 or less, or may be within a range defined as a non-contradictory combination thereof. Each of the number of the reference substances, the number of the positive controls, and the number of the negative controls may be, specifically, for example, 1 to 10000, 1 to 1000, 1 to 100, 1 to 10, 10 to 10000, 10 to 1000, 10 to 100, 100 to 10000, 100 to 1000, or 1000 to 10000. Each of the number of the reference substances, the number of the positive controls, and the number of the negative controls may be, specifically, for example, 1 to 10, 10 to 100, 100 to 200, 200 to 500, 500 to 1000, 1000 to 2000, 2000 to 5000, or 5000 to 10000.

The ratio of the positive control in the reference substance, for example, may be 1% or more, 3% or more, 5% or more, 10% or more, 20% or more, 30% or more, 40% or more, 50% or more, 60% or more, 70% or more, 80% or more, 90% or more, or 95% or more, may be 100% or less, 99% or less, 97% or less, 95% or less, 90% or less, 80% or less, 70% or less, 60% or less, 50% or less, 40% or less, 30% or less, 20% or less, 10% or less, or 5% or less, or may be within a range defined as a non-contradictory combination thereof. The ratio of the positive control in the reference substance may be, specifically, for example, 1 to 100%, 1 to 50%, 1 to 20%, 1 to 10%, 1 to 5%, 5 to 100%, 5 to 50%, 5 to 20%, 5 to 10%, 10 to 100%, 10 to 50%, 10 to 20%, 20 to 100%, 20 to 50%, or 50 to 100%. The ratio of the positive control in the reference substance may be, specifically, for example, 1 to 10%, 10 to 20%, 20 to 30%, 30 to 40%, 40 to 50%, 50 to 60%, 60 to 70%, 70 to 80%, 80 to 90%, or 90 to 100%. The phrase "ratio of a positive control in a reference substance" refers to the ratio of the number of the positive controls to the total number of the reference substances.

<1-4> Maximum similarity of stereochemical structure between substances

The phrase "maximum similarity of stereochemical structure between substances" refers to a maximum value of the similarities of stereochemical structure between substances, which substances are hereinafter referred to as a certain substance A and another substance B. The phrase "maximum similarity of stereochemical structure between substances" refers to, specifically, a maximum value of the similarities of all pairs between multiple conformations of the substance A and multiple conformations of the substance B. The phrase "multiple conformations of a substance" refers to two or more conformations that the substance has, and in other words, refers to conformations of two or more conformational isomers that the substance has. That is, when the substance A has n conformations (A1 to An) and the substance B has m conformations (B 1 to Bm), the phrase "maximum similarity of stereochemical structure between substances" refers to a maximum value of the similarities of n×m pairs (i.e., the par of A1 and B1 to the pair of An and Bm). Each of the number of conformations that the test substance has and the number of conformations that the reference substance has is not particularly limited, so long as it is 2 or more. The maximum similarity of stereochemical structure is also referred to simply as "maximum similarity".

Methods for generating multiple conformations of a substance are not particularly limited. The multiple conformations of a substance can be generated, for example, by known methods. The multiple conformations of a substance can be generated, specifically, for example, by using software such as conformation generating software OMEGA (OpenEye). That is, the multiple conformations of a substance can be generated from structural data of the substance by using the software. The software can be used, for example, according to the manufacturer's manual. In the case of OMEGA, for example, multiple conformations of macrocyclic compounds (e.g., cyclic compounds having a 12- or more membered ring) may be generated in OMEGA macrocyclic mode, and multiple conformations of the other compounds may be generated in OMEGA classic mode.

The phrase "structural data of a substance" refers to data indicating the structure of the substance. The structural data of a substance is not particularly limited, so long as the multiple conformations can be generated. The structural data of a substance can be selected according to various conditions, such as the type of software used for generating the multiple conformations of the substance. As the structural data of a substance, for example, existing data may be obtained and used, or data may be obtained by conversion from chemical structural formula and used. The existing data can be obtained, for example, from chemical databases such as PubChem and ChemSpider, or from websites of reagent companies such as Sigma Aldrich. Conversion from chemical structural formula can be carried out, for example, by using software such as ChemDraw or websites. The obtained structural data of a substance, for example, may be used as is for generating the multiple conformations, or may be used for generating the multiple conformations after appropriate modifications. For example, when the data is in isomeric SMILES format, it may be canonicalized to absolute SMILES format, converted to 3D structural data such as those in MOL format or SDF format containing MOL format data, subject to processing such as hydrogenation and optimization, and then used for generating the multiple conformations. Canonicalization of SMILES data and conversion to 3D structure data can be carried out, for example, by using software such as chemoinformatics software RDKit (http://www.rdkit.org). Processing, such as hydrogenation and optimization, of 3D structure data can be carried out, for example, by using software such as an integrated computational chemistry system MOE (CCG).

The maximum similarity between the substances A and B can be obtained, for example, by calculating the similarities of respective pairs of the multiple conformations of the substance A and the multiple conformations of the substance B, as the maximum value among the calculated similarities. The similarities of pairs may be calculated for all pairs, or only for partial pairs that at least include a pair providing the maximum value. For example, pair(s) providing low similarity may be preliminarily excluded from the calculation of the similarities of pairs on the basis of appropriate criteria. The similarities of pairs may be calculated typically for all pairs.

Examples of the similarity of stereochemical structure include Tanimoto coefficient. Examples of the Tanimoto coefficient include Shape Tanimoto score, which indicates the similarity of surface shape, Color Tanimoto score, which indicates the similarity of surface chemical properties, and Tanimoto Combo score, which indicates the similarity of surface shape and surface chemical properties. Tanimoto Combo score is calculated as the sum of Shape Tanimoto score and Color Tanimoto score. The Tanimoto coefficient can be calculated, for example, by using software such as molecular surface shape similarity calculation software ROCS (OpenEye). When calculating the similarity of stereochemical structures by using ROCS, the calculated similarity may vary depending on which of the compared substances is used as the query. In such a case, any of the calculated similarities may be used for calculation of the maximum similarity, so long as the prediction can be carried out with the desired accuracy. For example, the lower of the calculated similarities may be used for calculation of the maximum similarity, or the higher of the calculated similarities may be used for calculation of the maximum similarity. Alternatively, for example, the average value of the calculated similarities may be used for calculation of the maximum similarity. The maximum similarity obtained as the maximum value of the Tanimoto coefficient is also referred to as "maximum similarity based on Tanimoto coefficient".

<1-5> Prediction step

The prediction can be carried out on the basis of the maximum similarity between the test substance and the reference substance.

The prediction may be carried out, for example, by directly evaluating the maximum similarity between the test substance and the reference substance. That is, the phrase "predicting the presence or absence of an objective property for a test substance on the basis of the maximum similarity of stereochemical structure between the test substance and a reference substance" may include carrying out the prediction by directly evaluating the maximum similarity between the test substance and the reference substance. Furthermore, the prediction step may comprise, for example, a step of directly evaluating the maximum similarity between the test substance and the reference substance.

That is, for example, when the maximum similarity between the test substance and the positive control is high, the test substance may be predicted to have the objective property. The phrase "the maximum similarity between a test substance and a positive control is high" in cases where the positive control is one kind of substance means that the maximum similarity between the test substance and this one kind of positive control is high. The phrase "the maximum similarity between a test substance and a positive control is high" in cases where the positive control is a combination of two or more kinds of substances may mean, for example, that the average or maximum value of the maximum similarities between the test substance and these two or more kinds of positive controls is high. The phrase "the maximum similarity between a test substance and a positive control is high" in cases where the positive control is a combination of two or more kinds of substances may also mean, for example, that the number or ratio of the positive control(s) showing a high maximum similarity to the test substance is large. Furthermore, for example, when the maximum similarity between the test substance and the positive control is not high, the test substance may be predicted not to have the objective property.

The phrase "the maximum similarity is high" may mean, for example, that the maximum similarity is not less than a predetermined value. The predetermined value is not particularly limited, so long as the prediction can be carried out with the desired accuracy. The phrase "the maximum similarity is high" may mean, for example, that the maximum similarity normalized to a range of 0 to 1 is 0.5 or more, 0.6 or more, 0.7 or more, 0.8 or more, or 0.9 or more. The phrase "the maximum similarity is high" may mean, specifically, for example, that the maximum similarity based on Shape Tanimoto score is 0.5 or more, 0.6 or more, 0.7 or more, 0.8 or more, or 0.9 or more. The phrase "the maximum similarity is high" may mean, specifically, for example, that the maximum similarity based on Color Tanimoto score is 0.5 or more, 0.6 or more, 0.7 or more, 0.8 or more, or 0.9 or more. The phrase "the maximum similarity is high" may mean, specifically, for example, that the maximum similarity based on Tanimoto Combo score is 1 or more, 1.2 or more, 1.4 or more, 1.6 or more, or 1.8 or more. A high maximum similarity between the substances A and B may also be expressed as "the substance A shows a high maximum similarity to the substance B" or "the substance B shows a high maximum similarity to the substance A".

The phrase "the average value of the maximum similarities is high" or "the maximum value of the maximum similarities is high" may mean, for example, that the average or maximum value of the maximum similarities is not less than a predetermined value. The predetermined value is not particularly limited, so long as the prediction can be carried out with the desired accuracy. The phrase "the average value of the maximum similarities is high" or "the maximum value of the maximum similarities is high" may mean, for example, that the average or maximum value of the maximum similarities normalized to a range of 0 to 1 is 0.5 or more, 0.6 or more, 0.7 or more, 0.8 or more, or 0.9 or more. The phrase "the average value of the maximum similarities is high" or "the maximum value of the maximum similarities is high" may mean, specifically, for example, that the average or maximum value of the maximum similarities based on Shape Tanimoto score is 0.5 or more, 0.6 or more, 0.7 or more, 0.8 or more, or 0.9 or more. The phrase "the average value of the maximum similarities is high" or "the maximum value of the maximum similarities is high" may mean, specifically, for example, that the average or maximum value of the maximum similarities based on Color Tanimoto score is 0.5 or more, 0.6 or more, 0.7 or more, 0.8 or more, or 0.9 or more. The phrase "the average value of the maximum similarities is high" or "the maximum value of the maximum similarities is high" may mean, specifically, for example, that the average or maximum value of the maximum similarities based on Tanimoto Combo score is 1 or more, 1.2 or more, 1.4 or more, 1.6 or more, or 1.8 or more.

The phrase "the number of positive controls showing a high maximum similarity to a test substance is large" may mean, for example, that the number of the positive controls showing a high maximum similarity to the test substance is 1 or more, 2 or more, 3 or more, 5 or more, 7 or more, 10 or more, 15 or more, 20 or more, 25 or more, 30 or more, 40 or more, 50 or more, 70 or more, 100 or more, 150 or more, 200 or more, 300 or more, 400 or more, or 500 or more.

The phrase "the ratio of a positive control showing a high maximum similarity to a test substance is large" may mean, for example, that the ratio of the positive control showing a high maximum similarity to the test substance is 1% or more, 3% or more, 5% or more, 10% or more, 20% or more, 30% or more, 40% or more, 50% or more, 60% or more, 70% or more, 80% or more, 90% or more, or 95% or more. The phrase "ratio of a positive control showing a high maximum similarity to a test substance" refers to the ratio of the number of the positive controls showing a high maximum similarity to the test substance to the total number of the reference substances.

The prediction may also be carried out, for example, by clustering the test substance and the reference substance on the basis of the maximum similarity between these substances. That is, the phrase "predicting the presence or absence of an objective property for a test substance on the basis of the maximum similarity of stereochemical structure between the test substance and a reference substance" may include carrying out the prediction by clustering the test substance and the reference substance on the basis of the maximum similarity between these substances. Furthermore, the prediction step may comprise, for example, a step of clustering the test substance and the reference substance on the basis of the maximum similarity between these substances. The clustering may be carried out, in particular, when a combination of two or more kinds of reference substances is used.

The clustering can be carried out by using the maximum similarity between the test substance and the reference substance as a variable. The variable to be used for the clustering may or may not consist only of the maximum similarity between the test substance and the reference substance. That is, in addition to the maximum similarity between the test substance and the reference substance, other variable(s) may also be used for the clustering. The other variables are not particularly limited, so long as the prediction can be carried out with the desired accuracy. Examples of the other variables include the similarity, such as the maximum similarity, of stereochemical structure between the test and reference substances and other substance(s). In other words, in the prediction step, only the test substance and the reference substance may be subject to clustering, or other substance(s) in addition to the test substance and the reference substance may be subject to clustering.

The maximum similarity between substances may be used for the prediction (e.g., clustering) alone or in combination with a structural similarity between substances other than the maximum similarity. The structural similarity between substances other than the maximum similarity is also referred to as "additional structural similarity". A combination of the maximum similarity and the additional structural similarity is also referred to as "mixed similarity". When carrying out the prediction on the basis of the mixed similarity, the "maximum similarity" in the aforementioned description of the prediction step may be read as the "mixed similarity". That is, for example, the phrase "the maximum similarity between a test substance and a positive control is high" when carrying out the prediction on the basis of the mixed similarity may mean that the mixed similarity between the test substance and the positive control is high. Furthermore, for example, the phrase "the maximum similarity between a test substance and a positive control is low" when carrying out the prediction on the basis of the mixed similarity may mean that the mixed similarity between the test substance and the positive control is low. Examples of the additional structural similarity include structural similarities between substances in which multiple conformations are not taken into account, such as the molecular fingerprinting similarity. Upon calculation of the mixed similarity, the maximum similarity and the additional structural similarity may be corrected to have a uniform scale as appropriate, and then may be mutually combined. The ratio of the maximum similarity in the mixed similarity is not particularly limited, so long as the prediction can be carried out with the desired accuracy. The ratio of the maximum similarity in the mixed similarity, for example, may be 1% or more, 3% or more, 5% or more, 20% or more, 30% or more, 40% or more, 50% or more, 60% or more, 70% or more, 80% or more, or 90% or more, may be 99% or less, 97% or less, 95% or less, 90% or less, 80% or less, 70% or less, 60% or less, 50% or less, 40% or less, or 30% or less, or may be within a range defined as a non-contradictory combination thereof. The ratio of the maximum similarity in the mixed similarity may be, specifically, for example, 1 to 99%, 10 to 99%, 30 to 99%, 50 to 99%, 60 to 95%, or 70 to 90%. By carrying out the prediction on the basis of the mixed similarity, for example, the accuracy of the prediction may be improved as compared to the case where the prediction is carried out only on the basis of the maximum similarity.

The ratio of the total number of the test substances and the reference substances to the total number of the substances to be subject to clustering may be, for example, 50% or more, 60% or more, 70% or more, 80% or more, 90% or more, 95% or more, 97% or more, or 99% or more. Also, the ratio of the number of the reference substances to the total number of the substances to be subject to clustering may be, for example, 50% or more, 60% or more, 70% or more, 80% or more, 90% or more, 95% or more, 97% or more, or 99% or more.

The clustering may be carried out at once or separately at two or more times, so long as the prediction can be carried out with the desired accuracy. For example, some substances may be preliminarily subject to clustering, and then the remaining substance(s) may be further subject to clustering on the basis of the obtained clustering results. Specifically, for example, substances other than the test substance may be preliminarily subject to clustering, and then the test substance may be further subject to clustering on the basis of the obtained clustering results. That is, specifically, for example, it may be determined afterwards to which cluster the test substance is clustered among the clusters preliminarily created for substances other than the test substance. When two or more kinds of test substances are used in combination, those test substances may be subject to clustering together at once or may be subject to clustering separately at two or more times.

Methods for clustering is not particularly limited. The clustering can be carried out, for example, by known methods. Examples of such methods include hierarchical cluster analysis and dimensionality reduction method. Examples of the hierarchical cluster analysis include the Ward method, the nearest neighbor method, the furthest neighbor method, and the group average method. Particular examples of the hierarchical cluster analysis include the Ward method. Examples of the distance between substances to be used in the hierarchical cluster analysis include Euclidean distance, Mahalanobis distance, Manhattan distance, Chebyshev distance, Minkowski distance, Canberra distance, distance based on cosine similarity, angular distance, distance based on the Pearson's correlation coefficient, and distance based on the extended Jaccard coefficient. Particular examples of the distance between substances to be used in the hierarchical cluster analysis include Euclidean distance. The hierarchical cluster analysis may be carried out, specifically, for example, by the Ward method using the Euclidean distance. Examples of the dimensionality reduction method include Random Projection, Principal Component Analysis (PCA), Linear Discriminant Analysis (LDA), Isometric mapping (Isomap), Locally Linear Embedding (LLE), Modified LLE (MLLE), Hessian-based LLE (HLLE), Spectral Embedding, Local Tangent Space Alignment (LTSA), Multi-dimensional Scaling (MDS), t-distributed Stochastic Neighbor Embedding (t-SNE), Random Forest Embedding, Uniform Manifold Approximation and Projection (UMAP), Kernel PCA, and Autoencoder. Particular examples of the dimensionality reduction method include t-SNE. These methods may be used individually or in combination as appropriate.

The number of the clusters is not particularly limited, so long as the prediction can be carried out with the desired accuracy. The number of the clusters, for example, may be 2 or more, 3 or more, 4 or more, 5 or more, 6 or more, 7 or more, 8 or more, 9 or more, or 10 or more, may be 100 or less, 50 or less, 30 or less, 25 or less, 20 or less, 15 or less, 12 or less, 10 or less, 9 or less, 8 or less, 7 or less, 6 or less, or 5 or less, or may be within a range defined as a non-contradictory combination thereof. The number of the clusters may be, specifically, for example, 2 to 30, 3 to 20, or 4 to 15.

For example, when the test substance is clustered into a cluster indicating a high possibility of possession of the objective property, the test substance may be predicted to have the objective property. Incidentally, for example, when the test substance is clustered into a cluster indicating a high possibility of possession of the objective property, it may be judged that the maximum similarity between the test substance and the positive control is high. Also, for example, when the test substance is not clustered into a cluster indicating a high possibility of possession of the objective property, the test substance may be predicted not to have the objective property. Incidentally, for example, when the test substance is not clustered into a cluster indicating a high possibility of possession of the objective property, it may be judged that the maximum similarity between the test substance and the positive control is not high. The cluster indicating a high possibility of possession of the objective property is also referred to as "positive cluster". As a result of the clustering, only one positive cluster may be created, or two or more positive clusters may be created. Examples of the cluster indicating a high possibility of possession of the objective property include a cluster containing the positive control. The cluster containing the positive control may contain one or more kinds of positive controls. The cluster containing the positive control may or may not contain substance(s) other than the positive control. The cluster containing the positive control may or may not contain, for example, the negative control. The cluster containing the positive control may be, for example, a cluster having a high ratio of the positive control. The phrase "cluster having a high ratio of a positive control" may refer to, for example, a cluster in which the ratio of the positive control is 1% or more, 3% or more, 5% or more, 10% or more, 20% or more, 30% or more, 40% or more, 50% or more, 60% or more, 70% or more, 80% or more, 90% or more, or 95% or more. The phrase "ratio of a positive control" in a certain cluster refers to the ratio of the number of the positive controls contained in the cluster to the number of the reference substances contained in the cluster. The cluster containing the positive control may also be, for example, a cluster indicating a high degree of the objective property. The phrase "cluster indicating a high degree of an objective property" may refer to, for example, a cluster containing the positive control having the highest degree of the objective property. The phrase "cluster indicating a high degree of an objective property" may also refer to, for example, a cluster having the highest average value of the degrees of the objective property. When two or more positive clusters are created, for example, clusters that satisfy the criteria exemplified above may be selected as the positive clusters in turn. The phrase "average value of the degrees of an objective property" in a certain cluster refers to the average value of the degrees of the objective property of all reference substances contained in the cluster.

The prediction method of the present invention may further comprise a step of evaluating a result of the prediction. That is, by evaluating the objective property of the test substance, it can be confirmed whether the test substance actually has the objective property. Specifically, for example, by evaluating the objective property of the test substance predicted to have the objective property, it can be confirmed whether the test substance actually has the objective property. That is, the step of evaluating a result of the prediction may be, for example, a step of confirming the presence or absence of the objective property for the test substance predicted to have the objective property. Methods for evaluating a result of the prediction is not particularly limited. The descriptions concerning the identification of the presence or absence of the objective property in the reference substance can be similarly applied to the methods for evaluating a result of the prediction.

<2> Design method of the present invention according to the 1st embodiment of the present invention

The design method of the present invention is a method for designing a substance having the objective property.

The phrases "design of a substance" and "design of the structure of a substance" may be used interchangeably. Designing a substance having the objective property is hereinafter also referred to simply as "design". The design can be carried out on the basis of the maximum similarity of stereochemical structure between the substance to be designed and a reference substance. That is, the design method of the present invention may comprise a step of designing the substance to be designed on the basis of the maximum similarity of stereochemical structure between the substance to be designed and the reference substance. This step is also referred to as "design step".

The design can be carried out, for example, so that the substance to be designed is predicted to have the objective property on the basis of the prediction method of the present invention. In other words, the substance to be designed can be designed so as to have a structure predicted to have the objective property on the basis of the prediction method of the present invention. For example, the structure of an existing substance may be modified so as to have a structure predicted to have the objective property on the basis of the prediction method of the present invention. Also, for example, the structures of a number of compounds may be designed, and that/those predicted to have the objective property on the basis of the prediction method of the present invention may be selected. The design can be carried out, specifically, for example, so that the substance to be designed is clustered into a cluster indicating a high possibility of possession of the objective property, such as a cluster containing the positive control.

(B) 2nd embodiment of the present invention

Hereinafter, the 2nd embodiment of the present invention, specifically, the prediction model production method of the present invention and the prediction method of the present invention according to the 2nd embodiment of the present invention, will be described.

<1> Prediction model production method of the present invention according to the 2nd embodiment of the present invention

The prediction model production method of the present invention is a method for producing a model for predicting the presence or absence of an objective constituent for a test substance. The phrase "predicting the presence or absence of an objective constituent for a test substance" refers to predicting whether or not the test substance has the objective constituent. Predicting the presence or absence of the objective constituent for the test substance is hereinafter also referred to simply as "prediction". The model for predicting the presence or absence of the objective constituent for the test substance is hereinafter also referred to simply as "prediction model".

<1-1> Prediction model

The prediction model is a model for predicting the presence or absence of the objective constituent for the test substance. Hence, the prediction model can be used for the prediction. The prediction model can be used for the prediction, specifically, in a manner described in the prediction method of the present invention.

The prediction model may contain a decision tree. A decision tree or a model containing the same is also referred to as "tree model". The decision tree is not particularly limited, so long as it outputs a conclusion usable as an indicator for the prediction. The prediction can be carried out on the basis of test olfactory receptor activation data of the test substance. The test olfactory receptor activation data of the test substance is hereinafter also referred to simply as "test olfactory receptor activation data". That is, the decision tree may be one that outputs a conclusion usable as an indicator for the prediction on the basis of the test olfactory receptor activation data, i.e., by using the test olfactory receptor activation data as a variable. Examples of the conclusion usable as an indicator for the prediction include classification result for the presence or absence of the objective constituent in the test substance. That is, the decision tree may be, for example, one that outputs a classification result for the presence or absence of the objective constituent in the test substance on the basis of the test olfactory receptor activation data. The phrase "classification result for the presence or absence of an objective constituent in a test substance" refers to a classification result indicating whether or not the test substance has the objective constituent. The classification result for the presence or absence of the objective constituent in the test substance is obtained, specifically, as a result of classifying the test substance into any one of leaf nodes contained in the decision tree. That is, the decision tree may be, specifically, one that classifies the test substance into any one of leaf nodes contained in the decision tree on the basis of the test olfactory receptor activation data.

<1-2> Objective constituent

The phrase "objective constituent" refers to a constituent to be predicted. Examples of the constituent include aroma property and molecular structure.

The aroma, the aroma property, and the presence or absence of the aroma property are as described in the 1st embodiment of the present invention.

The phrase "molecular structure" refers to a parameter related to the structure of a substance. The type of the molecular structure is not particularly limited. Examples of the molecular structure include partial structure of a molecule. Examples of the partial structure of a molecule include functional groups, skeletons, bonds, and atoms. Specific examples of the molecular structure include carbonyl group, acyl group, aldehyde group, ketone group, carboxyl group, carboxamide group, alkanoyl group, benzoyl group, alkoxycarbonyl group, phenoxycarbonyl group, imide group, enone group, alkyl group, alkenyl group, hydroxyl group, amino group, imino group, aryl group, oxo group, alkoxy group, phenoxy group, alkylenedioxy group, thiol group, sulfo group, nitro group, ester bond, ether bond, amide bond, glycoside bond, nitrogen atom, oxygen atom, sulfur atom, halogen atoms, monocyclic skeletons, heterocyclic skeletons, and terpenoid skeletons. Examples of the heterocyclic skeletons include heterocyclic skeletons containing a hetero atom such as nitrogen, sulfur, and oxygen. The heterocyclic skeleton may contain one or more hetero atoms. Specific examples of the heterocyclic skeletons include heterocyclic skeletons containing nitrogen, such as pyrazine skeleton and pyrrole skeleton, and heterocyclic skeletons containing nitrogen and sulfur, such as thiazole skeleton. The molecular structure may be one kind of molecular structure, or may be a combination of two or more kinds of molecular structures. That is, the phrase "presence or absence of a molecular structure" may refer to the presence or absence of any one kind of molecular structure, or may refer to the presence or absence of two or more kinds of molecular structures, i.e., patterns indicating which molecular structure(s) is/are present and which molecular structure(s) is/are not present for two or more kinds of molecular structures.

<1-3> Test substance

The phrase "test substance" refers to a substance for which the presence or absence of the objective constituent is to be predicted. In other words, the phrase "test substance" refers to a substance to be used as a candidate for the substance having the objective constituent in the method of screening the substance having the objective constituent. The test substance is not particularly limited, so long as the test olfactory receptor activation data thereof is available.

The phrase "test olfactory receptor activation data of a test substance" refers to data related to activation of the test olfactory receptor by the test substance. The phrase "activation of a test olfactory receptor by a test substance" may be used interchangeably with the phrase "response of a test olfactory receptor to a test substance". Examples of the test olfactory receptor activation data include data indicating the presence or absence of activation of the test olfactory receptor by the test substance and data indicating the degree of activation of the test olfactory receptor by the test substance. Particular examples of the test olfactory receptor activation data include data indicating the degree of activation of the test olfactory receptor by the test substance. The phrase "degree of activation of a test olfactory receptor by a test substance" may refer to an intensity with which the test substance activates the test olfactory receptor. The test olfactory receptor activation data is used, specifically, in a branch contained in the decision tree.

The phrase "test olfactory receptor" refers to an olfactory receptor to be used in a branch contained in the decision tree. The phrase "an olfactory receptor is used in a branch contained in a decision tree" may mean that the test olfactory receptor activation data for this olfactory receptor, i.e., data related to activation of this olfactory receptor by the test substance, is used in a branch contained in the decision tree. Examples of the test olfactory receptor include the following olfactory receptors. The test olfactory receptor may be one kind of olfactory receptor, or may be a combination of two or more kinds of olfactory receptors.

The olfactory receptor and a gene encoding the same (olfactory receptor gene) are as described in the 1st embodiment of the present invention.

The test olfactory receptor activation data may or may not be known. When the test olfactory receptor activation data is not known, it is sufficient to obtain the test olfactory receptor activation data as required prior to carrying out the prediction. Methods for obtaining the test olfactory receptor activation data are not particularly limited. The test olfactory receptor activation data can be obtained, for example, by known methods for identifying the presence or absence or degree of activation of olfactory receptors by substances. The test olfactory receptor activation data can be obtained, specifically, for example, by bringing the test olfactory receptor into contact with the test substance and measuring the presence or absence or degree of activation of the test olfactory receptor by the contact with the test substance. Contact between the test olfactory receptor and the test substance and measurement of the presence or absence or degree of activation of the test olfactory receptor by the contact can be carried out, for example, by referring to methods for screening substances exhibiting an objective aroma using a response of olfactory receptors as an indicator (e.g., JP 2019-037197 A). The test olfactory receptor may be used, for example, in the form carried on cells such as animal cells. Activation of the test olfactory receptor can be measured, for example, on the basis of an increased in the intracellular calcium level or the intracellular cAMP level as an indicator. Examples of methods for measuring the intracellular cAMP level include, for example, ELISA and reporter assay. Examples of the reporter assay include, for example, luciferase assay. According to the reporter assay, the intracellular cAMP level can be measured by using a reporter gene (e.g.,

luciferase gene) configured to be expressed in a cAMP level-dependent manner. Examples of methods for measuring the intracellular calcium level include, for example, calcium imaging.

Contact between the test olfactory receptor and the test substance and measurement of the presence or absence or degree of activation of the test olfactory receptor by the contact can be carried out, specifically, for example, according to the following procedure.

That is, the presence or absence or degree of activation of the test olfactory receptor by the test substance can be determined by bringing the test olfactory receptor into contact with the test substance and using an activation degree D1, which is the degree of activation of the test olfactory receptor upon carrying out the contact (i.e., under conditions of bringing the test olfactory receptor into contact with the test substance), as an indicator. The concentration of the test substance in contact with the test olfactory receptor can be set according to various conditions, such as the type of the test olfactory receptor and the type of the test substance. The concentration of the test substance in contact with the test olfactory receptor may be, for example, 3 to 1000 $\mu$M. The concentration of the test substance in contact with the test olfactory receptor may be, typically, 300 $\mu$M. For a test substance cytotoxic at 300 $\mu$M, the concentration of the test substance in contact with the olfactory receptor may be, for example, 3 $\mu$M, 10 $\mu$M, 30 $\mu$M, or 100 $\mu$M.

The presence or absence or degree of activation of the test olfactory receptor by the test substance can be determined, specifically, by comparing the activation degree D1 and an activation degree D2, which is the degree of activation of the test olfactory receptor under control conditions. Examples of the control conditions include conditions of not bringing the test olfactory receptor into contact with the test substance.

The activation degree D1 and the activation degree D2 each can be obtained and used as data reflecting a parameter that acts as an indicator of activation of the test olfactory receptor. Examples of the parameter that acts as an indicator of activation of the test olfactory receptor include intracellular calcium level and intracellular cAMP level. Examples of data reflecting the intracellular cAMP level in the case of the luciferase assay include luminescence intensity. The data reflecting a parameter that acts as an indicator of activation of the test olfactory receptor can be used as it is, or can be subject to processing such as correction as required and then used.

When the activation degree D1 is high, it may be judged that the test olfactory receptor was activated by the test substance. For example, when the ratio of the activation degree D1 to the activation degree D2 (i.e., D1/D2) is 1.5 or more, 2 or more, 3 or more, 5 or more, 10 or more, 20 or more, 50 or more, or 100 or more, it may be judged that the test olfactory receptor was activated by the test substance. Examples of the ratio of the activation degree D1 to the activation degree D2 include the "normalized response" value described in Examples.

Furthermore, the degree of activation of the test olfactory receptor by the test substance can be determined on the basis of a comparison result between the activation degree D1 and the activation degree D2 as an indicator. For example, the ratio of the activation degree D1 to the activation degree D2 (i.e., D1/D2) can be regarded as the degree of activation of the test olfactory receptor by the test substance. Examples of the ratio of the activation degree D1 to the activation degree D2 include the "normalized response" value described in Examples.

The test substance may be a known substance or a novel substance. The test substance may be a natural substance or an artificial substance. The test substance may be, for example, a compound library produced using combinatorial chemistry techniques. Examples of the test substance include, for example, alcohols, ketones, aldehydes, ethers, esters, hydrocarbons, sugars, organic acids, nucleic acids, amino acids, peptides, and other various organic or inorganic ingredients. Furthermore, particular examples of the test substance include existing food additives. The phrase "existing food additive" refers to a substance that have already been approved for use as a food additive. As the test substance, one kind of test substance may be used, or a combination of two or more kinds of test substances may be used. The test substance may be selected so as to include such a substance as exemplified above, such as existing food additives. That is, as the test substance, for example, one kind of existing food additive may be used, a combination of two or more kinds of food additives may be used, or a combination of one or more kinds of food additives and one or more kinds of other substances may be used. The phrase "a combination of two or more kinds of test substances is used" refers to predicting the presence or absence of the objective constituent for each of the two or more kinds of test substances.

In an embodiment, the test substance may be a mixture.

When the test substance is a mixture, the phrase "the presence or absence or degree of activation of a test olfactory receptor by a test substance" refers to the presence or absence or degree of activation of the test olfactory receptor by the whole of the mixture, regardless of the presence or absence or degree of activation of the test olfactory receptor by each of substances constituting the mixture.

When the test substance is a mixture, the phrase "the presence or absence of an objective constituent in a test substance" refers to the presence or absence of the objective constituent in the whole of the mixture, regardless of the presence or absence of the objective constituent in each of substances constituting the mixture. That is, for example, when the test substance is a mixture, the phrase "a test substance has an objective aroma property" means that the mixture as a whole has the objective aroma property, regardless whether or not each of substances constituting the mixture has the objective aroma property. Also, the phrase "a test substance has an objective molecular structure" means that the mixture as a whole has the objective molecular structure, i.e., that at least one substance selected from substances constituting the mixture has the objective molecular structure, regardless whether or not each of substances constituting the mixture other than the at least one substance has the objective molecular structure.

<1-4> Generation of decision tree

The decision tree can be generated by machine learning. That is, the prediction model production method of the present invention may comprise a step of generating the decision tree by machine learning. This step is also referred to as "decision tree generation step".

Conditions for the machine learning are not particularly limited, so long as the decision tree with which the prediction can be carried out with the desired accuracy is obtained.

The machine learning can be carried out by using a data set containing constituent data and reference olfactory receptor activation data of reference substances. The constituent data of the reference substances is hereinafter also referred to simply as "constituent data". The reference olfactory receptor activation data of the reference substances is hereinafter also referred to simply as "reference olfactory receptor activation data".

The machine learning can be carried out, for example, by using the constituent data as the objective variable and using the reference olfactory receptor activation data as the explanatory variable.

Methods for the machine learning are not particularly limited, so long as the decision tree is generated. Examples of the methods for the machine learning include CART (Classification and Regression Trees), CHAID (Chi-squared Automatic Interaction Detection), ID3 (Iterative Dichotomiser 3), and C4.5. Particular examples of the methods for the machine learning include CART.

The machine learning may be carried out, for example, by ensemble learning. Examples of the ensemble learning include bagging and boosting. Examples of the bagging include Random Forest and Extremely Randomized Trees (ExtraTrees). Examples of the boosting include XGboost and LightGBM. When the machine learning is carried out by ensemble learning, the decision tree contained in the prediction model may be the decision tree after the ensemble learning. That is, for example, when bagging is carried out, the prediction model may contain a plurality of decision trees obtained by the bagging. In this case, a plurality of decision trees can be used in combination in the prediction step. That is, according to bagging, a plurality of decision trees can be generated as weak learners, and a combination of such a plurality of weak learners can be used as a strong learner. Also, for example, when boosting is carried out, the prediction model may contain a decision tree whose learning level has been improved by the boosting. That is, according to boosting, a decision tree as a strong learner can be generated and used on the basis of a decision tree generated as a weak learner.

The phrase "reference substance" refers to a substance that can be used for generation of the decision tree as an indicator of the presence or absence of the objective constituent. The reference substances are not particularly limited, so long as the constituent data and reference olfactory receptor activation data thereof are available.

The phrase "constituent data of reference substances" refers to data related to the objective constituent in the reference substances. The constituent data in cases where the objective constituent is the aroma property is also referred to as "aroma property data". The constituent data in cases where the objective constituent is the molecular structure is also referred to as "molecular structure data". Examples of the constituent data include data indicating the presence or absence of the objective constituent in the reference substances.

The phrase "reference olfactory receptor activation data of reference substances" refers to data related to activation of the reference olfactory receptors by the reference substances. Examples of the reference olfactory receptor activation data include data indicating the presence or absence of activation of the reference olfactory receptors by the reference substances and data indicating the degree of activation of the reference olfactory receptors by the reference substances. Particular examples of the reference olfactory receptor activation data include data indicating the degree of activation of the reference olfactory receptors by the reference substances.

The phrase "reference olfactory receptor" refers to an olfactory receptor to be used for generation of the decision tree. The phrase "an olfactory receptor is used for generation of a decision tree" may mean that the reference olfactory receptor activation data for this olfactory receptor, i.e., data related to activation of this olfactory receptor by the reference substances, is used for generation of the decision tree. Examples of the reference olfactory receptors include the aforementioned olfactory receptors. That is, the reference olfactory receptors may include the aforementioned olfactory receptor(s). For example, 50% or more, 60% or more, 70% or more, 80% or more, 90% or more, or 95% or more of the total number of the reference olfactory receptors may be selected from the aforementioned olfactory receptors. As the reference olfactory receptors, a combination of two or more kinds of olfactory receptors including the test olfactory receptor is used. The reference olfactory receptors may consist of the test olfactory receptor, or may include, in addition to the test olfactory receptor, other olfactory receptor(s). In other words, a part or all of the reference olfactory receptors is selected as the test olfactory receptor. That is, the olfactory receptor to be used in a branch contained in the decision tree among the reference olfactory receptors is selected as the test olfactory receptor.

The number of the reference olfactory receptors is not particularly limited, so long as the decision tree with which the prediction can be carried out with the desired accuracy is obtained. The number of the reference olfactory receptors can be set, for example, according to various conditions, such as the type of the objective constituent and the method for machine learning.

The number of the reference olfactory receptors, for example, may be 50 or more, 70 or more, 100 or more, 150 or more, 200 or more, 300 or more, 400 or more, or 500 or more, may be 2000 or less, 1500 or less, 1000 or less, 500 or less, 400 or less, 300 or less, 200 or less, 150 or less, or 100 or less, or may be within a range defined as a non-contradictory combination thereof. The number of the reference olfactory receptors may be, specifically, for example, 50 to 2000, 100 to 1000, or 300 to 500.

The constituent data may or may not be known. When the constituent data is not known, it is sufficient to obtain the constituent data as required prior to generation of the decision tree. Methods for obtaining the constituent data are not particularly limited. The constituent data can be obtained, for example, by known methods for identifying the presence or absence or degree of constituents of substances. The presence or absence or degree of the objective aroma property in the reference substances can be identified, for example, by sensory evaluation by expert panels. The presence or absence of the objective molecular structure in the reference substances can be identified, for example, by known methods for identifying the structure of substances. Examples of such methods include nuclear magnetic resonance (NMR), electron spin resonance (ESR), ultraviolet-visible-near-infrared spectroscopy (UV-Vis-NIR), infrared spectroscopy (IR), Raman spectroscopy, and mass spectrometry (MS). These methods may be used individually or in combination as appropriate.

The reference olfactory receptor activation data may or may not be known. When the reference olfactory receptor activation data is not known, it is sufficient to obtain the reference olfactory receptor activation data as required prior to generation of the decision tree. Methods for obtaining the reference olfactory receptor activation data are not particularly limited. The reference olfactory receptor activation data can be obtained, for example, by known methods for identifying the presence or absence or degree of activation of olfactory receptors by substances. The reference olfactory receptor activation data can be obtained, specifically, for example, by bringing each reference olfactory receptor into contact with each reference substance and measuring the presence or absence or degree of activation of the reference olfactory receptor by the contact with the reference substance. The aforementioned descriptions concerning the contact between the test olfactory receptor and the test substance and measurement of the presence or absence or degree of activation of the test olfactory receptor by the contact can be similarly applied to the contact between the reference olfactory receptor and the reference substance and measurement of the presence or absence or degree of activation of the reference olfactory receptor by the contact.

As the reference substances, a combination of two or more kinds of substances including a positive control and a negative control is used. The phrase "positive control" refers to a substance having the objective constituent. The phrase "negative control" refers to a substance not having the objective constituent.

The reference substances each may be a known substance or a novel substance. The reference substances each may be a natural substance or an artificial substance. The reference substances each may be, for example, a compound library produced using combinatorial chemistry techniques. Examples of the reference substances include, for example, alcohols, ketones, aldehydes, ethers, esters, hydrocarbons, sugars, organic acids, nucleic acids, amino acids, peptides, and other various organic or inorganic ingredients. Specific examples of the reference substances include substances in which the presence or absence and/or degree of the objective constituent is known. Examples of the substances in which the presence or absence and/or degree of the objective constituent is known include substances listed in The Good Scents Company (http://www.thegoodscentscompany.com/). That is, the reference substances may include the substance(s) listed in The Good Scents Company. For example, 50% or more, 60% or more, 70% or more, 80% or more, 90% or more, or 95% or more of the total number of the reference substances may be selected from the substances listed in The Good Scents Company. The substances listed in The Good Scents Company each may be regarded, for example, as a substance exhibiting the aroma(s) listed in "Odor Description" therein, i.e., as a positive control for the aroma(s) listed in "Odor Description" therein. The substances listed in The Good Scents Company each may be regarded, for example, as a substance not exhibiting the aroma(s) not listed in "Odor Description" therein, i.e., as a negative control for the aroma(s) listed in "Odor Description" therein. Examples of the substances in which the presence or absence and/or degree of the objective constituent is known also include substances listed in Atlas of odor character profiles (Dravnieks, A., ASTM data series publication, DS 61, PCN 05-061000-36, 1985). That is, the reference substances may include the substance(s) listed in Atlas of odor character profiles. The substances listed in Atlas of odor character profiles each may be regarded, for example, as a positive control or a negative control for each aroma depending on the "percentage of applicability" value of the aroma. That is, the substances listed in Atlas of odor character profiles each may be regarded, for example, as a positive control for a certain aroma when the "percentage of applicability" value of the certain aroma is high. Furthermore, the substances listed in Atlas of odor character profiles each may be regarded, for example, as a negative control for a certain aroma when the "percentage of applicability" value of the certain aroma is low. The phrase "the percentage of applicability value is high" may mean, for example, that the "percentage of applicability" value is 4 or more, 7 or more, 10 or more, 15 or more, or 20 or more. The phrase "the percentage of applicability value is low" may mean, for example, that the "percentage of applicability" value is less than 4, 3 or less, 2 or less, 1 or less, or 0.5 or less. Such substances as exemplified above each may be regarded as a positive control for the molecular structure present in the respective substances. Also, such substances as exemplified above each may be regarded as a negative control for the molecular structure absent in the respective substances.

In an embodiment, the reference substances each may be a mixture.

When the reference substances each are a mixture, the phrase "the presence or absence or degree of activation of a reference olfactory receptor by a reference substance" refers to the presence or absence or degree of activation of the reference olfactory receptor by the whole of the mixture, regardless of the presence or absence or degree of activation of the reference olfactory receptor by each of substances constituting the mixture.

When the reference substances each are a mixture, the phrase "the presence or absence of an objective constituent in a reference substance" refers to the presence or absence of the objective constituent in the whole of the mixture, regardless of the presence or absence of the objective constituent in each of substances constituting the mixture. That is, for example, when the reference substances each are a mixture, the phrase "a reference substance has an objective aroma property" means that the mixture as a whole has the objective aroma property, regardless whether or not each of substances constituting the mixture has the objective aroma property. Also, the phrase "a reference substance has an objective molecular structure" means that the mixture as a whole has the objective molecular structure, i.e., that at least one substance selected from substances constituting the mixture has the objective molecular structure, regardless whether or not each of substances constituting the mixture other than the at least one substance has the objective molecular structure.

The number of the reference substances, the number of the positive controls, and the number of the negative controls, and the ratios thereof are not particularly limited, so long as the decision tree with which the prediction can be carried out with the desired accuracy is obtained. The number of the reference substances, the number of the positive controls, and the number of the negative controls, and the ratios thereof can be set, for example, according to various conditions, such as the type of the objective constituent and the method for machine learning.

The number of the reference substances, for example, may be 100 or more, 150 or more, 200 or more, 300 or more, 400 or more, 500 or more, 600 or more, 700 or more, 800 or more, 900 or more, 1000 or more, 1500 or more, 2000 or more, 3000 or more, 5000 or more, 10000 or more, 20000 or more, 50000 or more, or 100000 or more, may be 1000000 or less, 500000 or less, 200000 or less, 100000 or less, 50000 or less, 20000 or less, 10000 or less, 5000 or less, 3000 or less, 2000 or less, 1500 or less, 1000 or less, or 500 or less, or may be within a range defined as a non-contradictory combination thereof. The number of the reference substances may be, specifically, for example, 100 to 1000000, 200 to 500000, 500 to 100000, or 1000 to 20000. The number of the reference substances may be, specifically, for example, 100 to 200, 200 to 500, 500 to 1000, 1000 to 2000, 2000 to 5000, 5000 to 10000, 10000 to 20000, 20000 to 50000, 50000 to 100000, or 100000 to 200000.

Each of the number of the positive controls and the number of the negative controls, for example, may be 5 or more, 10 or more, 15 or more, 20 or more, 25 or more, 30 or more, 40 or more, 50 or more, 70 or more, 100 or more, 150 or more, 200 or more, 300 or more, 400 or more, 500 or more, 600 or more, 700 or more, 800 or more, 900 or more, 1000 or more, 1500 or more, 2000 or more, 3000 or more, 5000 or more, 10000 or more, 20000 or more, 50000 or more, or 100000 or more, may be 1000000 or less, 500000 or less, 200000 or less, 100000 or less, 50000 or less, 20000 or less, 10000 or less, 5000 or less, 3000 or less, 2000 or less, 1500 or less, 1000 or less, 500 or less, 200 or less, 150 or less, 100 or less, 70 or less, or 50 or less, or may be within a range defined as a non-contradictory combination thereof. Each of the number of the positive controls and the number of the negative controls may be, specifically, for example, 5 to 1000000, 100 to 1000000, 200 to 500000, 500 to 100000, or 1000 to 20000. Each of the number of the positive controls and the number of the negative controls may be, specifically, for example, 5 to 10, 10 to 100, 100 to 200, 200 to 500, 500 to 1000, 1000 to 2000, 2000 to 5000, 5000 to 10000, 10000 to 20000, 20000 to 50000, 50000 to 100000, or 100000 to 200000.

Each of the ratio of the positive control and the ratio of the negative control in the reference substances, for example, may be more than 0%, 1% or more, 3% or more, 5% or more, 10% or more, 20% or more, 30% or more, 40% or more, 50% or more, 60% or more, 70% or more, 80% or more, 90% or more, or 95% or more, may be less than 100%, 99% or less, 97% or less, 95% or less, 90% or less, 80% or less, 70% or less, 60% or less, 50% or less, 40% or less, 30% or less, 20% or less, 10% or less, or 5% or less or may be within a range defined as a non-contradictory combination thereof. Each of the ratio of the positive control and the ratio of the negative control in the reference substances may be, specifically, for example, 1 to 99%, 1 to 50%, 1 to 20%, 1 to 10%, 1 to 5%, 5 to 99%, 5 to 50%, 5 to 20%, 5 to 10%, 10 to 99%, 10 to 50%, 10 to 20%, 20 to 99%, 20 to 50%, or 50 to 99%. Each of the ratio of the positive control and the ratio of the negative control in the reference substances may be, specifically, for example, 1 to 10%, 10 to 20%, 20 to 30%, 30 to 40%, 40 to 50%, 50 to 60%, 60 to 70%, 70 to 80%, 80 to 90%, or 90 to 99%. The phrase "ratio of a positive control in reference substances" refers to the ratio of the number of the positive controls to the total number of the reference substances. The phrase "ratio of a negative control in reference substances" refers to the ratio of the number of the negative controls to the total number of the reference substances. The total number of the reference substances may be the sum of the number of the positive controls and the number of the negative controls.

By carrying out the machine learning in this way, the decision tree can be generated. The decision tree contains two or more leaf nodes. One or more of the leaf nodes contained in the decision tree each are regarded as a positive leaf node. That is, the decision tree contains one or more positive leaf nodes. The phrase "positive leaf node" refers to a leaf node indicating a high possibility of possession of the objective constituent. The phrase "positive leaf node" specifically refers to a leaf node indicating a high possibility that a substance classified thereinto has the objective constituent.

The number of the leaf nodes contained in the decision tree is not particularly limited, so long as the prediction can be carried out with the desired accuracy. The number of the leaf nodes contained in the decision tree, for example, may be 2 or more, 3 or more, 4 or more, 5 or more, 6 or more, 7 or more, 8 or more, 9 or more, or 10 or more, may be 100 or less, 50 or less, 30 or less, 25 or less, 20 or less, 15 or less, 12 or less, 10 or less, 9 or less, 8 or less, 7 or less, 6 or less, or 5 or less, or may be within a range defined as a non-contradictory combination thereof. The number of the leaf nodes contained in the decision tree may be, specifically, for example, 2 to 30, 3 to 20, or 4 to 15.

The number of the positive leaf nodes contained in the decision tree is not particularly limited, so long as the prediction can be carried out with the desired accuracy. The decision tree may contain only one positive leaf node, or may contain two or more positive leaf nodes. The number of the positive leaf nodes contained in the decision tree, for example, may be 1 or more, 2 or more, 3 or more, 4 or more, or 5 or more, may be 10 or less, 9 or less, 8 or less, 7 or less, 6 or less, 5 or less, 4 or less, 3 or less, or 2 or less, or may be within a range defined as a non-contradictory combination thereof. The number of the positive leaf nodes contained in the decision tree may be, specifically, for example, 1 to 10, 1 to 6, or 1 to 4.

It is not particularly limited which leaf node is regarded as the positive leaf node, so long as the prediction can be carried out with the desired accuracy. Examples of the positive leaf node include a leaf node containing the positive control. The leaf node containing the positive control may contain one or more kinds of positive controls. The leaf node containing the positive control may or may not contain the negative control. The leaf node containing the positive control may be, for example, a leaf node having a high ratio of the positive control. The phrase "leaf node having a high ratio of a positive control" may refer to, for example, a leaf node in which the ratio of the positive control is 30% or more, 40% or more, 50% or more, 60% or more, 70% or more, 80% or more, 90% or more, or 95% or more. The phrase "ratio of a positive control" in a certain leaf node refers to the ratio of the number of the positive controls contained in the leaf node to the number of the reference substances contained in the leaf node. Also, for example, a desired number of leaf nodes may be used as the positive leaf nodes in descending order of the ratio of the positive control.

<2> Prediction method of the present invention according to the 2nd embodiment of the present invention

The prediction method of the present invention is a method for predicting the presence or absence of the objective constituent for the test substance. The prediction can be carried out by using the prediction model of the present invention. The prediction can be carried out, specifically, on the basis of the test olfactory receptor activation data of the test substance and the prediction model of the present invention. That is, the prediction method of the present invention may comprise a step of predicting the presence or absence of the objective constituent for the test substance on the basis of the test olfactory receptor activation data of the test substance and the prediction model of the present invention. This step is also referred to as "prediction step".

In addition, by predicting the presence or absence of the objective constituent for the test substance, a substance having the objective constituent can be screened. That is, the test substance predicted to have the objective constituent can be selected as the substance having the objective constituent, and thereby the substance having the objective constituent can be screened. That is, an embodiment of the prediction method of the present invention may be a method for screening the substance having the objective constituent. That is, the prediction method of the present invention may further comprise a step of selecting the test substance predicted to have the objective constituent as the substance having the objective constituent. That is, the screening method may be a method for screening the substance having the objective constituent, the method comprising a step of predicting the presence or absence of the objective constituent for the test substance on the basis of the test olfactory receptor activation data of the test substance and the prediction model, and a step of selecting the test substance predicted to have the objective constituent as the substance having the objective constituent. Also, in other words, the screening method may be a method for screening the substance having the objective constituent, the method comprising a step of predicting the presence or absence of the objective constituent for the test substance by the prediction method of the present invention, and a step of selecting the test substance predicted to have the objective constituent as the substance having the objective constituent.

The prediction method of the present invention may further comprise a step of producing the prediction model by the prediction model production method of the present invention, prior to the prediction step.

By applying the decision tree contained in the prediction model to the test olfactory receptor activation data of the test substance, the conclusion usable as an indicator for the prediction, specifically, the classification result for the presence or absence of the objective constituent in the test substance, can be outputted. Specifically, by applying the decision tree contained in the prediction model to the test olfactory receptor activation data of the test substance, the test substance can be classified into any one of the leaf nodes contained in the decision tree.

For example, when the test substance is classified into the positive leaf node, the test substance may be predicted to have the objective constituent. Also, for example, when the test substance is not classified into the positive leaf node, the test substance may be predicted not to have the objective constituent. Furthermore, when bagging is carried out, the classification results obtained from a plurality of decision trees may be comprehensively evaluated. For example, when the ratio of the number of decision trees in which the test substance is classified into the positive leaf node to the total number of decision trees is high, the test substance may be predicted to have the objective constituent. The phrase "the ratio of the number of decision trees in which a test substance is classified into a positive leaf node to the total number of decision trees is high" may mean, for example, that the ratio of the number of decision trees in which the test substance is classified into the positive leaf node to the total number of decision trees is more than 50%, 60% or more, 70% or more, 80% or more, or 90% or more.

The prediction method of the present invention may further comprise a step of evaluating a result of the prediction. That is, by evaluating the objective constituent of the test substance, it can be confirmed whether the test substance actually has the objective constituent. Specifically, for example, by evaluating the objective constituent of the test substance predicted to have the objective constituent, it can be confirmed whether the test substance actually has the objective constituent. That is, the step of evaluating a result of the prediction may be, for example, a step of confirming the presence or absence of the objective constituent for the test substance predicted to have the objective constituent. Methods for evaluating a result of the prediction is not particularly limited. The descriptions concerning the methods for obtaining the constituent data of the reference substances can be similarly applied to the methods for evaluating a result of the prediction.

(C) 3rd embodiment of the present invention

Hereinafter, the 3rd embodiment of the present invention, specifically, the prediction model production method of the present invention and the prediction method of the present invention according to the 3rd embodiment of the present invention, will be described.

<1> Prediction model production method of the present invention according to the 3rd embodiment of the present invention

The prediction model production method of the present invention is a method for producing a model for predicting the applicability to an objective aroma property for a test substance. Predicting the applicability to the objective aroma property for the test substance is hereinafter also referred to simply as "prediction". The model for predicting the applicability to the objective aroma property for the test substance is hereinafter also referred to simply as "prediction model".

<1-1> Prediction model

The prediction model is a model for predicting the applicability to the objective aroma property for the test substance. Hence, the prediction model can be used for the prediction. The prediction model can be used for the prediction, specifically, in a manner described in the prediction method of the present invention.
The prediction model may contain a regression equation. The regression equation is not particularly limited, so long as it outputs a conclusion usable as an indicator for the prediction. The prediction can be carried out on the basis of test olfactory receptor activation data of the test substance. The test olfactory receptor activation data of the test substance is hereinafter also referred to simply as "test olfactory receptor activation data". That is, the regression equation may be one that outputs a conclusion usable as an indicator for the prediction on the basis of the test olfactory receptor activation data, i.e., by using the test olfactory receptor activation data as a variable. Examples of the conclusion usable as an indicator for the prediction include prediction value of the applicability to the objective aroma property in the test substance. That is, the regression equation may be, for example, one that outputs a prediction value of the applicability to the objective aroma property in the test substance on the basis of the test olfactory receptor activation data. The regression equation may be, for example, a linear regression equation.

<1-2> Applicability to objective aroma property

The phrase "objective aroma property" refers to an aroma property to which the applicability is to be predicted.
The aroma and the aroma property are as described in the 1st embodiment of the present invention.
The phrase "applicability to an aroma property" refers to a qualitative closeness to a target aroma property.

That is, the phrase "the applicability to an aroma property is high" means possession of a property of exhibiting an aroma close to a target aroma itself. For example, the phrase "the applicability to an aroma property "STARWBERRY" is high" means possession of a property of exhibiting an aroma close to STARWBERRY itself. The phrase "the applicability to an aroma property is high" is also referred to as "having a high applicability to an aroma property". Examples of the applicability to an aroma property include the "percentage of applicability" value calculated according to the criteria described in Atlas of odor character profiles (Dravnieks, A., ASTM data series publication, DS 61, PCN 05-061000-36, 1985). The "percentage of applicability" value can be obtained, specifically, as a score ranging from 0 to 100 by evaluating the intensity of a target aroma in a target substance on a scale of 6 (0-5 points: 0, Absent; 1, Slightly; 3, Moderately; and 5, Extremely) by a plurality of expert panels and calculating the synergistic average of "the ratio (%) of the expert panel(s) providing a score of 1 or higher" and "the average of the scores of all of the expert panels divided by 5".

The aroma may be one kind of aroma, or may be a combination of two or more kinds of aromas. That is, the phrase "applicability to an aroma property" may refer to the applicability to any one kind of aroma property, or may refer to the respective applicabilities to two or more kinds of aroma properties.

<1-3> Test substance

The phrase "test substance" refers to a substance for which the applicability to the objective aroma property is to be predicted. In other words, the phrase "test substance" refers to a substance to be used as a candidate for the substance having a high applicability to the objective aroma property in the method of screening the substance having a high applicability to the objective aroma property. The test substance is not particularly limited, so long as the test olfactory receptor activation data thereof is available.

The phrase "test olfactory receptor activation data of a test substance" refers to data related to activation of the test olfactory receptor by the test substance. The phrase "activation of a test olfactory receptor by a test substance" may be used interchangeably with the phrase "response of a test olfactory receptor to a test substance". Examples of the test olfactory receptor activation data include data indicating the presence or absence of activation of the test olfactory receptor by the test substance and data indicating the degree of activation of the test olfactory receptor by the test substance. Particular examples of the test olfactory receptor activation data include data indicating the degree of activation of the test olfactory receptor by the test substance. The phrase "degree of activation of a test olfactory receptor by a test substance" may refer to an intensity with which the test substance activates the test olfactory receptor. The test olfactory receptor activation data is used, specifically, by assigning the same as a variable into the regression equation.

The phrase "test olfactory receptor" refers to an olfactory receptor to be used in the regression equation. The phrase "an olfactory receptor is used in a regression equation" may mean that the test olfactory receptor activation data for this olfactory receptor, i.e., data related to activation of this olfactory receptor by the test substance, is used by being assigned as a variable into the regression equation. Examples of the test olfactory receptor include the following olfactory receptors. The test olfactory receptor may be one kind of olfactory receptor, or may be a combination of two or more kinds of olfactory receptors.

The olfactory receptor and a gene encoding the same (olfactory receptor gene) are as described in the 1st embodiment of the present invention.

The test olfactory receptor activation data may or may not be known. When the test olfactory receptor activation data is not known, it is sufficient to obtain the test olfactory receptor activation data as required prior to carrying out the prediction. Methods for obtaining the test olfactory receptor activation data are not particularly limited. The test olfactory receptor activation data can be obtained, for example, by known methods for identifying the presence or absence or degree of activation of olfactory receptors by substances. The test olfactory receptor activation data can be obtained, specifically, for example, by bringing the test olfactory receptor into contact with the test substance and measuring the presence or absence or degree of activation of the test olfactory receptor by the contact with the test substance. Contact between the test olfactory receptor and the test substance and measurement of the presence or absence or degree of activation of the test olfactory receptor by the contact can be carried out, for example, by referring to methods for screening substances exhibiting an objective aroma using a response of olfactory receptors as an indicator (e.g., JP 2019-037197 A). The test olfactory receptor may be used, for example, in the form carried on cells such as animal cells. Activation of the test olfactory receptor can be measured, for example, on the basis of an increased in the intracellular calcium level or the intracellular cAMP level as an indicator. Examples of methods for measuring the intracellular cAMP level include, for example, ELISA and reporter assay. Examples of the reporter assay include, for example, luciferase assay. According to the reporter assay, the intracellular cAMP level can be measured by using a reporter gene (e.g., luciferase gene) configured to be expressed in a cAMP level-dependent manner. Examples of methods for measuring the intracellular calcium level include, for example, calcium imaging.

Contact between the test olfactory receptor and the test substance and measurement of the presence or absence or degree of activation of the test olfactory receptor by the contact can be carried out, specifically, for example, according to the following procedure.

That is, the presence or absence or degree of activation of the test olfactory receptor by the test substance can be determined by bringing the test olfactory receptor into contact with the test substance and using an activation degree D1, which is the degree of activation of the test olfactory receptor upon carrying out the contact (i.e., under conditions of bringing the test olfactory receptor into contact with the test substance), as an indicator. The concentration of the test substance in contact with the test olfactory receptor can be set according to various conditions, such as the type of the test olfactory receptor and the type of the test substance. The concentration of the test substance in contact with the test olfactory receptor may be, for example, 3 to 1000 $\mu$M. The concentration of the test substance in contact with the test olfactory receptor may be, typically, 300 $\mu$M. For a test substance cytotoxic at 300 $\mu$M, the concentration of the test substance in contact with the test olfactory receptor may be, for example, 3 $\mu$M, 10 $\mu$M, 30 $\mu$M, or 100 $\mu$M.

The presence or absence or degree of activation of the test olfactory receptor by the test substance can be determined, specifically, by comparing the activation degree D1 and an activation degree D2, which is the degree of activation of the test olfactory receptor under control conditions. Examples of the control conditions include conditions of not bringing the test olfactory receptor into contact with the test substance.

The activation degree D1 and the activation degree D2 each can be obtained and used as data reflecting a parameter that acts as an indicator of activation of the test olfactory receptor. Examples of the parameter that acts as an indicator of activation of the test olfactory receptor include intracellular calcium level and intracellular cAMP level. Examples of data reflecting the intracellular cAMP level in the case of the luciferase assay include luminescence intensity. The data reflecting a parameter that acts as an indicator of activation of the test olfactory receptor can be used as it is, or can be subject to processing such as correction as required and then used.

When the activation degree D1 is high, it may be judged that the test olfactory receptor was activated by the test substance. For example, when the ratio of the activation degree D1 to the activation degree D2 (i.e., D1/D2) is 1.5 or more, 2 or more, 3 or more, 5 or more, 10 or more, 20 or more, 50 or more, or 100 or more, it may be judged that the test olfactory receptor was activated by the test substance. Examples of the ratio of the activation degree D1 to the activation degree D2 include the "normalized response" value described in Examples.

Furthermore, the degree of activation of the test olfactory receptor by the test substance can be determined on the basis of a comparison result between the activation degree D1 and the activation degree D2 as an indicator. For example, the ratio of the activation degree D1 to the activation degree D2 (i.e., D1/D2) can be regarded as the degree of activation of the test olfactory receptor by the test substance. Examples of the ratio of the activation degree D1 to the activation degree D2 include the "normalized response" value described in Examples.

The test substance may be a known substance or a novel substance. The test substance may be a natural substance or an artificial substance. The test substance may be, for example, a compound library produced using combinatorial chemistry techniques. Examples of the test substance include, for example, alcohols, ketones, aldehydes, ethers, esters, hydrocarbons, sugars, organic acids, nucleic acids, amino acids, peptides, and other various organic or inorganic ingredients. Furthermore, particular examples of the test substance include existing food additives. The phrase "existing food additive" refers to a substance that have already been approved for use as a food additive. As the test substance, one kind of test substance may be used, or a combination of two or more kinds of test substances may be used. The test substance may be selected so as to include such a substance as exemplified above, such as existing food additives. That is, as the test substance, for example, one kind of existing food additive may be used, a combination of two or more kinds of food additives may be used, or a combination of one or more kinds of food additives and one or more kinds of other substances may be used. The phrase "a combination of two or more kinds of test substances is used" refers to predicting the applicability to the objective aroma property for each of the two or more kinds of test substances.

In an embodiment, the test substance may be a mixture.

When the test substance is a mixture, the phrase "the presence or absence or degree of activation of a test olfactory receptor by a test substance" refers to the presence or absence or degree of activation of the test olfactory receptor by the whole of the mixture, regardless of the presence or absence or degree of activation of the test olfactory receptor by each of substances constituting the mixture.

When the test substance is a mixture, the phrase "applicability to an objective aroma property in a test substance" refers to the applicability to an objective aroma property in the whole of the mixture, regardless of the applicability to an objective aroma property in each of substances constituting the mixture. That is, for example, when the test substance is a mixture, the phrase "a test substance has a high applicability to an objective aroma property" means that the mixture as a whole has a high applicability to the objective aroma property, regardless whether or not each of substances constituting the mixture has a high applicability to the objective aroma property.

<1-4> Generation of regression equation

The regression equation can be generated by machine learning. That is, the prediction model production method of the present invention may comprise a step of generating the regression equation by machine learning. This step is also referred to as "regression equation generation step".

Conditions for the machine learning are not particularly limited, so long as the regression equation with which the prediction can be carried out with the desired accuracy is obtained.

The machine learning can be carried out by using a data set containing aroma property data and reference olfactory receptor activation data of reference substances. The aroma property data of the reference substances is hereinafter also referred to simply as "aroma property data". The reference olfactory receptor activation data of the reference substances is hereinafter also referred to simply as "reference olfactory receptor activation data".

The machine learning can be carried out, for example, by using the aroma property data as the objective variable and using the reference olfactory receptor activation data as the explanatory variable.

Methods for the machine learning are not particularly limited, so long as the regression equation is generated. Examples of the methods for the machine learning include regression analysis. Examples of the regression analysis include single regression analysis and multiple regression analysis. Particular examples of the regression analysis include multiple regression analysis. Examples of the regression analysis by which a linear regression equation can be generated include linear regression analysis. Examples of the linear regression analysis include single linear regression analysis and multiple linear regression analysis. Particular examples of the linear regression analysis include multiple linear regression analysis.

The machine learning may be carried out, for example, by ensemble learning. Examples of the ensemble learning include bagging and boosting. When the machine learning is carried out by ensemble learning, the regression equation contained in the prediction model may be the regression equation after the ensemble learning. That is, for example, when bagging is carried out, the prediction model may contain a plurality of regression equations obtained by the bagging. In this case, a plurality of regression equations can be used in combination in the prediction step. That is, according to bagging, a plurality of regression equations can be generated as weak learners, and a combination of such a plurality of weak learners can be used as a strong learner. Also, for example, when boosting is carried out, the prediction model may contain a regression equation whose learning level has been improved by the boosting. That is, according to boosting, a regression equation as a strong learner can be generated and used on the basis of a regression equation generated as a weak learner.

The phrase "reference substance" refers to a substance that can be used for generation of the regression equation as an indicator of the applicability to the objective aroma property. The reference substances are not particularly limited, so long as the aroma property data and reference olfactory receptor activation data thereof are available.

The phrase "aroma property data of reference substances" refers to data indicating the applicability to the objective aroma property in the reference substances. Examples of the data indicating the applicability to the objective aroma property in the reference substances include the "percentage of applicability" value calculated according to the criteria described in Atlas of odor character profiles (Dravnieks, A., ASTM data series publication, DS 61, PCN 05-061000-36, 1985).

The phrase "reference olfactory receptor activation data of reference substances" refers to data related to activation of the reference olfactory receptors by the reference substances. Examples of the reference olfactory receptor activation data include data indicating the presence or absence of activation of the reference olfactory receptors by the reference substances and data indicating the degree of activation of the reference olfactory receptors by the reference substances. Particular examples of the reference olfactory receptor activation data include data indicating the degree of activation of the reference olfactory receptors by the reference substances.

The phrase "reference olfactory receptor" refers to an olfactory receptor to be used for generation of the regression equation. The phrase "an olfactory receptor is used for generation of a regression equation" may mean that the reference olfactory receptor activation data for this olfactory receptor, i.e., data related to activation of this olfactory receptor by the reference substances, is used for generation of the regression equation. Examples of the reference olfactory receptors include the aforementioned olfactory receptors. That is, the reference olfactory receptors may include the aforementioned olfactory receptor(s). For example, 50% or more, 60% or more, 70% or more, 80% or more, 90% or more, or 95% or more of the total number of the reference olfactory receptors may be selected from the aforementioned olfactory receptors. As the reference olfactory receptors, a combination of two or more kinds of olfactory receptors including the test olfactory receptor is used. The reference olfactory receptors may consist of the test olfactory receptor, or may include, in addition to the test olfactory receptor, other olfactory receptor(s). In other words, a part or all of the reference olfactory receptors is selected as the test olfactory receptor. That is, the olfactory receptor to be used in the regression equation among the reference olfactory receptors is selected as the test olfactory receptor. In other words, the machine learning may be carried out by using the reference olfactory receptor activation data for a part or all of the reference olfactory receptors as the explanatory variable. That is, the phrase "machine learning is carried out by using reference olfactory receptor activation data as an explanatory variable" may mean that the machine learning is carried out by using the reference olfactory receptor activation data for a part or all of the reference olfactory receptors as the explanatory variable. For example, an olfactory receptor providing a high correlation coefficient between the aroma property data and the reference olfactory receptor activation data among the reference olfactory receptors may be selected as the test olfactory receptor. In other words, the machine learning may be carried out by using the reference olfactory receptor activation data for the olfactory receptor providing a high correlation coefficient between the aroma property data and the reference olfactory receptor activation data among the reference olfactory receptors as the explanatory variable. The phrase "the correlation coefficient between aroma property data and reference olfactory receptor activation data is high" may mean, for example, that the absolute value of the correlation coefficient between the aroma property data and the reference olfactory receptor activation data is more than 0.1, more than 0.15, more than 0.2, more than 0.25, or more than 0.3. The olfactory receptor providing a high correlation coefficient between the aroma property data and the reference olfactory receptor activation data can be identified, for example, by calculating the correlation coefficient between the aroma property data and the reference olfactory receptor activation data. That is, the regression equation generation step may comprise, for example, a step of calculating the correlation coefficient between the aroma property data and the reference olfactory receptor activation data, prior to the machine learning.

The number of the reference olfactory receptors is not particularly limited, so long as the regression equation with which the prediction can be carried out with the desired accuracy is obtained. The number of the reference olfactory receptors can be set, for example, according to various conditions, such as the type of the objective aroma property and the method for machine learning.

The number of the reference olfactory receptors, for example, may be 50 or more, 70 or more, 100 or more, 150 or more, 200 or more, 300 or more, 400 or more, or 500 or more, may be 2000 or less, 1500 or less, 1000 or less, 500 or less, 400 or less, 300 or less, 200 or less, 150 or less, or 100 or less, or may be within a range defined as a non-contradictory combination thereof. The number of the reference olfactory receptors may be, specifically, for example, 50 to 2000, 100 to 1000, or 300 to 500.

The number of the reference olfactory receptors to be used in the regression equation, i.e., the number of the test olfactory receptors, for example, may be 10 or more, 15 or more, 20 or more, 25 or more, 30 or more, 40 or more, 50 or more, 70 or more, 100 or more, 150 or more, 200 or more, 300 or more, 400 or more, or 500 or more, may be 2000 or less, 1500 or less, 1000 or less, 500 or less, 400 or less, 300 or less, 200 or less, 150 or less, 100 or less, 70 or less, 50 or less, 40 or less, 30 or less, 25 or less, or 20 or less, or may be within a range defined as a non-contradictory combination thereof. The number of the reference olfactory receptors may be, specifically, for example, 10 to 1000, 15 to 500, or 20 to 200.

The aroma property data may or may not be known. When the aroma property data is not known, it is sufficient to obtain the aroma property data as required prior to generation of the regression equation. Methods for obtaining the aroma property data are not particularly limited. The aroma property data can be obtained, for example, by known methods for identifying the applicability to aroma properties in substances. The applicability to the objective aroma property in the reference substances can be identified, for example, by sensory evaluation by expert panels. Specifically, for example, the "percentage of applicability" value to the objective aroma property can be calculated according to the criteria described in Atlas of odor character profiles (Dravnieks, A., ASTM data series publication, DS 61, PCN 05-061000-36, 1985).

The reference olfactory receptor activation data may or may not be known. When the reference olfactory receptor activation data is not known, it is sufficient to obtain the reference olfactory receptor activation data as required prior to generation of the regression equation. Methods for obtaining the reference olfactory receptor activation data are not particularly limited. The reference olfactory receptor activation data can be obtained, for example, by known methods for identifying the presence or absence or degree of activation of olfactory receptors by substances. The reference olfactory receptor activation data can be obtained, specifically, for example, by bringing each reference olfactory receptor into contact with each reference substance and measuring the presence or absence or degree of activation of the reference olfactory receptor by the contact with the reference substance. The aforementioned descriptions concerning the contact between the test olfactory receptor and the test substance and measurement of the presence or absence or degree of activation of the test olfactory receptor by the contact can be similarly applied to the contact between the reference olfactory receptor and the reference substance and measurement of the presence or absence or degree of activation of the reference olfactory receptor by the contact.

As the reference substances, a combination of two or more kinds of substances is used.

The reference substances each may be a known substance or a novel substance. The reference substances each may be a natural substance or an artificial substance. The reference substances each may be, for example, a compound library produced using combinatorial chemistry techniques. Examples of the reference substances include, for example, alcohols, ketones, aldehydes, ethers, esters, hydrocarbons, sugars, organic acids, nucleic acids, amino acids, peptides, and other various organic or inorganic ingredients. Specific examples of the reference substances include substances whose applicability to the objective aroma property is known. Examples of the substances whose applicability to the objective aroma property is known include substances listed in Atlas of odor character profiles (Dravnieks, A., ASTM data series publication, DS 61, PCN 05-061000-36, 1985). That is, the reference substances may include the substance(s) listed in Atlas of odor character profiles. For example, 50% or more, 60% or more, 70% or more, 80% or more, 90% or more, or 95% or more of the total number of the reference substances may be selected from the substances listed in Atlas of odor character profiles.

In an embodiment, the reference substances each may be a mixture.

When the reference substances consist of a mixture, the phrase "the presence or absence or degree of activation of a reference olfactory receptor by reference substances" refers to the presence or absence or degree of activation of the reference olfactory receptor by the whole of the mixture, regardless of the presence or absence or degree of activation of the reference olfactory receptor by each of substances constituting the mixture.

When the reference substances each are a mixture, the phrase "applicability to an objective aroma property in a reference substance" refers to the applicability to the objective aroma property in the whole of the mixture, regardless of the applicability to the objective aroma property in each of substances constituting the mixture. That is, for example, when the reference substances each are a mixture, the phrase "a reference substance has a high applicability to an objective aroma property" means that the mixture as a whole has a high applicability to the objective aroma property, regardless whether or not each of substances constituting the mixture has a high applicability to the objective aroma property.

The number of the reference substances is not particularly limited, so long as the regression equation with which the prediction can be carried out with the desired accuracy is obtained. The number of the reference substances can be set, for example, according to various conditions, such as the type of the objective aroma property and the method for machine learning.

The number of the reference substances, for example, may be 30 or more, 40 or more, 50 or more, 70 or more, 100 or more, 150 or more, 200 or more, 300 or more, 400 or more, 500 or more, 600 or more, 700 or more, 800 or more, 900 or more, 1000 or more, 1500 or more, 2000 or more, 3000 or more, 5000 or more, 10000 or more, 20000 or more, 50000 or more, or 100000 or more, may be 1000000 or less, 500000 or less, 200000 or less, 100000 or less, 50000 or less, 20000 or less, 10000 or less, 5000 or less, 3000 or less, 2000 or less, 1500 or less, 1000 or less, 500 or less, 400 or less, 300 or less, 200 or less, 150 or less, 100 or less, 70 or less, or 50 or less, or may be within a range defined as a non-contradictory combination thereof. The number of the reference substances may be, specifically, for example, 30 to 1000000, 100 to 1000000, 200 to 500000, 500 to 100000, or 1000 to 20000. The number of the reference substances may be, specifically, for example, 30 to 100, 100 to 200, 200 to 500, 500 to 1000, 1000 to 2000, 2000 to 5000, 5000 to 10000, 10000 to 20000, 20000 to 50000, 50000 to 100000, or 100000 to 200000. The number of the reference substances may be, specifically, for example, 30 to 1000, 50 to 500, or 100 to 200.

<2> Prediction method of the present invention according to the 3rd embodiment of the present invention

The prediction method of the present invention is a method for predicting the applicability to the objective aroma property for the test substance. The prediction can be carried out by using the prediction model of the present invention. The prediction can be carried out, specifically, on the basis of the test olfactory receptor activation data of the test substance and the prediction model of the present invention. That is, the prediction method of the present invention may comprise a step of predicting the applicability to the objective aroma property for the test substance on the basis of the test olfactory receptor activation data of the test substance and the prediction model of the present invention. This step is also referred to as "prediction step".

In addition, by predicting the applicability to the objective aroma property for the test substance, a substance having a high applicability to the objective aroma property can be screened. That is, the test substance predicted to have a high applicability to the objective aroma property can be selected as the substance having a high applicability to the aroma property, and thereby the substance having a high applicability to the aroma property can be screened. That is, an embodiment of the prediction method of the present invention may be a method for screening the substance having a high applicability to the objective aroma property. That is, the prediction method of the present invention may further comprise a step of selecting the test substance predicted to have a high applicability to the objective aroma property as the substance having a high applicability to the objective aroma property. That is, the screening method may be a method for screening the substance having a high applicability to the objective aroma property, the method comprising a step of predicting the applicability to the objective aroma property for the test substance on the basis of the test olfactory receptor activation data of the test substance and the prediction model, and a step of selecting the test substance predicted to have a high applicability to the objective aroma property as the substance having a high applicability to the objective aroma property. Also, in other words, the screening method may be a method for screening the substance having a high applicability to the objective aroma property, the method comprising a step of predicting the applicability to the objective aroma property for the test substance by the prediction method of the present invention, and a step of selecting the test substance predicted to have a high applicability to the objective aroma property as the substance having a high applicability to the objective aroma property.

The prediction method of the present invention may further comprise a step of producing the prediction model by the prediction model production method of the present invention, prior to the prediction step.

By applying the regression equation contained in the prediction model to the test olfactory receptor activation

EP 4 130 736 A1

data of the test substance, the conclusion usable as an indicator for the prediction, specifically, the prediction value of the applicability to the objective aroma property in the test substance, can be outputted. Specifically, by applying the regression equation contained in the prediction model to the test olfactory receptor activation data of the test substance, the prediction value of the applicability to the objective aroma property in the test substance can be outputted. Furthermore, when bagging is carried out, the output results obtained from a plurality of regression equations may be comprehensively evaluated. When bagging is carried out, the phrase "prediction value of the applicability to an objective aroma property in a test substance" may refer to, for example, an average value of the prediction values of the applicability to the objective aroma property in the test substance outputted from a plurality of regression equations.

When the prediction value of the applicability to the objective aroma property in the test substance is high, the test substance may be predicted to have a high applicability to the objective aroma property in the test substance. The phrase "a prediction value of the applicability to an objective aroma property is high" may mean, for example, that the "percentage of applicability" value to the objective aroma property is 4 or more, 7 or more, 10 or more, 15 or more, or 20 or more.

The prediction method of the present invention may further comprise a step of evaluating a result of the prediction. That is, by evaluating the applicability to the objective aroma property in the test substance, it can be confirmed whether the test substance actually has a high applicability to the objective aroma property.

Specifically, for example, by evaluating the applicability to the objective aroma property of the test substance predicted to have a high applicability to the objective aroma property, it can be confirmed whether the test substance actually has a high applicability to the objective aroma property. That is, the step of evaluating a result of the prediction may be, for example, a step of confirming the applicability to the objective aroma property for the test substance predicted to have a high applicability to the objective aroma property. Methods for evaluating a result of the prediction is not particularly limited. The descriptions concerning the methods for obtaining the aroma property data of the reference substances can be similarly applied to the methods for evaluating a result of the prediction.

Examples

< Example A >

Hereinafter, the present invention will be more specifically explained with reference to non-limiting examples according to the 1st embodiment of the present invention. Although the substances used in the following Example each are referred to as "test substance", those substances can be used as reference substances in the prediction method of the present invention and the design method of the present invention.

<1> Preparation of human olfactory receptor-expressing cells

<1-1> Preparation of expression vectors of human olfactory receptors

As olfactory receptors, 352 kinds of olfactory receptors (OR1A1, OR1A2, OR1B1, OR1C1, OR1D2, OR1D5, OR1E1, OR1F1, OR1F12, OR1G1, OR1I1, OR1J1, OR1J2, OR1J4, OR1K1, OR1L1, OR1L3, OR1L4, OR1L8, OR1M1, OR1N1, OR1N2, OR1Q1, OR1R1P, OR1S1, OR2A1, OR2A2, OR2A4, OR2A5, OR2A12, OR2A14, OR2A25, OR2AE1, OR2AG1, OR2AG2, OR2AJ1P, OR2AK2, OR2AP1, OR2AT4, OR2B2, OR2B3, OR2B6, OR2B11, OR2C1, OR2C3, OR2D2, OR2D3, OR2F1, OR2G2, OR2G3, OR2G6, OR2H1, OR2H2, OR2J2, OR2J3, OR2K2, OR2L2, OR2L8, OR2L13, OR2M2, OR2M4, OR2M7, OR2S2, OR2T1, OR2T2, OR2T5, OR2T6, OR2T8, OR2T10, OR2T11, OR2T27, OR2T34, OR2V2, OR2W1, OR2W3, OR2Y1, OR2Z1, OR3A1, OR3A2, OR3A3, OR3A4, OR4A5, OR4A15, OR4A16, OR4A47, OR4B1, OR4C3, OR4C5, OR4C6, OR4C11, OR4C12, OR4C13, OR4C15, OR4C16, OR4C46, OR4D1, OR4D2, OR4D5, OR4D6, OR4D9, OR4D10, OR4D11, OR4E2, OR4F3, OR4F5, OR4F6, OR4F14P, OR4F15, OR4G11P, OR4H12P, OR4K1, OR4K2, OR4K5, OR4K13, OR4K14, OR4K15, OR4K17, OR4L1, OR4M1, OR4N2, OR4N4, OR4N5, OR4P4, OR4Q3, OR4S1, OR4S2, OR4X1, OR4X2, OR5A1, OR5A2, OR5AC2, OR5AK2, OR5AK3P, OR5AN1, OR5AP2, OR5AR1, OR5AS1, OR5AU1, OR5B2, OR5B3, OR5B12, OR5B17, OR5B21, OR5C1, OR5D13, OR5D14, OR5D16, OR5D18, OR5F1, OR5H1, OR5H2, OR5H6, OR5H14, OR5I1, OR5J2, OR5K1, OR5K3, OR5K4, OR5L2, OR5M3, OR5M8, OR5M9, OR5M10, OR5M11, OR5P3, OR5R1, OR5T1, OR5T2, OR5T3, OR5V1, OR5W2, OR6A2, OR6B1, OR6B2, OR6C1, OR6C2, OR6C3, OR6C4, OR6C6, OR6C65, OR6C66P, OR6C68, OR6C70, OR6C74, OR6C75, OR6C76, OR6F1, OR6J1, OR6K2, OR6K3, OR6K6, OR6M1, OR6N1, OR6N2, OR6P1, OR6Q1, OR6S1, OR6T1, OR6V1, OR6X1, OR6Y1, OR7A3P, OR7A5, OR7A10, OR7A17, OR7C1, OR7C2, OR7D2, OR7D4, OR7E24, OR7G1, OR7G2, OR7G3, OR8A1, OR8B3, OR8B4, OR8B8, OR8B12, OR8D1, OR8D2, OR8D4, OR8G2, OR8G5, OR8H3, OR8I2, OR8J1, OR8J3, OR8K1, OR8K3, OR8K5, OR8S1, OR8U1, OR9A4, OR9G1, OR9G4, OR9I1, OR9K2, OR9Q1, OR9Q2, OR10A3, OR10A4, OR10A5, OR10A6, OR10A7, OR10AD1, OR10AG1, OR10C1, OR10D3, OR10D4P, OR10G2, OR10G3, OR10G4, OR10G6, OR10G7, OR10G9, OR10H2, OR10H4, OR10J1, OR10J3, OR10J5, OR10K1, OR10K2, OR10P1, OR10Q1, OR10R2, OR10S1, OR10T2, OR10V1, OR10W1, OR10X1, OR10Z1, OR11A1, OR11G2, OR11H4, OR11H6, OR11H12, OR11L1, OR12D2, OR12D3, OR13A1, OR13C2, OR13C3, OR13C4, OR13C8, OR13D1, OR13F1, OR13G1, OR13H1, OR13J1, OR14A2, OR14A16, OR14C36, OR14I1, OR14J1, OR14K1, OR14L1P, OR51A1P, OR51A4, OR51A7, OR51B2, OR51B4, OR51B5, OR51B6, OR51D1, OR51E1, OR51E2, OR51F1, OR51F2, OR51F5P, OR51G1, OR51G2, OR51H1, OR51I1, OR51I2, OR51L1, OR51M1, OR51Q1, OR51S1, OR51T1, OR51V1, OR52A1, OR52A4, OR52A5, OR52B2, OR52B4, OR52B6, OR52D1, OR52E2, OR52E4, OR52E5, OR52E8, OR52H1, OR52I2, OR52J3, OR52K2, OR52L2P, OR52M1, OR52N1, OR52N2, OR52N4, OR52N5, OR52P2P, OR52R1, OR52W1, OR52Z1P, OR56A1, OR56A3, OR56A4, OR56A5, OR56B1, OR56B2P, and OR56B4) among human olfactory receptors were selected.

The 352 kinds of human olfactory receptor genes were purchased from TrueClone cDNA Clone Collection (OriGene). Fragments for subcloning of the 352 kinds of human olfactory receptor genes were amplified by PCR using primers designed based on sequence information registered in GenBank and the purchased human olfactory receptor genes as templates. The amplified fragments for subcloning of the respective genes were each subcloned downstream of the Rho tag sequence of the Rho-pME18S vector (K. Kajiya et al., Journal of Neuroscience 15 August 2001, 21 (16) 6018-6025) by using EcoRI and XhoI sites, to obtain 352 kinds of expression vectors for human olfactory receptors.

<1-2> Preparation of olfactory receptor-expressing cells

HEK293T cells expressing each of the 352 kinds of olfactory receptors were prepared according to the following procedure. The gene mixture shown in Table 1 and the transfection reagent mixture shown in Table 2 were prepared and left to stand at room temperature for 5 minutes. pcDNA3.1-microbat RTP1s is an expression vector for bat RTP1s, pcDNA3.1-Golf is an expression vector for human Golf, and pcDNA3.1-Ric8B is an expression vector for rat Ric8B (JP 2019-037197 A). The gene mixture and the transfection reagent mixture were mutually mixed, and a 12.5 $\mu$L-aliquot of the mixture was dispensed into each well of a poly-D-lysine coated 384 well plate and left to stand in a clean bench for 15 minutes. HEK293T cells seeded on the previous day into a 10 cm petri dish ($2.5 \times 10^6$ cells/10 cm petri dish) were adjusted to $1.2 \times 10^5$ cells/mL, seeded at 25 $\mu$L into each well of the 384 well plate, and cultured overnight in an incubator at 37°C, 5% $CO_2$. Thus, 352 kinds of cultures of HEK293T cells transfected with the expression vectors listed in Table 1 and appropriately expressing the genes encoded by those expression vectors were obtained.

Table 1

| OPTI-MEM (GIBCO) | 6.2 $\mu$L |
|---|---|
| Expression vector for human olfactory receptor | 0.0125 $\mu$g |
| pGL4.29[luc2P/CRE/Hygro] Vector, Promega | 0.0025 $\mu$g |
| pGL4.74[hRluc/TK] Vector, Promega | 0.00125 $\mu$g |
| pcDNA3.1-microbat RTP1s | 0.00250 $\mu$g |
| pcDNA3.1-Golf | 0.00125 $\mu$g |
| pcDNA3.1-Ric8B | 0.00125 $\mu$g |

Table 2

| OPTI-MEM (GIBCO) | 6.2 $\mu$L |
|---|---|
| Lipofectamine 2000 (Invitrogen) | 0.0425 $\mu$L |

<2> Preparation of human olfactory receptor activity database

<2-1> Luciferase assay

Responses of the olfactory receptors to test substances were measured using the olfactory receptor-expressing cells.

The 352 kinds of olfactory receptors expressed in HEK293T cells each activate adenylate cyclase in concert with Golf, to thereby increase the intracellular cAMP level. In this Example, a luciferase reporter gene assay, in which an increase in the intracellular cAMP level is monitored as an increase in a luminescence value derived from firefly luciferase, was used for measuring the responses of the olfactory receptors to test substances. The phrase "luciferase reporter gene assay" is also referred to as the "luciferase assay". The firefly luciferase is expressed from a firefly luciferase gene carried on the pGL4.29[luc2P/CRE/Hygro] Vector in an intracellular cAMP level-dependent manner. In addition, a luminescence value derived from renilla luciferase was used as an internal standard to correct for errors in gene transfer efficiency and cell number in each well. The renilla luciferase is expressed from a renilla luciferase gene carried on the pGL4.74[hRluc/TK] Vector constitutively under the control of the TK promoter.

As the test substances, 941 kinds of substances were selected from the substances listed in The Good Scents Company (http://www.thegoodscentscompany.com/). The culture medium was removed from the 352 kinds of cultures obtained in <1-2> above, and 15 $\mu$L of each of 941 kinds of test substance solutions was added to each culture, to thereby obtain 352×941 kinds of reaction solutions. The test substance solutions were each prepared by dissolving the corresponding test substance in CD293 (Life Technologies, Inc.). The concentrations of the test substances in the test substance solutions were set to 300 $\mu$M in principle. However, for some test substances that showed cytotoxicity at 300 $\mu$M, the concentrations of the test substances in the test substance solutions were set to 3 $\mu$M, 10 $\mu$M, 30 $\mu$M, or 100 $\mu$M. For a very limited number of test substances, the concentrations of test substances in the test substance solutions were set to 1000 $\mu$M. The reaction solution was placed in an incubator at 37°C, 5% $CO_2$, and cells were incubated for 4 hours, to thereby obtain a sufficient expression of the firefly luciferase gene in the cells. The luminescence value derived from the firefly luciferase in the cells was measured and designated as "Luc value". In addition, the luminescence value derived from the renilla luciferase in the cells was measured and designated as "hRLuc value". The luminescence values from the respective luciferases were measured by using Dual-Glo™ luciferase assay system (Promega) according to the product operation manual.

<2-2> Calculation of olfactory receptor activity

The luminescence value derived from the firefly luciferase induced by the test substance stimulation (Luc value) was divided by the luminescence value derived from the renilla luciferase (hRluc value) in the same well, to thereby obtain the "Luc/hRluc value". The Luc/hRluc value in cells subject to the test substance stimulation was divided by the Luc/hRluc value in cells not subject to the test substance stimulation, to thereby obtain the "fold increase". Furthermore, the fold increase in cells introduced with the olfactory receptor expression vector was divided by the fold increase in cells introduced with the empty vector Rho-pME18S, to thereby obtain the "normalized response". The logarithm of the normalized response was defined as the "olfactory receptor activity", which is a quantitative indicator of the intensity of the response of olfactory receptor to the test substance. Hereinafter, when referring to the olfactory receptor activity as -1, 0, or 1, it is assumed that the logarithm of the normalized response is -1, 0, or 1, i.e., that the normalized response is 0.1, 1, or 10, respectively, which indicates that the response of the olfactory receptor-introduced cells to the test substance stimulation was 1/10, 1, and 10 times stronger than the response of the empty vector-introduced cells to the test substance stimulation, respectively. For the sake of simplicity, possible effects of different concentrations of the test substances in the test substance solutions on the olfactory receptor activity were ignored.

<2-3> Molecular structure information of test substances

The isomeric SMILES of the test substances were obtained from PubChem (https://pubchem.ncbi.nlm.nih.gov/). The isomeric SMILES were canonicalized, then converted to 3D structural data, and saved in the SDF format, by using open source chemoinformatics software RDKit (http://www.rdkit.org).

<2-4> Aroma property information of test substances

As the aroma property information of the test substances, the descriptors listed in "Odor Description" of "Organoleptic Properties" in The Good Scents Company (http://www.thegoodscentscompany.com) were excerpted.

<3> Scoring of similarity of stereochemical structure in which multiple conformations are taken into account

<3-1> Generation of multiple conformations of aroma compounds

The SDF data obtained in <2-3> above was subject to hydrogenation and optimization of the structural data under the condition of pH 7.0 by using an integrated computational chemistry system MOE (CCG). The multiple conformations were generated by using conformation generating software OMEGA (OpenEye) with OMEGA macrocyclic for macrocyclic compounds or with OMEGA classic for other compounds.

<3-2> Calculation of similarity of stereochemical structure

The similarity for which the surface shape and the surface chemical property are focused on was calculated for all conformation pairs of all test substances generated in <3-1> above by using molecular surface shape similarity calculation software ROCS (OpenEye). The maximum value of the similarities among all conformation pairs between the test substances was used as the similarity of stereochemical structure between those substances. Due to the specification of the similarity calculation by ROCS, different similarity values may be calculated depending on which substance of the conformation pair is used as the query. In such cases, the average value of the two values is used as the similarity of the pair to obtain a symmetric matrix, and finally, a stereochemical structural similarity matrix among all test substances in which multiple conformations are taken into account was obtained.

<4> Molecular structure representation based on stereochemical structural similarity in which multiple conformations are taken into account

<4-1> Cluster analysis using stereochemical structural similarity in which multiple conformations are taken into account

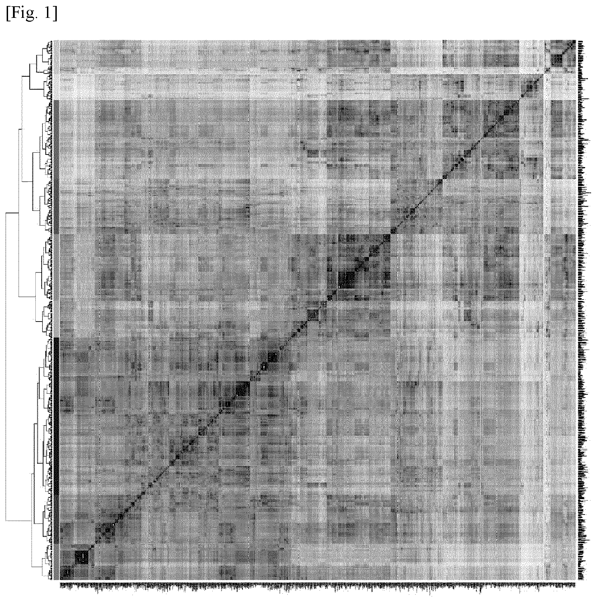

The stereochemical structural similarity matrix among all test substances in which multiple conformations are taken into account was considered as a matrix consisting of multidimensional feature vectors of stereochemical structure information for the respective test substances to calculate the Euclidean distance between each pair of the test substances, and a hierarchical cluster analysis was carried out by the Ward method. According to the results of the hierarchical cluster analysis, the stereochemical structural similarity matrix was rearranged, and a heat map indicating the degree of similarity was created (Fig. 1). On the left side of the heat map, a dendrogram generated by the hierarchical cluster analysis and results of classification of all test substances into nine clusters based on the dendrogram with color shade are shown.

<4-2> Visualization of stereochemical structural similarity matrix in which multiple conformations are taken into account by dimensionality reduction

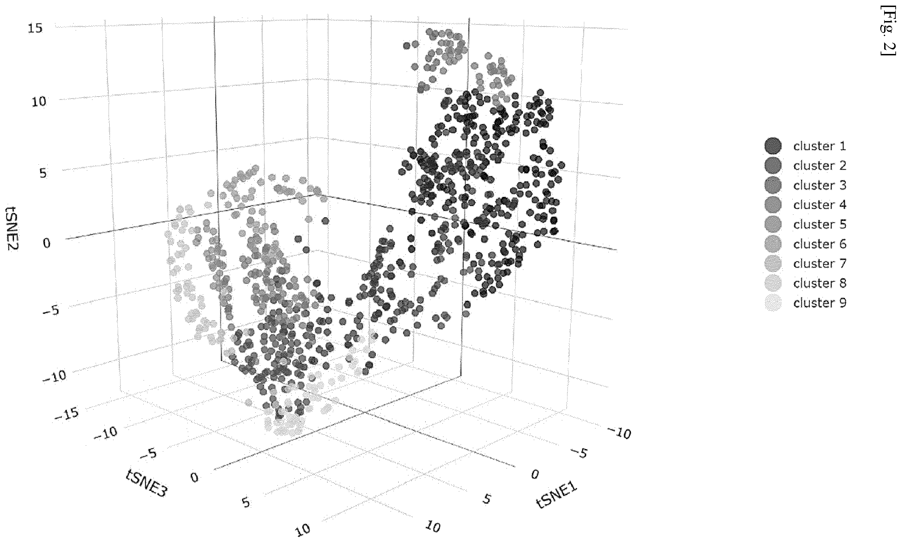

The stereochemical structural similarity matrix among all test substances in which multiple conformations are taken into account was considered as a matrix consisting of multidimensional feature vectors of stereochemical structure information for the respective test substances to carry out visualization reflecting the steric structural similarity relationships among the test substances by a dimensionality reduction method. The t-distributed Stochastic Neighbor Embedding method (t-SNE, Van der Maaten et al., 2008, Visualizing Data Using t-SNE, Journal of Machine Learning Research 9: 2579-2605) was used as the dimensionality reduction method, and results obtained by plotting all the test substances in a three-dimensional space are shown in Fig. 2. The shading of each point is the same as that of the clustering result in <4-1> above. In this three-dimensional map (hereinafter referred to as "chemical structural similarity space"), compounds with close stereochemical structures are placed near each other and compounds with distant stereochemical structures are placed far apart.

<4-3> Olfactory receptor activation property in stereochemical structural similarity space

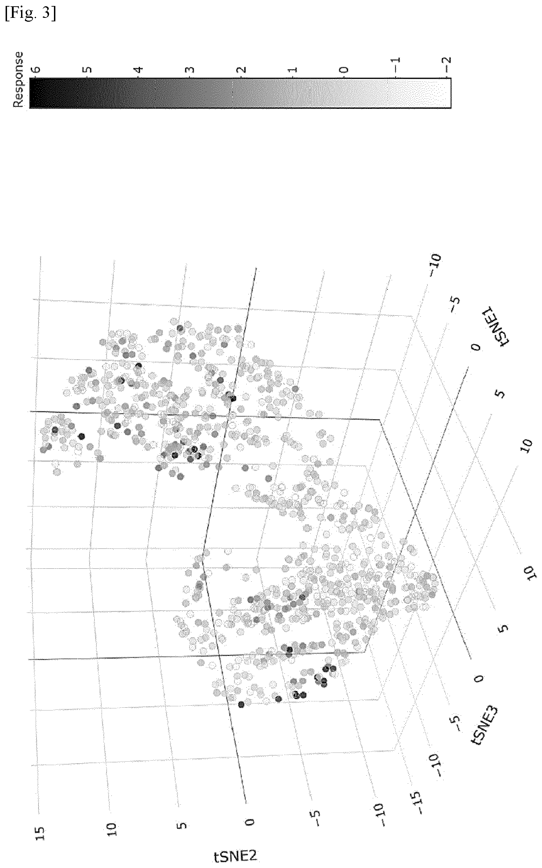

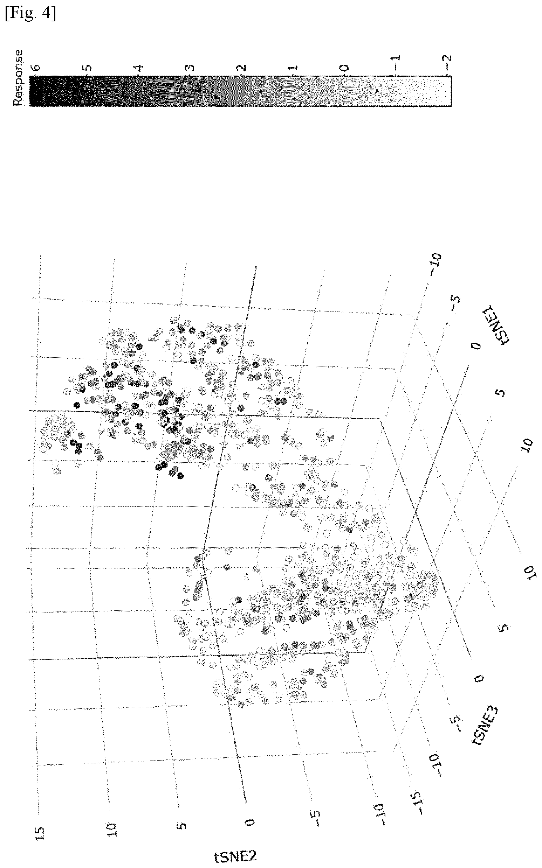

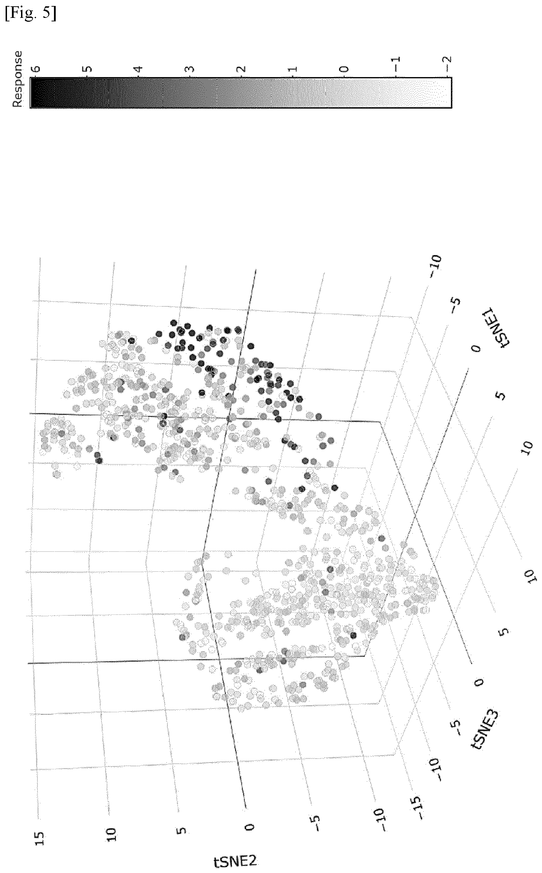

The points representing the respective test substances in the chemical structural similarity space generated in <4-2> above were color-coded on the basis of the degree of the olfactory receptor activity calculated in <2-2> above and converted to a heat map (Figs. 3-5). In the figures, the higher the olfactory receptor activity, the blacker the points representing the respective test substances are shown in, and the lower the olfactory receptor activity, the whiter the points representing the respective test substances are shown in. In the figures, "Response" indicates the olfactory receptor activity. Fig. 3 shows the results color-coded according to the degree of OR4S2 activity (i.e., olfactory receptor activity for the olfactory receptor OR4S2). Fig. 4 shows the results color-coded according to the degree of OR5K1 activity (i.e., olfactory receptor activity for the olfactory receptor OR5K1). Fig. 5 shows the results color-coded according to the degree of OR10G4 activity (i.e., olfactory receptor activity for the olfactory receptor OR10G4). Figs. 3-5 show that black dots are localized in a narrow range, i.e., the test substances exhibiting each olfactory receptor activation property is localized in a narrow range, in the stereochemical structural similarity space. Hence, it was revealed that the olfactory receptor activation properties of substances can be predicted by using as an indicator the stereochemical structural similarity in which multiple conformations are taken into account.

<4-4> Aroma property in stereochemical structural similarity space

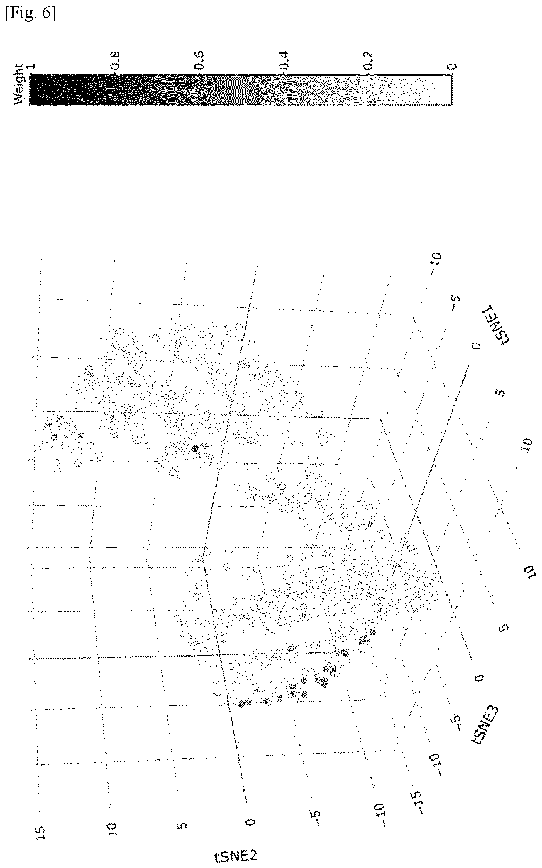

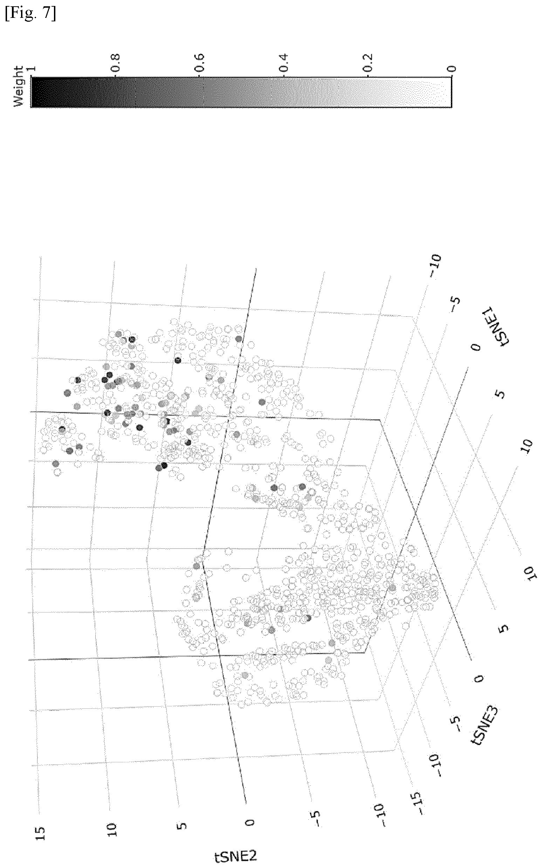

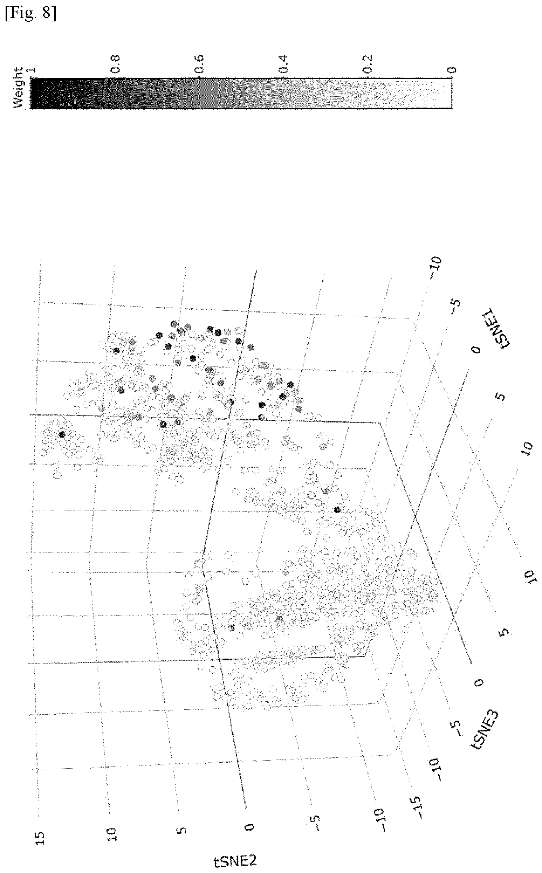

The points representing the respective test substances in the chemical structural similarity space generated in <4-2> above were color-coded on the basis of the presence or absence of the aroma property obtained in <2-4> above (Figs. 6-8). In the figures, the higher the order of appearance of the descriptor indicating the aroma property, the blacker the points representing the respective test substances are shown in, and the lower the order of appearance of the descriptor indicating the aroma property, the whiter the points representing the respective test substances are shown in. The phrase "order of appearance of a descriptor indicating an aroma property" refers to the listing order of the descriptor indicating the aroma property for each test substance in "Odor Description" of "Organoleptic Properties" in The Good Scents Company (http://www.thegoodscentscompany.com). In the figures, "Weight" indicates the square root of the reciprocal of the order of appearance of the descriptor indicating the aroma property, whereas it is set to 0 when no descriptor indicating the aroma property appears. Fig. 6 shows the results color-coded according to the presence or absence of the aroma property "onion". Fig. 7 shows the results color-coded according to the presence or absence of the aroma property "nutty". Fig. 8 shows the results color-coded according to the presence or absence of the aroma property "phenolic". Figs. 6-8 show that black dots are localized in a narrow range, i.e., the test substances exhibiting each aroma property is localized in a narrow range, in the stereochemical structural similarity space. Hence, it was revealed that the aroma properties of substances can be predicted by using as an indicator the stereochemical structural similarity in which multiple conformations are taken into account.

<5> Discussion

From this Example, it is considered that even aroma compounds that at first glance do not have similar structural formulas or similar most stable conformations, if they have conformations containing a common molecular surface shape and/or chemical property, they are likely to activate a common olfactory receptor and exhibit a common aroma. This is considered to be due to that after an aroma compound is dissolved into the olfactory mucus, it forms a variety of conformations (multiple conformations) via rotation around a single-bonded part, and activates an active site of the olfactory receptor in the form of an appropriate conformation. That is, although many aroma compounds have been reported to activate a plurality of kinds of olfactory receptors, it is considered that there is no necessity for a certain aroma compound to take the same conformation when it binds to an active site of a certain olfactory receptor and when it binds to an active site of another olfactory receptor. Information on the multiple conformations of aroma compounds is considered to be important for understanding the many-to-many combinatorial coding encoded by aroma compounds and olfactory receptors. That is, the present invention is expected to enable accurate prediction of the presence or absence of aroma properties or olfactory receptor activation properties in substances, which has not been possible by existing methods that ignore information on multiple conformations.

<6> Comparison between stereochemical structural similarity and existing method and investigation of mixing method

Comparison between the method of this Example and an existing method was carried out using the data on the similarity of odors between single substances among the results of sensory evaluation carried out in Non-Patent Document 1. In addition, a mixed method of both the methods was also investigated.

<6-1> Odor similarity in sensory evaluation

There were 83 cases of odor similarity data between single substances in Non-Patent Document 1. When the results of similarity evaluations between the same substances were excluded, there remained 77 cases. Among these, data of 17 cases in total, 9 cases having similarity values greater than 55 and 8 cases having similarity values less than 16, were referenced. The similarity evaluation of odors were carried out by visual analog scale method in Non-Patent Document 1, and the similarity was expressed as 0 (not at all similar) to 100 (very similar).

<6-2> Test Substances

The 25 kinds of compounds used in the data of the 17 cases narrowed down in <6-1> above were used.

<6-3> Calculation of similarity of stereochemical structure in which multiple conformations are taken into account

The stereochemical structural similarity in which multiple conformations are taken into account was calculated for the test Substances of <6-2> above in the same manner as in <3-1> and <3-2> above.

<6-4> Calculation of molecular fingerprint (MACCS Keys) similarity

MACCS Keys (155 bits) were generated by using Canvas (Schroedinger) from the SDF data obtained in <2-3> above. The Tanimoto similarity of the MACCS Keys between the test substances was calculated and used as the molecular fingerprint similarity.

<6-5> Mixing of stereochemical structural similarity and molecular fingerprint similarity

The stereochemical structural similarity of <6-3> above was calculated within a range of 0 to 2, and the molecular fingerprint similarity of <6-4> above was calculated in a range of 0 to 1. Hence, the stereo-chemical structural similarity was multiplied by a factor of 1/2, to thereby align the ranges of the similarities calculated by both the methods. A weighted average was calculated by mixing the similarities calculated by both the methods in the ratio of 100:0, 90:10, 80:20, 70:30, 60:40, 50:50, 40:60, 30:70, 20:80, 10:90, or 0:100.

<6-6> Comparison among stereochemical structural similarity, molecular fingerprint similarity, and mixing method

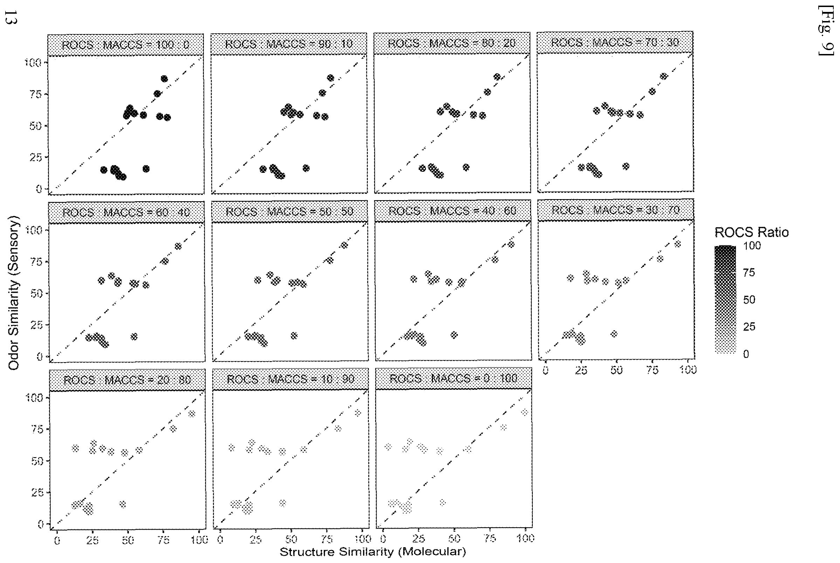

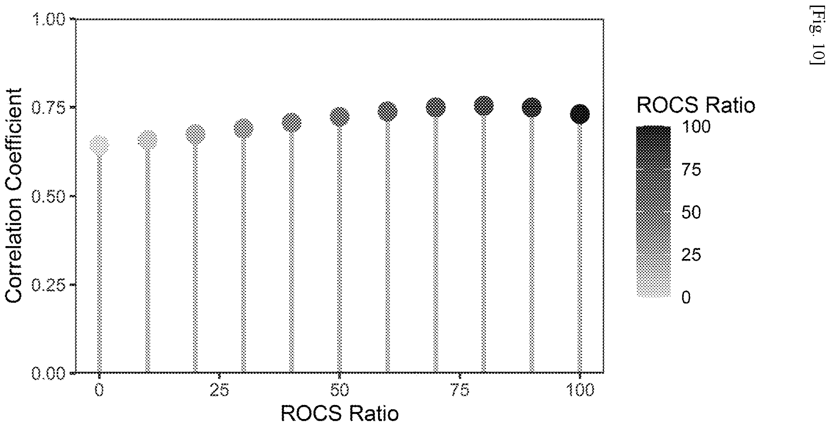

A scatter diagram in which the odor similarity referred to in <6-1> above was plotted on the y-axis and the weighted average of the stereochemical structural similarity and the molecular fingerprint similarity calculated in <6-5> above was placed on the x-axis is shown in Fig. 9, and the correlation coefficients between the odor similarity and the weighted average for each mixing ratio is shown in Fig. 10. In the figures, "ROCS" represents the stereochemical structural similarity and "MACCS" represents the molecular fingerprint similarity. Comparison of the correlation coefficients for the respective mixing ratios revealed that the method in which the stereochemical structural similarity and the molecular fingerprint similarity were mixed at a ratio of 80:20 (ROCS Ratio = 80) showed the highest correlation with sensory function (Fig. 10). In addition, comparison of the correlation coefficients for the mixing ratios of 100:0 and 0:100 revealed that the stereochemical structural similarity showed a higher correlation with sensory function than the molecular fingerprint similarity (Fig. 10).

< Example B >

Hereinafter, the present invention will be more specifically explained with reference to non-limiting examples according to the 2nd embodiment of the present invention. Although the substances used in the following Example each are referred to as "test substance", those substances can be used as reference substances in the prediction model production method of the present invention and the prediction method of the present invention.

<1> Preparation of human olfactory receptor-expressing cells

<1-1> Preparation of expression vectors of human olfactory receptors

The 352 kinds of expression vectors for human olfactory receptors were obtained according to the same procedure as in <1-1> in Example A.

<1-2> Preparation of olfactory receptor expressing cells

The 352 kinds of cultures of HEK293T cells expressing the 352 kinds of olfactory receptors, respectively, were obtained according to the same procedure as in <1-2> in Example A.

<2> Preparation of human olfactory receptor activity database

<2-1> Luciferase assay

The luciferase assay was carried out according to the same procedure as in <2-1> in Example A except that 1097 kinds of substances were selected from the substances listed in The Good Scents Company (http://www.thegoodscentscompany.com/) as the test substances.

<2-2> Calculation of olfactory receptor activity

The olfactory receptor activity was calculated according to the same procedure as in <2-2> in Example A.

<2-3> Aroma property information

As the aroma property information of the test substances, the descriptors listed in "Odor Description" of "Organoleptic Properties" in The Good Scents Company (http://www.thegoodscentscompany.com) were excerpted.

<3> Identification of olfactory receptor activity pattern characteristic for target aroma property

A tree model was constructed by CART (L.Breiman, J.H.Friedman, R.A.Olshen and C.J. Stone, "Classification and Regression Trees", (Chapman and Hall, CRC, 1984)) using a flagged value of the presence or absence of the descriptor indicating the aroma property obtained in <2-3> above (presence, 1; absence, 0) was used as the objective variable and the olfactory receptor activity calculated in <2-2> above as the explanatory variable.

The tree model is an algorithm that sequentially searches for branching conditions from the explanatory variable, which conditions enable the best split of data with respect to the objective variable. A result of the analysis returns a concise rule such as "if A, then B", and the rule can be illustrated in a tree structure, which is characterized by the ease of interpretation of the result. The Gini impurity was used as the statistic for basis of the split (an indicator objectively indicating how "cleanly" the data is split). For a node t of the tree model, if the number of samples in the node t is Nt, the number of categories in the node t is c, and the number of samples belonging to a category i in the node t is Ni, the Gini impurity I(t) at the node t is expressed by the following formula.

$$\text{Gini impurity at node } t \quad I(t) = 1 - \sum_{i=1}^{c} \left(\frac{N_i}{N}\right)^2$$

In this case, an information gain $IG(D_p, f)$ obtained by dividing a parent node $D_p$ into two child nodes $D_{left}$ and $D_{right}$ with respect to a feature f is expressed by the following formula. $N_p$, $N_{left}$, and $N_{right}$ are the numbers of samples contained in the nodes $D_p$, $D_{left}$, and $D_{right}$, respectively.

Information gain obtained by split of node Dp

$$IG(D_p, f) = I(D_p) - \frac{N_{left}}{N_p} I(D_{left}) - \frac{N_{right}}{N_p} I(D_{right})$$

The feature f that maximizes the information gain $IG(D_p, f)$ is adopted as the branching condition at the node $D_p$, and this step is repeated in turn until the amount of the information gain reaches below a certain level.

<3-1> Aroma property "burnt"

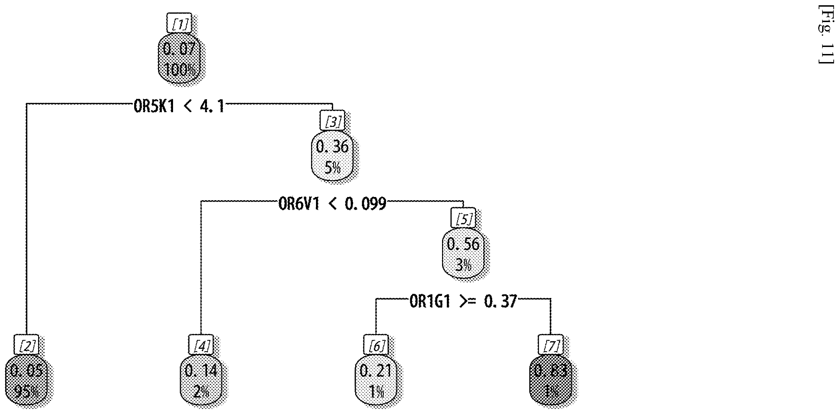

Compounds with burnt or roasted as the descriptors listed in "Odor Description" of "Organoleptic Properties" in The Good Scents Company were flagged and used for construction of a tree model, to thereby identify olfactory receptor activity patterns characteristic for compounds with the aroma property "burnt" (Fig. 11). The results of the tree model read as follows (the same shall apply for the subsequent experiments). The ellipses shown at the bottom are each called "leaf", and the other ellipses are each called "node". The numbers in [ ] above the ellipses are the identification numbers of the nodes and leaves. Of the two numbers above and below the ellipse, the lower number represents the ratio of the number of compounds contained in the node or leaf to the total number of compounds used in the analysis. The upper number represents the average value of the objective variable for the compounds contained in the node or leaf. In this analysis, 1 was given to compounds with the aroma property "burnt" (descriptor of burnt or roasted) and 0 was given to compounds without the same, and hence, the average value of the objective variables in Fig. 11 represents the percentage of compounds with the aroma property "burnt". The branching condition is indicated below the ellipse of each node. Compounds that satisfy the condition are classified into the bottom-left node or leaf, and compounds that do not are classified into the bottom-right node or leaf. Each compound is repeatedly subject to the conditional branching until it reaches a leaf. A main olfactory receptor activity pattern identified was "OR5K1 activity of 4.10 or more, and OR6V1 activity of 0.10 or more, and OR1G1 activity of less than 0.37" (identification number 7). That is, it can be predicted that substances classified into leaf with the identification number 7 are likely to have the aroma property "burnt".

<3-2> Aroma property "sweet"

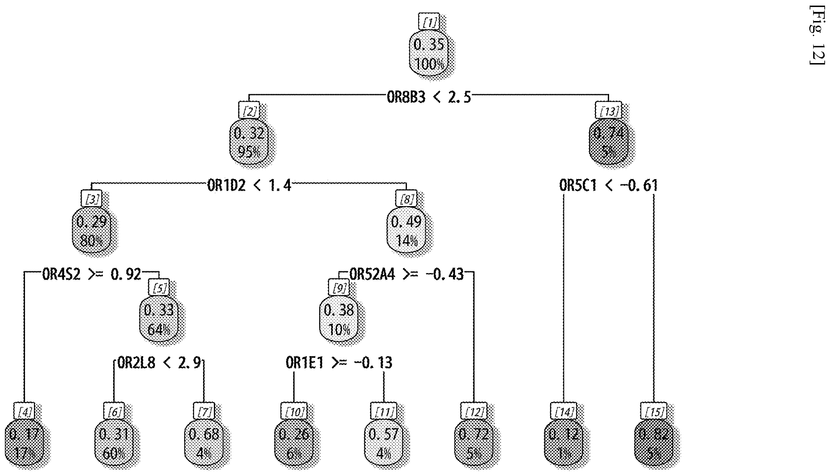

Compounds with sweet as the descriptors listed in "Odor Description" of "Organoleptic Properties" in The Good Scents Company were flagged and used for construction of a tree model, to thereby identify olfactory receptor activity patterns characteristic for compounds with the aroma property "sweet" (Fig. 12). Main olfactory receptor activity patterns identified were "OR8B3 activity of 2.50 or more, and OR5C1 activity of -0.61 or more" (identification number 15), "OR8B3 activity of less than 2.50, and OR1D2 activity of 1.40 or more, and OR52A4 activity of less than - 0.43" (identification number 12), "OR8B3 activity of less than 2.50, and OR1D2 activity of 1.40 or more, and OR52A4 activity of -0.43 or more, and OR1E1 activity of less than -0.13" (identification number 11), and "OR8B3 activity of less than 2.50, and OR1D2 activity of less than 1.40, and OR4S2 activity of less than 0.92, and OR2L8 activity of 2.90 or more" (identification number 7). That is, it can be predicted that substances classified into leaf with the identification number 15, 12, 1, or 7 are likely to have the aroma property "sweet".

<3-3> Aroma property "nutty"

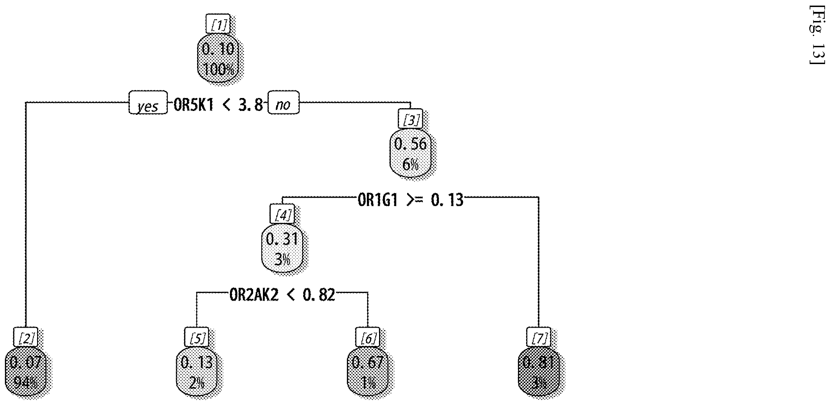

Compounds with nutty as the descriptors listed in "Odor Description" of "Organoleptic Properties" in The Good Scents Company were flagged and used for construction of a tree model, to thereby identify olfactory receptor activity patterns characteristic for compounds with the aroma property "nutty" (Fig. 13). Main olfactory receptor activity patterns identified were "OR5K1 activity of 3.80 or more, and OR1G1 activity of less than 0.13" (identification number 7) and "OR5K1 activity of 3.80 or more, and OR1G1 activity of 0.13 or more, and OR2AK2 activity of 0.82 or more" (identification number 6). That is, it can be predicted that substances classified into leaf with the identification number 7 or 6 are likely to have the aroma property "nutty".

<4> Identification of olfactory receptor activity pattern characteristic for target molecular structure

A tree model was constructed by CART according to the procedure described in <3> above using a flagged value of the target molecular structure (presence, 1; absence, 0) was used as the objective variable and the olfactory receptor activity as the explanatory variable.

<4-1> Pyrazine skeleton

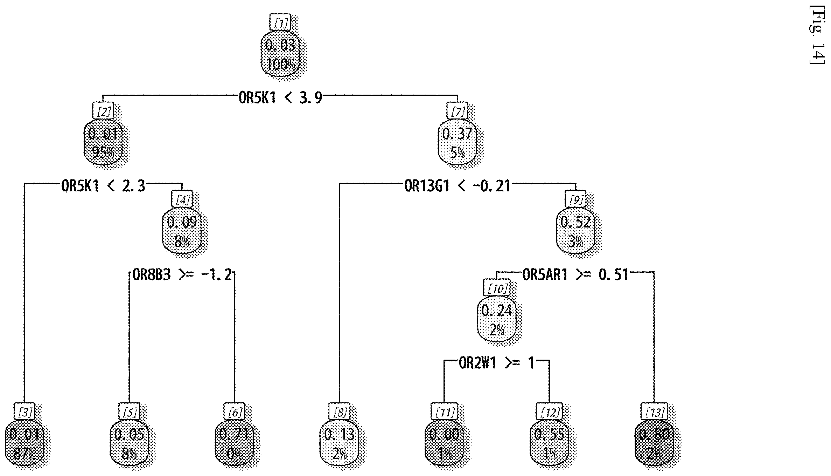

Compounds having a pyrazine skeleton were flagged and used for construction of a tree model, to thereby identify olfactory receptor activity patterns characteristic for compounds having a pyrazine skeleton (Fig. 14). Main olfactory receptor activity patterns identified were "OR5K1 activity of 3.90 or more, and OR13G1 activity of -0.21 or more, and ORSAR1 activity of less than 0.51" (identification number 13), "OR5K1 activity of 3.90 or more, and OR13G1 activity of -0.21 or more, and ORSAR1 activity of 0.51 or more, and OR2W1 activity of less than 1.00" (identification number 12), and "OR5K1 activity of 2.30 or more but less than 3.90, and OR8B3 activity of less than - 1.2" (identification number 6). That is, it can be predicted that substances classified into leaf with the identification number 13, 12, or 6 are likely to have a pyrazine skeleton.

<4-2> Aldehyde group

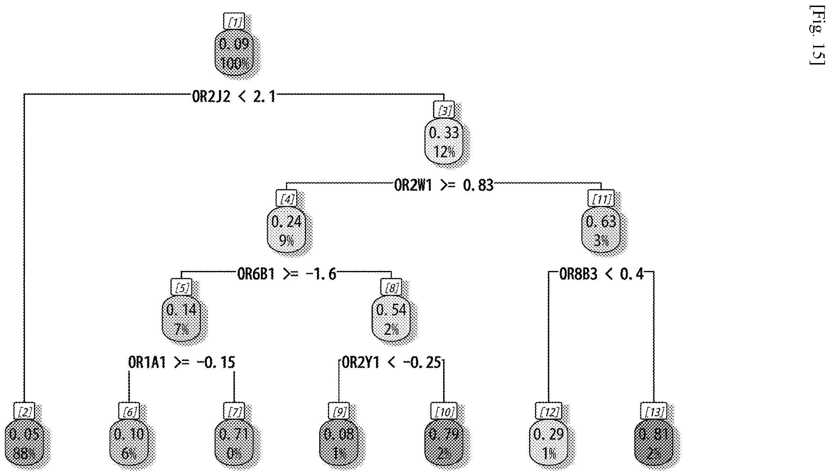

Compounds having an aldehyde group were flagged and used for construction of a tree model, to thereby identify olfactory receptor activity patterns characteristic for compounds having an aldehyde group (Fig. 15). Main olfactory receptor activity patterns identified were "OR2J2 activity of 2.10 or more, and OR2W1 activity of less than 0.83, and OR8B3 activity of 0.40 or more" (identification number 13), "OR2J2 activity of 2.10 or more, and OR2W1 activity of 0.83 or more, and OR6B1 activity of less than -1.60, and OR2Y1 activity of -0.25 or more" (identification number 10), and "OR2J2 activity of 2.10 or more, and OR2W1 activity of 0.83 or more, and OR6B1 activity of -1.60 or more, and OR1A1 activity of less than -0.15" (identification number 7). That is, it can be predicted that substances classified into leaf with the identification number 13, 10, or 7 are likely to have an aldehyde group.

<4-3> Ester bond

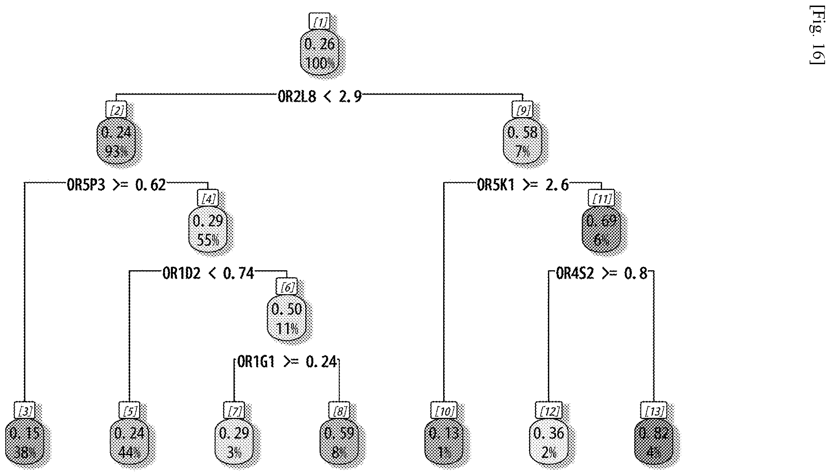

Compounds having an ester bond were flagged and used for construction of a tree model, to thereby identify olfactory receptor activity patterns characteristic for compounds having an ester bond (Fig. 16). Main olfactory receptor activity patterns identified were "OR2L8 activity of 2.90 or more, and OR5K1 activity of less than 2.60, and OR4S2 activity of less than 0.80" (identification number 13) and "OR2L8 activity of less than 2.90, and OR5P3 activity of less than 0.62, and OR1D2 activity of 0.74 or more, and OR1G1 activity of less than 0.24" (identification number 8). That is, it can be predicted that substances classified into leaf with the identification number 13 or 8 are likely to have an ester bond.

< Example C >

Hereinafter, the present invention will be more specifically explained with reference to non-limiting examples according to the 3rd embodiment of the present invention. Although the substances used in the following Example each are referred to as "test substance", those substances can be used as reference substances in the prediction model production method of the present invention and the prediction method of the present invention.

<1> Preparation of human olfactory receptor-expressing cells

<1-1> Preparation of expression vectors of human olfactory receptors

The 352 kinds of expression vectors for human olfactory receptors were obtained according to the same procedure as in <1-1> in Example A.

<1-2> Preparation of olfactory receptor expressing cells

The 352 kinds of cultures of HEK293T cells expressing the 352 kinds of olfactory receptors, respectively, were obtained according to the same procedure as in <1-2> in Example A.

<2> Preparation of human olfactory receptor activity database

<2-1> Luciferase assay

The luciferase assay was carried out according to the same procedure as in <2-1> in Example A except that all the 144 kinds of substances listed in Atlas of odor character profiles (Dravnieks, A., ASTM data series publication, DS 61, PCN 05-061000-36, 1985) as the test substances.

<2-2> Calculation of olfactory receptor activity

The olfactory receptor activity was calculated according to the same procedure as in <2-2> in Example A.

<2-3> Aroma property information

As the aroma property information of the test substances, the "percentage of applicability" (P.A. values) listed in Atlas of odor character profiles (Dravnieks, A., ASTM data series publication, DS 61, PCN 05-061000-36, 1985) were excerpted.

<3> Prediction of applicability to aroma property based on olfactory receptor activation data

For each combination of the test substance and the olfactory receptor, the correlation coefficient between the P.A. value obtained in <2-3> above and the olfactory receptor activity calculated in <2-2> above was calculated. Linear regression models (Equations 1-3) were constructed by machine learning using the olfactory receptor activity as the explanatory variable and the P.A. value as the objective variable, wherein the olfactory receptor activity and P.A. value used provides the absolute value of the correlation coefficient exceeded the threshold value (0.2). In the equations, to the olfactory receptor name (e.g., OR1F1), the olfactory receptor activity for that olfactory receptor is assigned.

<3-1> Quantitative aroma property "STARWBERRY"

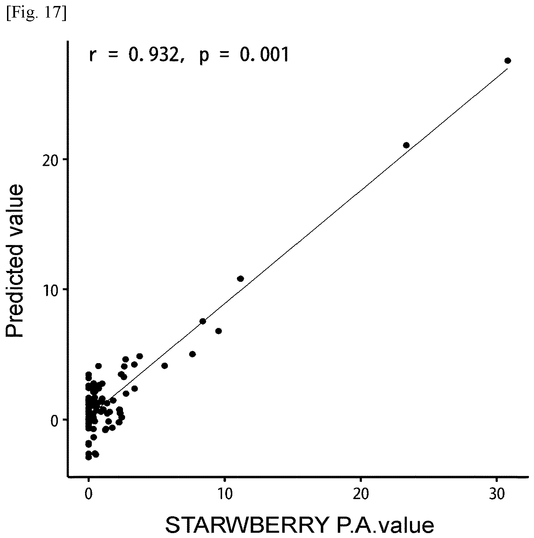

The absolute value of the correlation coefficient between the P.A. value and the olfactory receptor activity for "STARWBERRY" exceeded 0.2 for 61 olfactory receptors. A linear regression model to predict the P.A. value of "STARWBERRY" (Equation 1) was constructed using the olfactory receptor activities for these 61 olfactory receptors. The correlation coefficient between the predicted P.A. values by the constructed regression model and the measured P.A. values of the 144 compounds was 0.932 (p < 0.001) (Fig. 17).

The predicted P.A. value of "STARWBERRY" = 0.305 + 1.560OR1F1 - 1.428OR1I1 + 0.982OR1J1 + 0.738OR2B6 + 0.415OR2B11 - 0.194OR2C3 - 1.092OR2G6 + 0.733OR2K2 + 1.313OR2L8 - 0.981OR2T1 + 0.018OR2T6 + 0.660OR2W3 - 0.6510R4A47 + 1.546OR4B1 + 0.1310R4C13 + 1.377OR4D10 - 0.348OR4F15 + 0.458OR4K13 - 0.843OR4P4 - 0.758OR4Q3 + 1.342OR4X1 + 0.085OR5AK2 + 0.537OR5D14 - 2.108OR5H14 + 1.377OR5I1 + 0.265OR5J2 - 0.072OR5M3 - 0.332OR5M8 - 0.019OR6C2 - 0.695OR6C66P + 0.142OR6K2 - 1.751OR6T1 + 0.146OR8D4 + 3.164OR8K1 - 2.203OR8U1 - 0.562OR10A4 + 0.921OR10A7 - 1.501OR10D3 + 2.699OR10H2 - 1.454OR10J5 + 0.733OR10T2 - 2.356OR12D3 - 1.530OR13F1 - 1.186OR13G1 - 3.013OR13H1 + 0.075OR13J1 - 0.109OR14K1 + 0.587OR51B2 - 1.775OR51B4 - 0.116OR51M1 + 0.968OR51T1 + 1.253OR51V1 - 0.327OR52A4 - 1.189OR52B2 + 1.613OR52D1 - 1.592OR52H1 + 1.186OR52J3 + 0.560OR52N5 + 0.611OR52P2P - 2.133OR52R1 + 1.951OR56A5 ···(Equation 1).

<3-2> Quantitative aroma property "ANISE (LICORICE)"

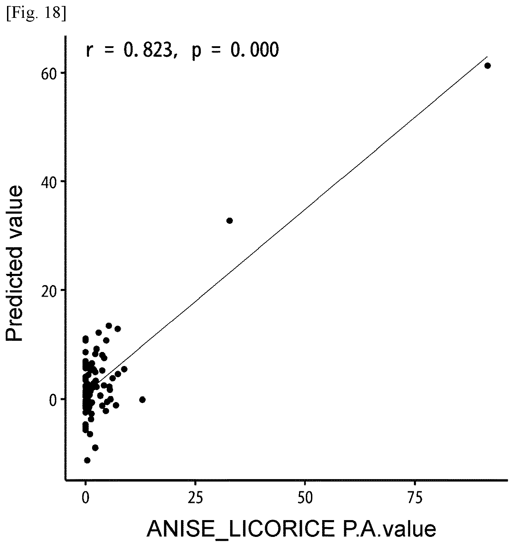

The absolute value of the correlation coefficient between the P.A. value and the olfactory receptor activity for "ANISE (LICORICE)" exceeded 0.2 for 27 olfactory receptors. A linear regression model to predict the P.A. value of "ANISE (LICORICE)" (Equation 2) was constructed using the olfactory receptor activities for these 27 olfactory receptors. The correlation coefficient between the predicted P.A. values by the constructed regression model and the measured P.A. values of the 144 compounds was 0.823 (p < 0.001) (Fig. 18).

The predicted P.A. value of "ANISE (LICORICE)" = 3.334 - 1.835OR1J2 - 2.644OR2A25 - 1.425OR2G2 - 0.561OR2L2 - 1.147OR2T11 + 5.260OR3A3 + 3.676OR4C13 + 0.353OR4D2 - 1.731OR4P4 + 0.273OR4X1 + 0.049OR5AK2 - 0.645OR6C6 + 1.418OR6T1 - 0.144OR7D4 - 2.990OR8G5 + 0.613OR9Q2 - 0.169OR10A3 - 0.535OR10J3 + 5.271OR13C3 + 1.047OR13D1 - 2.075OR51A4 - 1.535OR51B6 + 0.880OR51G1 + 0.551OR51H1 - 0.467OR51M1 - 0.839OR52A4 - 2.291OR52N1 ···(Equation 2).

<3-3> Quantitative aroma property "NEW RUBBER"

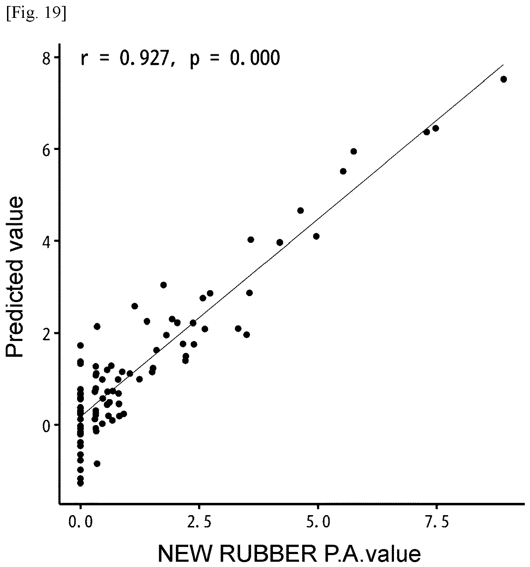

The absolute value of the correlation coefficient between the P.A. value and the olfactory receptor activity for "NEW RUBBER" exceeded 0.2 for 56 olfactory receptors. A linear regression model to predict the P.A. value of "NEW RUBBER" (Equation 3) was constructed using the olfactory receptor activities for these 56 olfactory receptors. The correlation coefficient between the predicted P.A. values by the constructed regression model and the measured P.A. values of the 144 compounds was 0.927 (p < 0.001) (Fig. 19).

The predicted P.A. value of "NEW RUBBER" = 1.442 + 0.769OR1G1 + 0.148OR1J1 - 0.718OR1L3 + 0.350OR2A2 - 0.289OR2AP1 + 0.184OR2D2 + 0.041OR2L8 + 0.211OR2M2 - 0.060OR2M4 - 0.077OR4A16 + 0.470OR4C6 - 0.712OR4C12 - 1.136OR4D9 + 0.786OR4E2 + 0.019OR4G11P + 0.248OR4H12P - 0.262OR4N2 + 0.512OR4S1 + 1.233OR5AU1 - 0.331OR5B2 - 0.117OR5C1 - 0.869OR5L2 - 0.823OR5T2 + 0.126OR6B2 - 0.131OR6C70 + 0.463OR6K3 - 0.465OR6M1 + 0.004OR6Q1 + 0.108OR7A17 - 0.210OR8B3 - 0.471OR8G2 - 0.094OR8H3 - 0.978OR8K1 - 0.378OR9K2 + 0.658OR9Q2 - 1.111OR10A5 + 0.319OR10G3 + 0.183OR10G4 + 0.271OR10J3 - 0.038OR10K1 + 0.240OR10P1 + 0.584OR13D1 + 0.164OR14C36 - 0.772OR14I1 + 0.872OR51B2 + 0.179OR51H1 + 0.185OR51I2 - 0.936OR51L1 + 0.651OR51Q1 + 0.220OR52A5 - 0.001OR52B4 - 0.374OR52N2 + 0.344OR52W1 + 0.920OR56A3 - 0.786OR56A5 - 0.056OR56B1 ···(Equation 3).

Industrial Applicability

In an embodiment, according to the present invention, the presence or absence of an aroma property or an olfactory receptor activation property in a substance can be predicted. In an embodiment, according to the present invention, the presence or absence of a constituent, such as an aroma property or a molecular structure, in a substance can be predicted. In an embodiment, according to the present invention, the applicability to an aroma property in a substance can be predicted.

**Claims**

1. A method for predicting the presence or absence of an objective property for a test substance, the method comprising:

   a step of predicting the presence or absence of the objective property for the test substance on the basis of the maximum similarity of stereochemical structure between the test substance and a reference substance, wherein the property is an aroma property or an olfactory receptor activation property.

2. The method according to claim 1, wherein the reference substance includes a positive control for the objective property.

3. The method according to claim 1 or 2, wherein the reference substance is one kind of substance.

**EP 4 130 736 A1**

4. The method according to claim 1 or 2, wherein the reference substance is a combination of two or more kinds of substances.

5. The method according to any one of claims 1 to 4,

   wherein the reference substance includes a positive control for the objective property, and
   wherein the test substance is predicted to have the objective property when the maximum similarity of stereo-chemical structure between the test substance and the positive control is high.

6. The method according to any one of claims 1 to 5, wherein said predicting comprises a step of clustering the test substance and the reference substance on the basis of the maximum similarity of stereochemical structure between the test substance and the reference substance.

7. The method according to claim 6,

   wherein the reference substance includes a positive control for the objective property, and
   wherein the test substance is predicted to have the objective property when the test substance is clustered into a cluster containing the positive control.

8. The method according to any one of claims 1 to 7, wherein the method further comprises a step of calculating the maximum similarity, prior to said predicting.

9. A method for screening a substance having an objective property, the method comprising:

   a step of predicting the presence or absence of the objective property for a test substance by the method according to any one of claims 1 to 8, and
   a step of selecting the test substance predicted to have the objective property as the substance having the objective property,
   wherein the property is an aroma property or an olfactory receptor activation property.

10. The method according to any one of claims 1 to 9, wherein the method further comprises a step of confirming the presence or absence of the objective property for the test substance predicted to have the objective property.

11. The method according to any one of claims 1 to 10, wherein the maximum similarity is used for said predicting in combination with a structural similarity between the test substance and the reference substance other than the maximum similarity.

12. A method for designing a substance having an objective property, the method comprising:

   a step of designing the substance to be designed on the basis of the maximum similarity of stereochemical structure between the substance to be designed and a reference substance,
   wherein the property is an aroma property or an olfactory receptor activation property.

13. The method according to claim 12,

   wherein the reference substance includes a positive control for the objective property,
   wherein said designing is carried out so that the substance to be designed is clustered into a cluster containing the positive control, and
   wherein said clustering comprises a step of clustering the substance to be designed and the reference substance on the basis of the maximum similarity of stereochemical structure between the substance to be designed and the reference substance.

43

[Fig. 1]

[Fig. 2]

[Fig. 3]

[Fig. 4]

[Fig. 5]

[Fig. 6]

[Fig. 7]

[Fig. 8]

[Fig. 9]

[Fig. 10]

[Fig. 11]

[Fig. 12]

[Fig. 13]

[Fig. 14]

[Fig. 15]

[Fig. 16]

[Fig. 17]

r = 0.932, p = 0.001

Predicted value

STARWBERRY P.A.value

[Fig. 18]

[Fig. 19]

## INTERNATIONAL SEARCH REPORT

International application No.

PCT/JP2021/013181

### A. CLASSIFICATION OF SUBJECT MATTER

G01N 33/00(2006.01)i; G06N 20/00(2019.01)i; G16C 20/40(2019.01)i; C07K 14/705(2006.01)n; C12Q 1/02(2006.01)n; C12Q 1/66(2006.01)n; C12Q 1/6897(2018.01)n

FI:       G01N33/00 C; G16C20/40; G06N20/00 160; C12Q1/66; C12Q1/6897 Z;         C07K14/705; C12Q1/02

According to International Patent Classification (IPC) or to both national classification and IPC

### B. FIELDS SEARCHED

Minimum documentation searched (classification system followed by classification symbols)
G01N33/00; G06N20/00; G16C20/40

Documentation searched other than minimum documentation to the extent that such documents are included in the fields searched

| | |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)
JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| A | US 2018/0107803 A1 (INTERNATIOIRAL BUSINESS MACHINES CORPORATION) 19 April 2018 (2018-04-19) entire text, all drawings | 1–13 |
| A | KR 10-1289948 B1 (INJE UNIVERSITY INDUSTRY-ACADEMIC COOPERATION FOUNDATION) 26 July 2013 (2013-07-26) entire text, all drawings | 1–13 |

☒ Further documents are listed in the continuation of Box C.     ☒ See patent family annex.

| | | |
|---|---|---|
| * | Special categories of cited documents: | |
| "A" | document defining the general state of the art which is not considered to be of particular relevance | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 24 May 2021 (24.05.2021) | 01 June 2021 (01.06.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**

International application No.

PCT/JP2021/013181

C (Continuation).   DOCUMENTS CONSIDERED TO BE RELEVANT

| Category* | Citation of document, with indication, where appropriate, of the relevant passages | Relevant to claim No. |
|---|---|---|
| A | 小坂宏四郎ほか，機械学習に基づく嗅覚受容体のリガンド予測，第 71 回（平成 21 年）全国大会講演論文集(4) インタフェースコンピュータと人間社会，2009, pp. 667-668, entire text, all drawings, non-official translation (KOSAKA, Koshiro et al., "Ligand Prediction for the Olfactory Receptors based on Machine Learning", Lecture proceedings (4) of the 71st (2009) national conference: Interface computer and human society) | 1-13 |
| A | 原田祐希ほか，平成 27 年電気学会全国大会講演論文集 [3] エレクトロニクス／情報工学システム／センサ・マイクロマシン THE 2015 ANNUAL MEETING RECORD I.E.E. JAPAN, 2015, p. 115, entire text, all drawings, non-official translation (HARADA, Yuki et al., Papers [3] from the 2015 IEEJ National Convention: Electronics/Information Engineering Systems/Sensors and Micromachines) | 1-13 |
| A | 神崎亮平，並木重宏，分子の物理化学的特徴に基づく複合臭の快適性の予測，コスメロジー研究報告，01 September 2013, vol. 21, pp. 118-121, entire text, all drawings, (KANZAKI, Ryohei, NAMIKI, Shigehiro, "Predicting odor pleasantness for olfactory mixture based on physicochemical property", Annual report of cosmetology) | 1-13 |
| A | MAHLKE, Inga T. et al., "Chemical Indices and Methods of Multivariate Statistics as a Tool for Odor Classification", Environmental Science & Technology, 01 April 2007, vol. 41, no. 7, pp. 2414-2421, entire text, all drawings | 1-13 |
| A | 河村元ほか，化合物活性予測のための Tanimoto 係数と Random Forest の Proximity Measure の組合せ手法，情報処理学会研究報告，03 March 2008, vol. 2008, no. 15 (BIO-12), pp. 39-46, entire text, all drawings, (KAWAMURA, Gen et al., "A Combination Method of the Tanimoto Coefficient and Proximity Measure of Random Forest for Compound Activity Prediction", IPSJ SIG Technical Report) | 1-13 |
| A | 石原由一朗，高橋由雅，構造類似性を基礎とした化学物質の毒性予測のシステム化に関する研究，情報化学討論会・構造活性相関シンポジウム講演要旨集，07 November 2001, vol. 24th-29th, pp. 213-214, entire text, all drawings, (ISHIHARA, Yuichiro, TAKAHASHI, Yoshimasa, "Computerization of Structure-Toxicity Modeling Based on Structural Similarity", Lecture abstracts of Symposium on Chemical Information and Computer Sciences and Symposium on Structure-Activity Relationships) | 1-13 |

Form PCT/ISA/210 (continuation of second sheet) (January 2015)

**INTERNATIONAL SEARCH REPORT**

| International application No. |
| --- |
| PCT/JP2021/013181 |

C (Continuation).    DOCUMENTS CONSIDERED TO BE RELEVANT

| Category* | Citation of document, with indication, where appropriate, of the relevant passages | Relevant to claim No. |
| --- | --- | --- |
| A | JP 2008-100918 A (NEC CORP.) 01 May 2008 (2008-05-01) entire text, all drawings | 1-13 |
| P, A | US 2020/0399558 A1 (THE REGENTS OF THE UNIVERSITY OF CALIFORNIA) 24 December 2020 (2020-12-24) entire text, all drawings | 1-13 |

Form PCT/ISA/210 (continuation of second sheet) (January 2015)

**INTERNATIONAL SEARCH REPORT**

Information on patent family members

| International application No. |
| --- |
| PCT/JP2021/013181 |

| Patent Documents referred in the Report | Publication Date | Patent Family | Publication Date |
| --- | --- | --- | --- |
| US 2018/0107803 A1 | 19 Apr. 2018 | (Family: none) | |
| KR 10-1289948 B1 | 26 Jul. 2013 | (Family: none) | |
| JP 2008-100918 A | 01 May 2008 | (Family: none) | |
| US 2020/0399558 A1 | 24 Dec. 2020 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019037197 A

**Non-patent literature cited in the description**

- **KOBI SNITZ.** Predicting Odor Perceptual Similarity from Odor Structure. *PLoS Comput Biol,* September 2013, vol. 9 (9), e1003184
- **ANDREAS KELLER.** Predicting human olfactory perception from chemical features of odor molecules. *Science,* February 2017, vol. 355 (6327), 820-826
- **BENJAMIN SANCHEZ-LENGELING.** Machine Learning for Scent: Learning Generalizable Perceptual Representations of Small Molecules. *arXiv:1910.10685v1,* October 2019
- **K. KAJIYA et al.** *Journal of Neuroscience,* 15 August 2001, vol. 21 (16), 6018-6025
- **VAN DER MAATEN et al.** Visualizing Data Using t-SNE. *Journal of Machine Learning Research,* 2008, vol. 9, 2579-2605